# EUROPEAN PATENT APPLICATION

(11) **EP 4 592 302 A1**
(43) Date of publication of application: **30.07.2025**
(21) Application number: 23867651.4
(22) Date of filing: 23.09.2023
(51) Int. Cl.: C07F 9/6584, C07F 9/6506, A61K 31/66, A61P 35/00

(54) **SMALL MOLECULE COMPOUND HAVING PHOSPHORYLATED ARYL STRUCTURE, AND USE THEREOF**

(30) Priority: 23.09.2022 CN 202211170257; 02.06.2023 CN 202310645010
(71) Applicant: Hangzhou Phecdamed Co., Ltd., Hangzhou, Zhejiang 311100 (CN)
(72) Inventor: LIU, Dong, Hangzhou, Zhejiang 311100 (CN)
(74) Representative: Viering, Jentschura & Partner mbB Patent- und Rechtsanwälte
(86) International application number: PCT/CN2023/120910
(87) International publication number: WO 2024/061366

(57) **Abstract**

The present application discloses a small molecule compound having a phosphorylated aromatic structure and application thereof. The compound and pharmaceutically acceptable salts, stereoisomers, solvates or prodrugs thereof provided in the present application or the pharmaceutical composition provided in the second aspect of the present invention, by targeting the TEAD palmitoyl pocket, effectively inhibits/blocks YAP-TEAD binding, inhibits/blocks the transcription function of YAP-TEAD, thereby preventing and/or treating diseases related to increased TEAD transcription levels and/or YAP phosphorylation disorders and/or Hippo signaling pathway disorders.

## Description

### Cross-reference to related applications

This patent application claims priority to the Chinese patent application No.2022111702572 filed on September 23, 2022, and entitled with "Small molecule compounds with phosphorylated aryl structures and uses thereof", which is incorporated herein by reference in its entirety.

This patent application claims priority to the Chinese patent application No.202310645010X filed on June 2, 2023, and entitled with "Small molecule compounds with phosphorylated aryl structures and uses thereof", which is incorporated herein by reference in its entirety.

### Technical Field

The present invention relates to the field of chemical medicine, and in particular to a class of small molecule compound with phosphorylated aryl structure and use thereof.

### Background Art

The Hippo signaling pathway plays a key regulatory role in embryonic development, cell proliferation, wound healing, tissue regeneration, and homeostasis. Its dysfunction is one of the driving factors for the occurrence and progression of various diseases. The Hippo signaling pathway mainly includes the kinase module in the cytoplasm and the transcription module in the nucleus, and YAP/TAZ serves as a link between them and controls the opening and closing of the Hippo signal through its phosphorylation state: when YAP/TAZ is phosphorylated by LATS1/2 of the kinase module, they are remained in the cytoplasm and degraded by the proteasome (Hippo-off); when YAP/TAZ is dephosphorylated, they are transported into the nucleus and bind to the transcription factor TEAD family as a transcription co-activator, greatly increasing the transcriptional activity of TEAD and induces effects such as promoting proliferation (Hippo-on). Since the Hippo signaling pathway kinase module negatively regulates transcriptional output, traditional small molecule kinase inhibitor strategies cannot theoretically inhibit this process, but on the contrary, so it is not suitable as a drug target. At present, studies have found that some small molecule compounds can block the output of the Hippo signaling, but most of them have problems such as unclear mechanisms and poor biological activity and pharmacokinetic properties. The reason is that this pathway lacks druggable targets and ideal small molecule binding sites.

In order to solve the above problems, the inventors found in the study that the Hippo signaling pathway finds that the transcription factor TEAD family in the transcription module has a conserved cysteine S-palmitoylation modification on its YAP binding domain, and is different from the usual protein acylation modification, the fifteen-carbon fatty long chain of the palmitoyl is not exposed outside the protein to participate in the membrane localization or transport of the protein, but is deeply buried in a central pocket of TEAD. Functionally, this palmitoylation modification may serve to stabilize TEAD protein, and also affects YAP-TEAD interaction and TEAD transcriptional activity. Most importantly, this palmitoyl pocket has a tubular hydrophobic cavity and a hydrophilic branch pocket, which is very suitable as a binding site for small molecule drugs; and the residue of TEAD palmitoyl is a conserved cysteine, which is also be used to design covalent inhibitors.

The undruggable nature of YAP has always been a difficulty in the research of small molecule drugs. The discovery of the palmitoyl pocket of TEAD has opened the door to the use of small molecules to intervene in the YAP-TEAD signaling axis. That is, small molecules can bind to the palmitoyl pocket of the transcription factor TEAD, destroy the binding of YAP-TEAD, and achieve the purpose of inhibiting its transcriptional activity. Therefore, it is urgent to develop YAP-TEAD inhibitor drugs to treat diseases caused by disorders of the Hippo signaling pathway and overactivation of YAP1, and to solve the problems of drug resistance and recurrence of existing disease treatment drugs.

### Summary of the invention

The object of the present invention is to provide a class of small molecule compound with multi-substituted phosphorylated aryl structure or pharmaceutically acceptable salts, stereoisomers, solvates or prodrugs thereof.

Another object of the present invention is to provide a pharmaceutical composition comprising the above-mentioned compoundsor pharmaceutically acceptable salts, stereoisomers, solvates or prodrugs thereof.

Another object of the present invention is to provide the use of the above-mentioned compounds or pharmaceutically acceptable salts, stereoisomers, solvates or prodrugs thereof.

In order to solve the above technical problems, the first aspect of the present invention provides compounds with a structure represented by general formula (I), 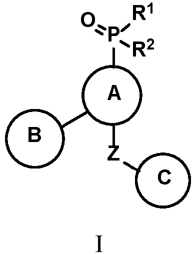
or pharmaceutically acceptable salts, stereoisomers, solvates or prodrugs thereof,
wherein Z is -CH₂-, -NH(CH₂)n-, -O(CH₂ )n-, -S-, -SO-, -SO₂-, each n is independently an integer of 0 to 3;
R¹ is C₁₋₆ alkyl, C₂₋₆ alkenyl, C₁₋₆ alkyl with at least one hydrogen replaced by R¹⁻¹, C₂₋₆ alkenyl with at least one hydrogen replaced by R¹⁻¹;
R² is C₁₋₆ alkyl, C₂₋₆ alkenyl, C₁₋₆ alkyl with at least one hydrogen replaced by R¹⁻¹, C₂₋₆ alkenyl with at least one hydrogen replaced by R¹⁻¹;
or, R¹ and R² are bonded to form a 4 to 6-membered ring or a 4 to 6-membered ring with at least one hydrogen replaced by R¹⁻¹, wherein each R¹⁻¹ is independently amino, hydroxyl, hydroxyl-substituted C₁₋₆ alkyl, and 5- or 6-membered monocyclic heteroaryl;
Ring A is 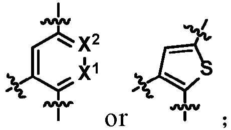 wherein X¹ is CH or N, X² is CH or N;
Ring B is a 5 to 10-membered heteroaryl, 5 to 10-membered heteroaryl with at least one hydrogen replaced by 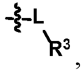 5 to 10-membered heteroaryl ketone group, 5 to 10-membered heteroaryl ketone group with at least one hydrogen replaced by 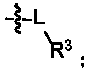
wherein L is none, -CH₂-, -NH-, -O-, -CH₂O-, -S-, -SO-, or -SO₂-;
each R³ is independently hydroxyl, cyano( 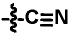 ), C₁₋₆ alkyl, C₁₋₆ alkyl with at least one hydrogen replaced by R³⁻¹, amino, amino with at least one hydrogen replaced by R³⁻¹, 3 to 6-membered heterocycloalkyl, 3 to 6-membered heterocycloalkyl with at least one hydrogen replaced by R³⁻¹, 5- or 6-membered monocyclic heteroaryl, 5- or 6-membered monocyclic heteroaryl with at least one hydrogen replaced by R³⁻¹;
wherein each R³⁻¹ is independently hydroxyl, C₁₋₄ alkyl with at least one hydrogen replaced by hydroxyl , C₁₋₄ alkoxy, halogen;
Ring C is phenyl, phenyl with at least one hydrogen replaced by R⁴, 3 to 7-membered monocyclic alkyl, 3 to 7-membered monocyclic alkyl with at least one hydrogen replaced by R⁴ , C₅₋₁₂ bridged bicycloalkyl, C₅₋₁₂ bridged bicycloalkyl with at least one hydrogen replaced by R⁴, C₁₀₋₂₀ bridged tricycloalkyl, C₁₀₋₂₀ bridged tricycloalkyl with at least one hydrogen replaced by R⁴, 8 to 10-membered benzocycloalkyl, 8 to 10-membered benzocycloalkyl with at least one hydrogen replaced by R⁴;
wherein each R⁴ is independently C₁₋₄ alkyl, C₁₋₄ alkyl with at least one hydrogen replaced by halogen, halogen, 3 to 6-membered cycloalkyl, C₁₋₄ alkoxy, C₁₋₄ alkoxy with at least one hydrogen replaced by halogen, C₁₋₄ alkyl thiol, C₁₋₄ alkyl thiol with at least one hydrogen replaced by halogen, halogen-substituted thiol.

In some preferred embodiments, the structure represented by general formula (I) is general formula (I'): 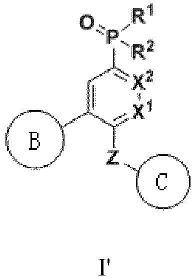 wherein X¹ is CH or N, and X² is CH or N.

In some preferred embodiments, X¹ and X² are not N at the same time.

In some preferred embodiments, each R¹ is independently methyl, ethyl, methyl with at least one hydrogen atom replaced by R¹⁻¹, ethyl with at least one hydrogen atom replaced by R¹⁻¹, ethenyl, 1-propenyl, or 2-propenyl.

In some preferred embodiments, each R² is independently methyl, ethyl, methyl with at least one hydrogen atom replaced by R¹⁻¹ , ethyl with at least one hydrogen atom replaced by R¹⁻¹, ethenyl 1-propenyl, or 2-propenyl.

In some preferred embodiments, R¹⁻¹ is amino, hydroxyl or 5- or 6-membered nitrogen-containing monocyclic heteroaryl;

In some preferred embodiments, R¹ and R² are bonded to each other to form a 4 to 6-membered ring, or R¹ and R² are bonded to each other to form a 4 to 6-membered ring with at least one hydrogen replaced by R¹⁻¹ .

In some preferred embodiments, R¹ and R² are bonded to each other to form a 4 to 6-membered ring, or R¹ and R² are bonded to each other to form a 4 to 6-membered ring with at least one hydrogen replaced by R¹⁻¹; the 4 to 6-membered ring contains none or 1, 2, or 3 other heteroatoms selected from N, O or S (except the P atom to which R¹ and R² are commonly connected).

In some preferred embodiments, each R¹⁻¹ is independently amino, hydroxy, hydroxy-substituted methyl, hydroxy-substituted ethyl, or a 5- or 6-membered nitrogen-containing monocyclic heteroaryl.

In some preferred embodiments, Ring A is

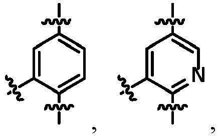

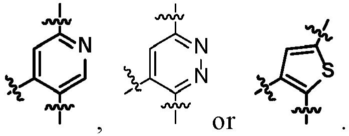

In some preferred embodiments, Ring B is a 5-membered nitrogen-containing monocyclic heteroaryl, a 6-membered nitrogen-containing monocyclic heteroaryl, a 5-membered nitrogen-containing monocyclic heteroaryl group with at least one hydrogen replaced by 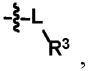 a 6-membered nitrogen-containing monocyclic heteroaryl group with at least one hydrogen replaced by 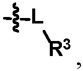 an 8 to 10-membered nitrogen-containing fused ring heteroaryl, an 8 to 10-membered nitrogen-containing fused ring heteroaryl with at least one hydrogen replaced by 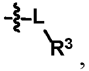 a 5-membered nitrogen-containing monocyclic heteroaromatic ketone group, a 6-membered nitrogen-containing monocyclic heteroaromatic ketone group, a 5-membered nitrogen-containing monocyclic heteroaromatic ketone group with at least one hydrogen replaced by 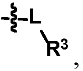 or a 6-membered nitrogen-containing monocyclic heteroaromatic ketone group with at least one hydrogen replaced by

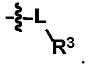

In some preferred embodiments, Ring C is phenyl, phenyl with at least one hydrogen replaced by R⁴ , 4 to 6-membered monocycloalkyl, 4 to 6-membered monocycloalkyl with at least one hydrogen replaced by R⁴, C₅₋₈ bridged bicycloalky, C₅₋₁₂ bridged bicycloalkyl with at least one hydrogen replaced by R⁴, C₁₀₋₂₀ bridged tricycloalkyl, C₁₀₋₂₀ bridged tricycloalkyl with at least one hydrogen replaced by R⁴ , 8 to 10-membered benzocycloalkyl, 8 to 10-membered benzocycloalkyl with at least one hydrogen replaced by R⁴.

In some preferred embodiments, in the Ring B, the 5- or 6-membered nitrogen-containing monocyclic heteroaryl is

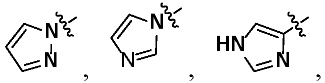

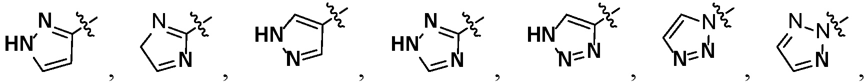

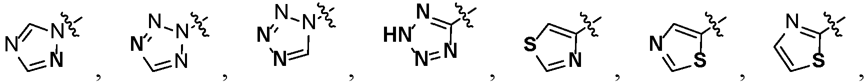

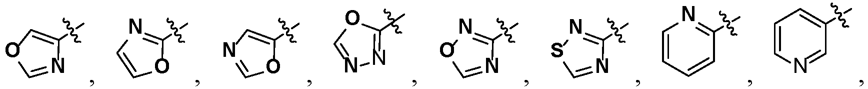

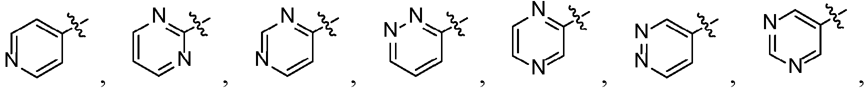

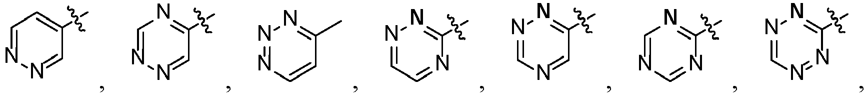

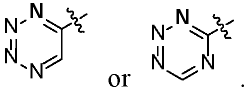

In some preferred embodiments, in the Ring B, the 5-membered nitrogen-containing monocyclic heteroaryl with at least one hydrogen replaced by

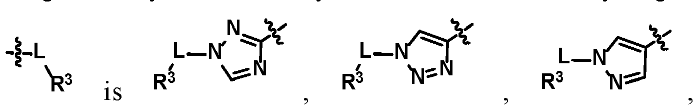

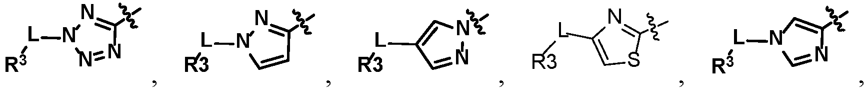

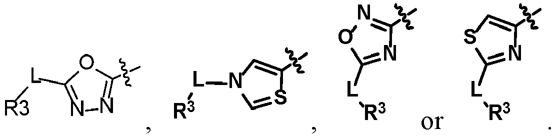

In some preferred embodiments, in the Ring B, the 6-membered nitrogen-containing monocyclic heteroaryl with at least one hydrogen replaced by

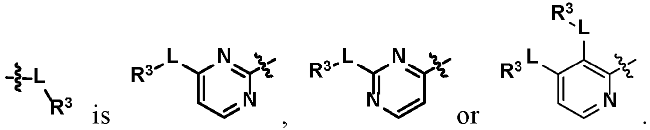

In some preferred embodiments, in the Ring B, the 8 to 10-membered nitrogen-containing monocyclic heteroaryl is

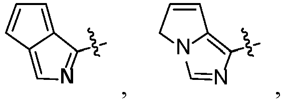

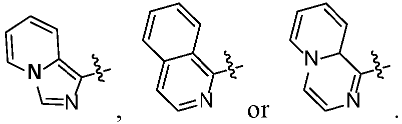

In some preferred embodiments, in the Ring B, the 8 to 10-membered nitrogen-containing monocyclic heteroaryl with at least one hydrogen replaced by 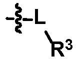 is

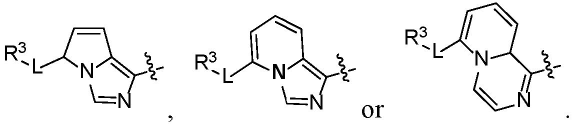

In some preferred embodiments, in the Ring B, the 6-membered nitrogen-containing monocyclic heteroaromatic ketone group is a pyridone group.

In some preferred embodiments, the 6-membered nitrogen-containing monocyclic heteroaromatic ketone group is

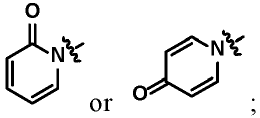

In some preferred embodiments, the 6-membered nitrogen-containing monocyclic heteroaromatic ketone group with at least one hydrogen replaced by 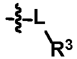 is a pyridone group with at least one hydrogen replaced by

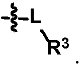

In some preferred embodiments, the 6-membered nitrogen-containing monocyclic heteroaromatic ketone group with at least one hydrogen replaced by 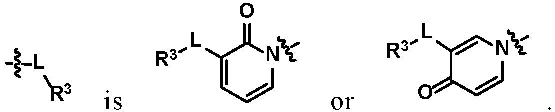 In some preferred embodiments, in the Ring C, the phenyl with at least one hydrogen replaced by R⁴ is

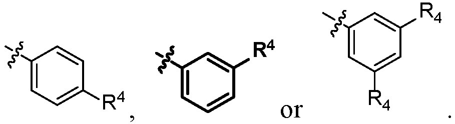

In some preferred embodiments, in the Ring C, the 4 to 6-membered monocyclic alkyl is cyclohexyl, cyclopentyl or cyclobutyl.

In some preferred embodiments, in the Ring C, the 4 to 6-membered monocyclic alkyl with one hydrogen replaced by R⁴ is 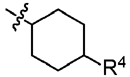 or

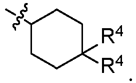

In some preferred embodiments, in the Ring C, the C₅₋₈ bridged bicycloalkyl group is bicyclo[1.1.1]pentanyl or bicyclo[2.2.2]octanyl.

In some preferred embodiments, in the Ring C, the C₅₋₁₂ bridged bicycloalkyl with at least one hydrogen replaced by R⁴ is a bicyclo[1.1.1]pentanyl with one hydrogen replaced by R⁴ or a bicyclo[2.2.2]octyl group with one hydrogen replaced by R⁴ .

In some preferred embodiments, in the Ring C, the C₁₀₋₂₀ bridged tricycloalkyl is adamantyl.

In some preferred embodiments, in the Ring C, the C₁₀₋₂₀ bridged tricycloalkyl with at least one hydrogen replaced by R⁴ is an adamantyl with one hydrogen replaced by R⁴.

In some preferred embodiments, in the Ring C, the 8 to 10-membered benzocycloalkyl is benzocyclopentyl, benzocyclobutyl or benzocyclohexyl.

In some preferred embodiments, in the Ring C, the 8 to 10-membered benzocycloalkyl with at least one hydrogen replaced by R⁴ is a benzocyclopentyl with at least one hydrogen replaced by R⁴, a benzocyclobutyl with at least one hydrogen replaced by R⁴, or a benzocyclohexyl with at least one hydrogen replaced by R⁴.

In some preferred embodiments, each R³ is independently hydroxyl, cyano, C₁₋₄ alkyₗ, C₁₋₄ alkyl with at least one hydrogen replaced by R³⁻¹ , amino, amino with at least one hydrogen replaced by R³⁻¹ , 3 to 6-membered nitrogen heterocycloalkyl, 3 to 6-membered oxygen heterocycloalkyl, 3 to 6-membered nitrogen heterocycloalkyl with at least one hydrogen replaced by R³⁻¹, 3 to 6-membered oxygen heterocycloalkyl with at least one hydrogen replaced by R³⁻¹, 6-membered nitrogen-containing monocyclic heteroaryl, and 6-membered nitrogen-containing monocyclic heteroaryl with at least one hydrogen replaced by R³⁻¹.

In some preferred embodiments, each R³ is independently hydroxyl, cyano, methyl, ethyl, n-propyl, isopropyl, n-butyl, isobutyl, tert-butyl, methyl with at least one hydrogen replaced by R³⁻¹, ethyl with at least one hydrogen replaced by R³⁻¹ , n-propyl with at least one hydrogen replaced by R³⁻¹, isopropyl with at least one hydrogen replaced by R³⁻¹, amino, with at least one hydrogen replaced by R³⁻¹, 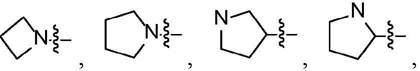 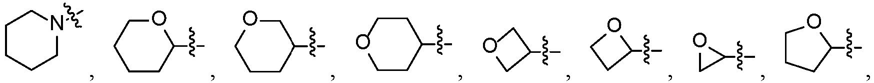 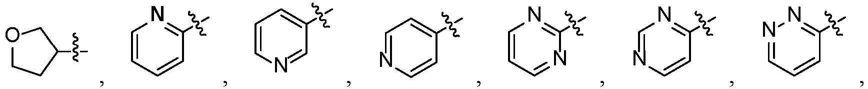 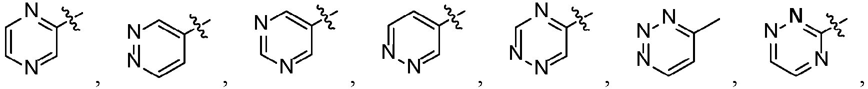 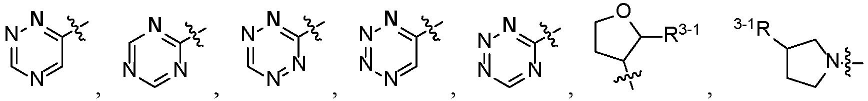 or

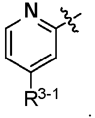

**In** some preferred embodiments, each R³⁻¹ is independently hydroxyl, methyl with at least one hydrogen replaced by hydroxyl, ethyl with at least one hydrogen replaced by hydroxyl, n-propyl with at least one hydrogen replaced by hydroxyl, isopropyl with at least one hydrogen replaced by hydroxyl, methoxy, ethoxy, n-propoxy, isopropoxy, fluorine, chlorine, bromine, or iodine;

In some preferred embodiments, each R⁴ is independently methyl, ethyl, n-propyl, isopropyl, n-butyl, isobutyl, tert-butyl, methyl with at least one hydrogen is replaced by fluorine, ethyl with at least one hydrogen replaced by fluorine, n-propyl with at least one hydrogen replaced by fluorine, isopropyl with at least one hydrogen replaced by fluorine, fluorine, chlorine, bromine, iodine, cyclopropyl, cyclobutyl, cyclopentyl, cyclohexyl, methoxy, ethoxy, n-propoxy, isopropoxy, trifluoromethoxy, hexafluoroethoxy, methyl thiol, ethyl thiol, n-propyl thiol, isopropyl thiol, methyl thiol with at least one hydrogen replaced by fluorine, or ethyl thiol with at least one hydrogen replaced by fluorine, or halogen-substituted thiol (preferably fluorine-substituted thiol, more preferably pentafluoro thiol).

In some preferred embodiments, the compound is selected from any one of the following:

| | |
|---|---|
| 1 | 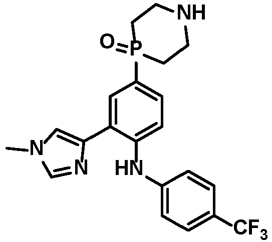 |
| 2 | 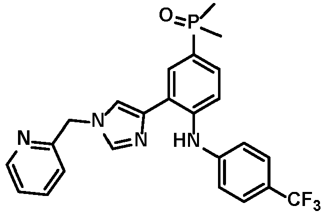 |
| 3 | 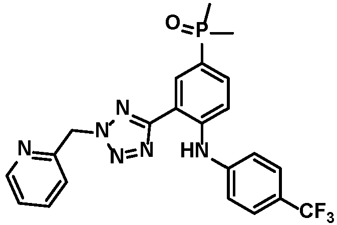 |
| 4 | 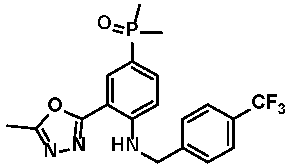 |
| 5 | 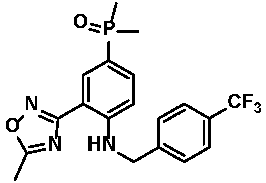 |
| 6 | 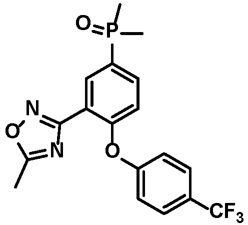 |
| 7 | 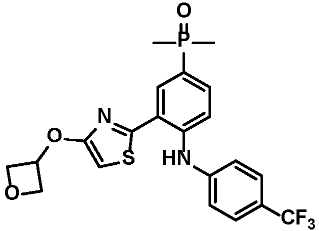 |
| 8 | 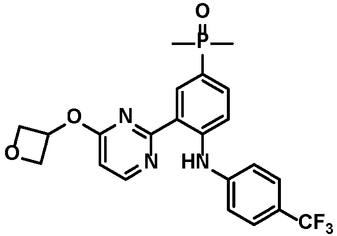 |
| 9 | 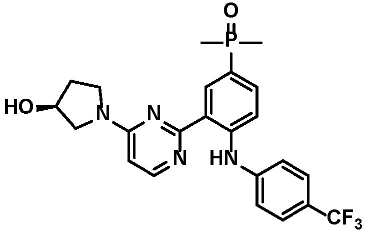 |
| 10 | 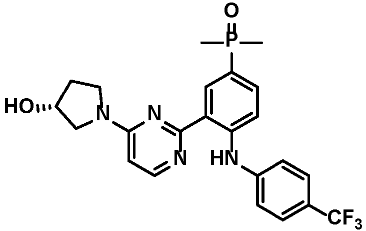 |
| 11 | 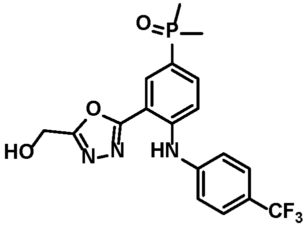 |
| 12 | 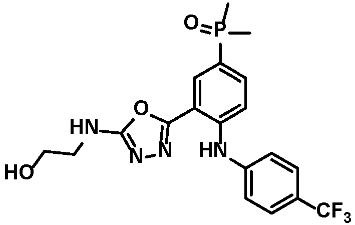 |
| 13 | 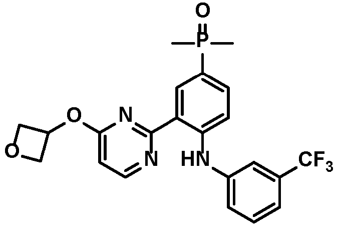 |
| 14 | 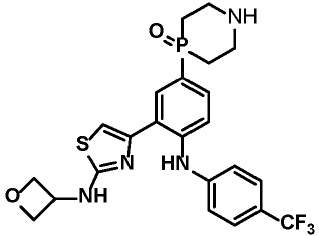 |
| 15 | 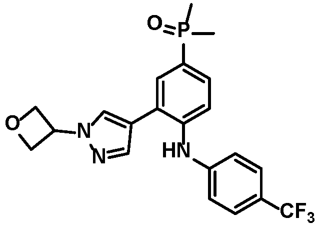 |
| 16 | 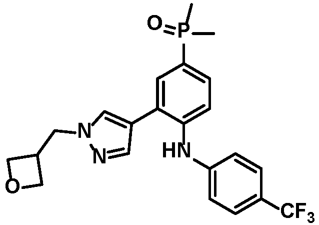 |
| 17 | 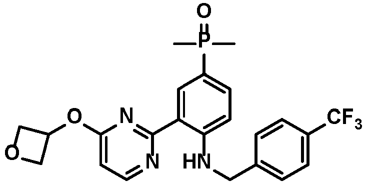 |
| 18 | 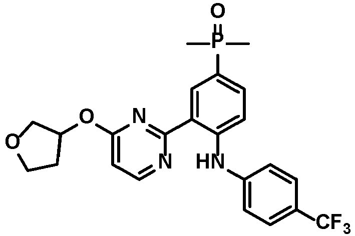 |
| 19 | 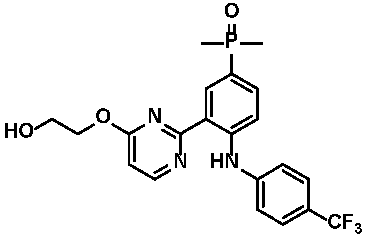 |
| 20 | 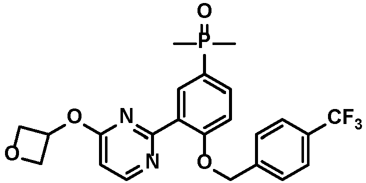 |
| twenty one | 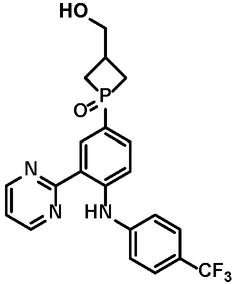 |
| twenty two | 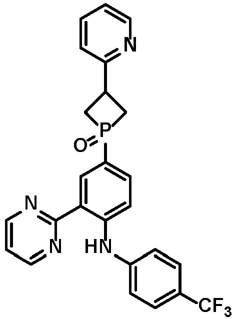 |
| twenty three | 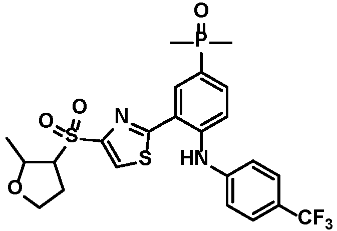 |
| twenty four | 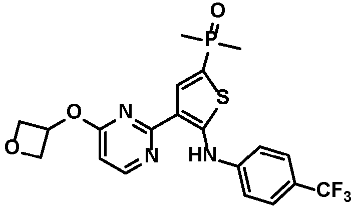 |
| 25 | 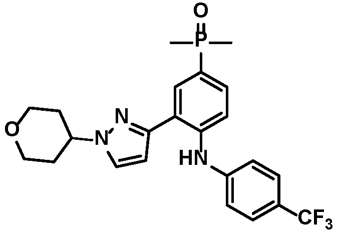 |
| 26 | 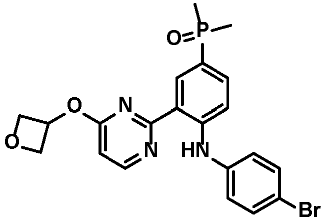 |
| 27 | 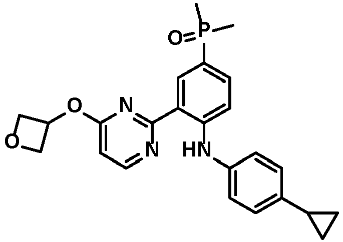 |
| 28 | 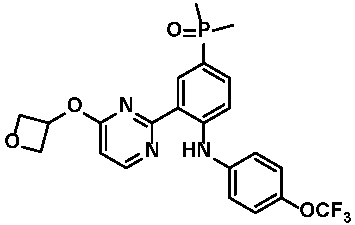 |
| 29 | 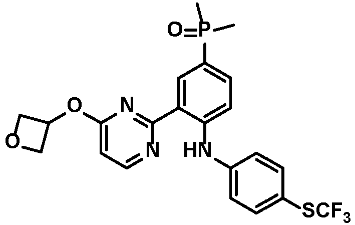 |
| 30 | 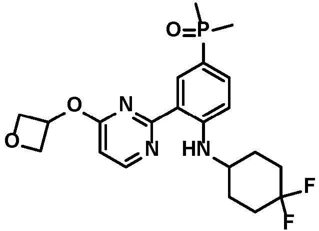 |
| 31 | 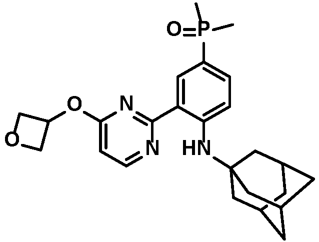 |
| 32 | 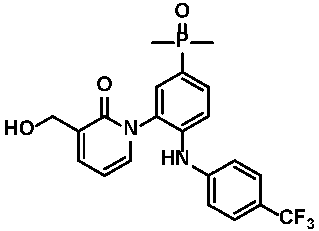 |
| 33 | 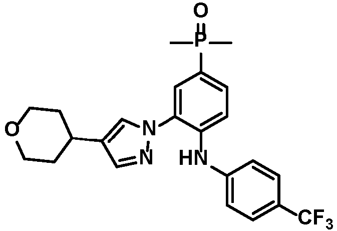 |
| 34 | 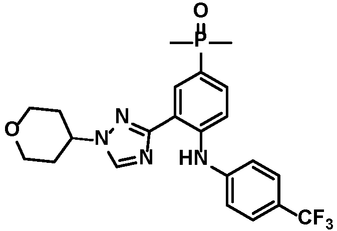 |
| 35 | 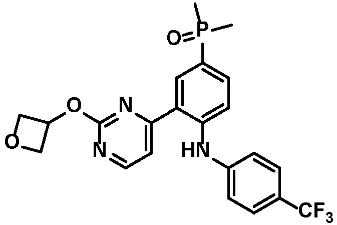 |
| 36 | 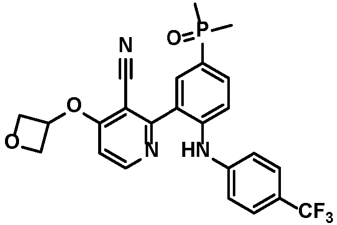 |
| 37 | 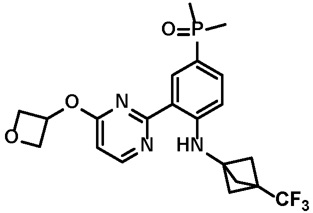 |
| 38 | 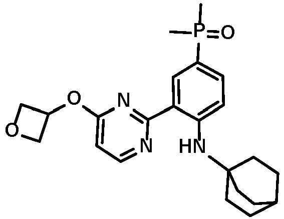 |
| 39 | 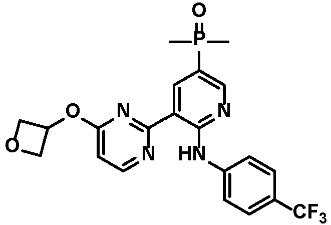 |
| 40 | 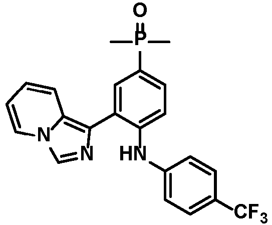 |
| 41 | 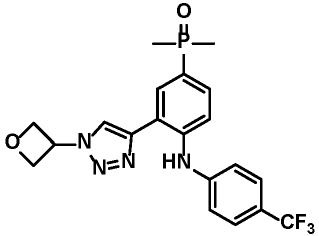 |
| 42 | 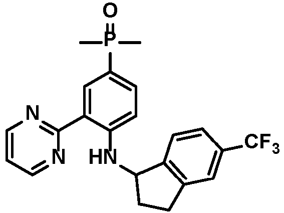 |
| 43 | 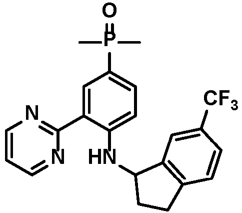 |
| 44 | 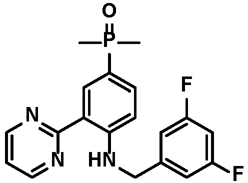 |
| 45 | 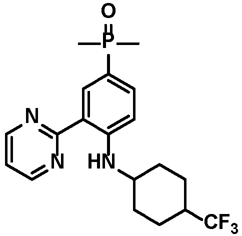 |
| 46 | 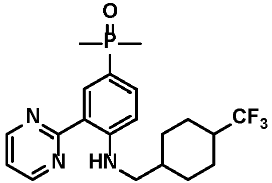 |
| 47 | 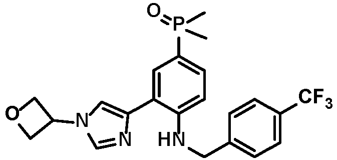 |
| 48 | 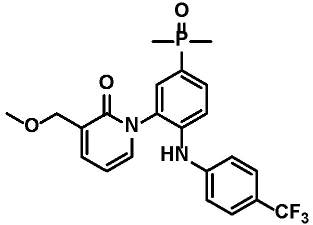 |
| 49 | 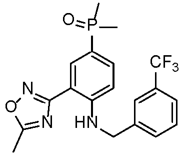 |
| 50 | 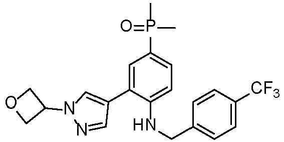 |
| 51 | 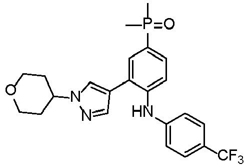 |
| 52 | 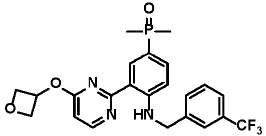 |
| 53 | 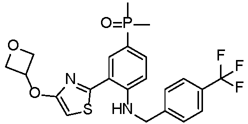 |
| 54 | 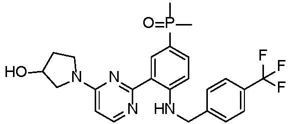 |
| 55 | 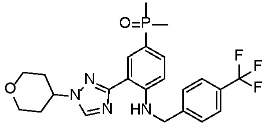 |
| 56 | 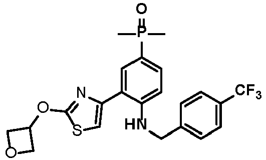 |
| 57 | 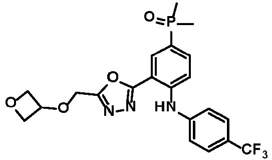 |
| 58 | 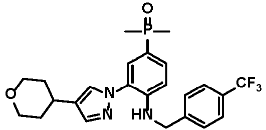 |
| 59 | 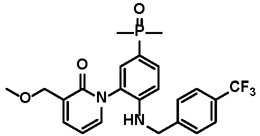 |
| 60 | 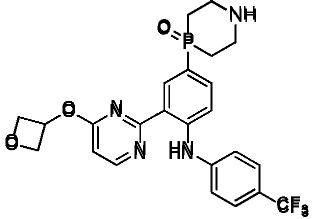 |
| 61 | 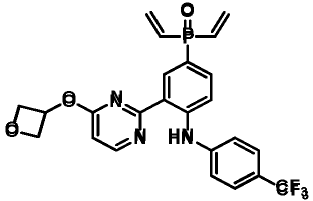 |
| 62 | 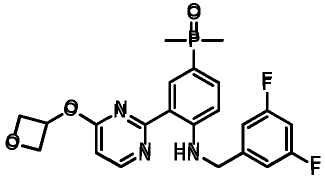 |
| 63 | 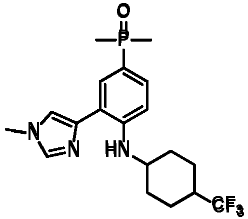 |
| 64 | 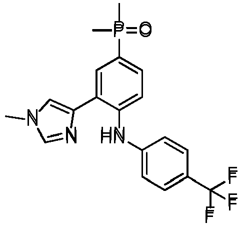 |
| 65 | 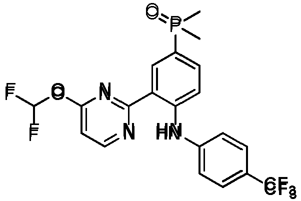 |
| 66 | 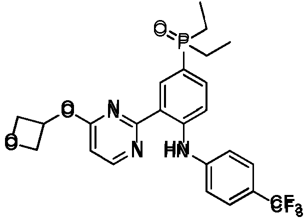 |
| 67 | 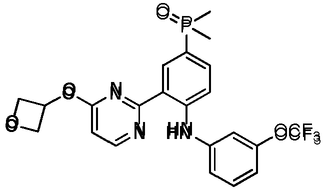 |
| 68 | 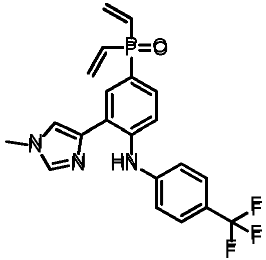 |
| 69 | 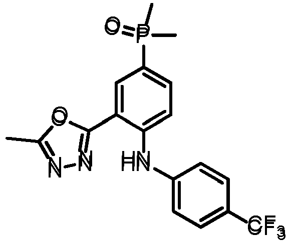 |
| 70 | 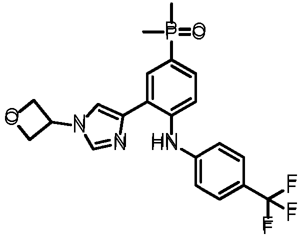 |
| 71 | 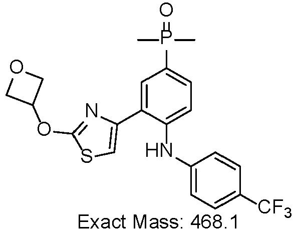 |
| 72 | 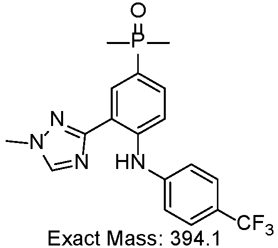 |
| 73 | 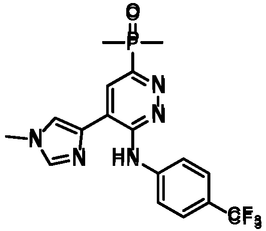 |
| 74 | 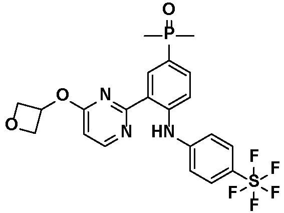 |
| 75 | 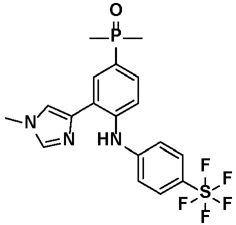 |
| 76 | 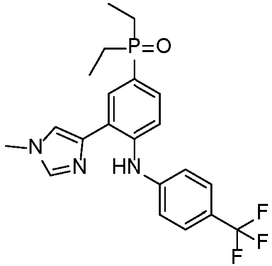 |

The second aspect of the present invention provides pharmaceutical compositions, which comprise the compounds or the pharmaceutically acceptable salts, stereoisomers, solvates or prodrugs thereof described in the first aspect of the present invention, and pharmaceutically acceptable carriers.

The third aspect of the present invention provides uses of the compounds or the pharmaceutically acceptable salts, stereoisomers, solvates or prodrugs thereof according to the first aspect of the present invention, or the pharmaceutical compositions according to the second aspect of the present invention, for preparing drugs or pharmaceutical compositions, wherein the drugs or pharmaceutical compositions are used for one or more uses selected from the following:
(i) binding to TEAD; preferably binding to the palmitoyl pocket of TEAD;
(ii) inhibiting TEAD transcription levels;
(iii) inhibiting/blocking YAP-TEAD binding;
(iv) regulating the Hippo signaling pathway; and
( v) treating diseases associated with increased TEAD transcription levels and/or YAP phosphorylation disorders and/or Hippo signaling pathway disorders.

The fourth aspect of the present invention provides uses of the compound or pharmaceutically acceptable salts, stereoisomers, solvates or prodrugs thereof according to the first aspect of the present invention, or the pharmaceutical compositions according to the second aspect of the present invention, for:
(i) binding to TEAD; preferably binding to the palmitoyl pocket of TEAD;
(ii) inhibiting TEAD transcription levels;
(iii) inhibiting/blocking YAP-TEAD binding;
(iv) regulating the Hippo signaling pathway;
(v) treating diseases associated with increased TEAD transcription levels and/or YAP phosphorylation disorders and/or Hippo signaling pathway disorders; and/or
(vi) preparing drugs for treating diseases associated with increased TEAD transcription levels and/or YAP phosphorylation disorders and/or Hippo signaling pathway disorders.

A fifth aspect of the present invention provides a method for treating diseases associated with increased TEAD transcription levels and/or YAP phosphorylation disorders and/or Hippo signaling pathway disorders, the method comprising the steps of:
administering the compounds according to the first aspect of the present invention or pharmaceutically acceptable salts, stereoisomers, solvates or prodrugs thereof to a subject, or
administering the pharmaceutical compositions according to the second aspect of the present invention is to a subject.

Compared with the prior art, the present invention has at least the following advantages:
(1) The compounds and pharmaceutically acceptable salts, stereoisomers, solvates or prodrugs thereof provided in the first aspect of the present invention or the pharmaceutical compositions provided in the second aspect of the present invention, by targeting the palmitoyl pocket of TEAD, effectively inhibit/block YAP-TEAD binding and inhibit/block YAP-TEAD transcription function, thereby preventing and/or treating diseases associated with increased TEAD transcription level and/or YAP phosphorylation disorders and/or Hippo signaling pathway disorders;
(2) The compounds and pharmaceutically acceptable salts, stereoisomers, solvates or prodrugs thereof provided in the first aspect of the present invention or the pharmaceutical compositions provided in the second aspect of the present invention have a strong affinity for TEAD protein;
( 3) The compounds and pharmaceutically acceptable salts, stereoisomers, solvates or prodrugs thereof provided in the first aspect of the present invention or the pharmaceutical compositions provided in the second aspect of the present invention have excellent metabolic stability and pharmacokinetic properties.

It should be understood that within the scope of the present invention, the above-mentioned technical features of the present invention and the technical features specifically described below (such as embodiments) can be combined with each other to form a new or preferred technical solution. Due to space limitations, they will not be described one by one here.

### BRIEF DESCRIPTION OF THE DRAWINGS

One or more embodiments are exemplarily described by the pictures in the corresponding drawings, and these exemplary descriptions do not constitute limitations on the embodiments.
FIG1 is a mass spectrum of compound 68 after incubation with TEAD1 protein in an embodiment of the present invention;
FIG2 is a mass spectrum of compound 68 after incubation with TEAD4 protein in an embodiment of the present invention;
FIG3a is a graph showing the in vitro liver microsome stability test results of Compound 8 in an embodiment of the present invention, wherein the mouse half-life is 75.4 min, the human half-life is 118 min, the rat half-life is 157 min, and the dog half-life is 177 min;
FIG3b is a graph showing the in vitro liver microsome stability test results of Compound 1 in an embodiment of the present invention, wherein the mouse half-life is 93.8 min and the human half-life is 359 min;
FIG3c is a graph showing the in vitro liver microsome stability test results of compound 9 in an embodiment of the present invention, with a mouse half-life of 85.6 min and a human half-life of 29.3 min;
FIG3d is a graph showing the in vitro liver microsome stability test results of compound 10 in an embodiment of the present invention, with a mouse half-life of 105 min and a human half-life of 50.8 min;
FIG3e is a graph showing the in vitro liver microsome stability test results of compound 17 in an embodiment of the present invention, with a mouse half-life of 33.9 min, a rat half-life of 48.0 min, a dog half-life of 141 min, and a human half-life of 107 min;
FIG3f is a graph showing the in vitro liver microsome stability test results of compound 64 in an embodiment of the present invention, wherein the left graph is for mice, the mouse half-life is >186 min, and the right graph is for humans, the human half-life is >186 min;
FIG3g is a graph showing the in vitro liver microsome stability test results of compound 28 in an embodiment of the present invention, wherein the left graph is for mice, the mouse half-life is >186 min, the right graph is for humans, the human half-life is >186 min;
FIG3h is a graph showing the in vitro liver microsome stability test results of compound 68 in an embodiment of the present invention, wherein the left graph is for mice, the mouse half-life is >186 min, the right graph is for humans, the human half-life is >186 min;
FIG4a is a graph showing the average blood drug concentration of compound 17 in an embodiment of the present invention after intragastric administration (10.0 mg/kg) and injection administration (1.0 mg/kg) to mice;
FIG4b is a graph showing the average blood drug concentration of compound 64 in an embodiment of the present invention after intragastric administration (10.0 mg/kg) and injection administration (1.0 mg/kg) to mice;
FIG4c is a semi-logarithmic graph of the average blood drug concentration of compound 64 in an embodiment of the present invention after intragastric administration (10.0 mg/kg) and injection administration (1.0 mg/kg) to mice;
FIG5 is a schematic diagram of a competitive thiol binding experiment of compounds in an embodiment of the present invention;
FIG6a is a graph showing the average blood concentration of SD in rats of compound 17 in an embodiment of the present invention;
FIG6b is a graph showing a semi-logarithm of the average blood concentration of SD in rats of compound 17 in an embodiment of the present invention.

### DETAILED DESCRIPTION OF EMBODIMENTS

The present invention provides a class of small molecule compounds with phosphorylated aryl structures, preferably small molecule compounds with multi-substituted phosphorylated aromatic structures, which can bind to the palmitoyl pocket of TEAD, thereby inhibiting/blocking YAP-TEAD binding, inhibiting/blocking YAP-TEAD transcription function, and blocking the transcriptional activity of the Hippo signaling pathway, and is expected to treat diseases with the Hippo signaling pathway disorders and overactivation of YAP1 and reverse the drug resistance and recurrence problems of existing disease treatment drugs.

### Compounds

In one aspect, an embodiment of the present invention provides a compound having a structure shown in general formula (I), 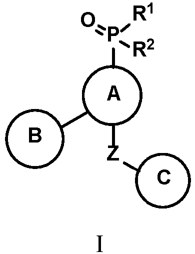
or pharmaceutically acceptable salts, stereoisomers, solvates or prodrugs thereof,
wherein Z is -CH₂ -, -NH(CH₂ )n-, -O(CH₂ )n-, -S-, -SO-, -SO₂-, each n is independently an integer of 0 to 3;
R¹ is C₁₋₆ alkyl, C₂₋₆ alkenyl, C₁₋₆ alkyl with at least one hydrogen replaced by R¹⁻¹, C₂₋₆ alkenyl with at least one hydrogen replaced by R¹⁻¹;
R² is C₁₋₆ alkyl, C₂₋₆ alkenyl, C₁₋₆ alkyl with at least one hydrogen replaced by R¹⁻¹, C₂₋₆ alkenyl with at least one hydrogen replaced by R¹⁻¹;
or, R¹ and R² are bonded to form a 4 to 6-membered ring or a 4 to 6-membered ring with at least one hydrogen replaced by R¹⁻¹, wherein each R¹⁻¹ is independently amino, hydroxyl, hydroxyl-substituted C₁₋₆ alkyl, and 5- or 6-membered monocyclic heteroaryl;
Ring A is 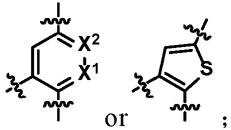 wherein X¹ is CH or N, X² is CH or N;
Ring B is a 5 to 10-membered heteroaryl, 5 to 10-membered heteroaryl with at least one hydrogen replaced by 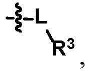 5 to 10-membered heteroaryl ketone group, 5 to 10-membered heteroaryl ketone group with at least one hydrogen replaced by 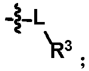
wherein L is none, -CH₂-, -NH-, -O-, -CH₂O-, -S-, -SO-, or -SO₂-;
each R³ is independently none, hydroxyl, cyano 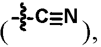 C₁₋₆ alkyl, C₁₋₆ alkyl with at least one hydrogen replaced by R³⁻¹, amino, amino with at least one hydrogen replaced by R³⁻¹, 3 to 6-membered heterocycloalkyl, 3 to 6-membered heterocycloalkyl with at least one hydrogen replaced by R³⁻¹, 5- or 6-membered monocyclic heteroaryl, 5- or 6-membered monocyclic heteroaryl with at least one hydrogen replaced by R³⁻¹;
wherein each R³⁻¹ is independently hydroxyl, C₁₋₄ alkyl with at least one hydrogen replaced by hydroxyl , C₁₋₄ alkoxy, halogen;
Ring C is phenyl, phenyl with at least one hydrogen replaced by R⁴, 3 to 7-membered monocyclic alkyl, 3 to 7-membered monocyclic alkyl with at least one hydrogen replaced by R⁴ , C₅₋₁₂ bridged bicycloalkyl, C₅₋₁₂ bridged bicycloalkyl with at least one hydrogen replaced by R⁴, C₁₀₋₂₀ bridged tricycloalkyl, C₁₀₋₂₀ bridged tricycloalkyl with at least one hydrogen replaced by R⁴, 8 to 10-membered benzocycloalkyl, 8 to 10-membered benzocycloalkyl with at least one hydrogen replaced by R⁴;
wherein each R⁴ is independently C₁₋₄ alkyl, C₁₋₄ alkyl with at least one hydrogen replaced by halogen, halogen, 3 to 6-membered cycloalkyl, C₁₋₄ alkoxy, C₁₋₄ alkoxy with at least one hydrogen replaced by halogen, C₁₋₄ alkyl thiol, C₁₋₄ alkyl thiol with at least one hydrogen replaced by halogen, halogen-substituted thiol.
**R¹ and R²**
In a preferred embodiment of the present invention, R¹ is C₁₋₄ alkyl, more preferably methyl or ethyl, for example methyl;
R² is C₁₋₄ alkyl, more preferably methyl or ethyl, for example methyl.

In a preferred embodiment of the present invention, R¹ is C₂₋₄ alkenyl, more preferably ethenyl, 1-propenyl, 2-propenyl, such as ethenyl ;
R² is C ₂₋₄ alkenyl, more preferably ethenyl, 1-propenyl, 2-propenyl, for example ethenyl.

In other preferred embodiments of the present invention, R¹ and R² are bonded to form a 4 to 6-membered ring or a 4 to 6-membered ring with at least one hydrogen replaced by R^{1-1 ,} wherein each R¹⁻¹ is independently amino, hydroxyl, hydroxyl-substituted C₁₋₆ alkyl, or 5- or 6-membered monocyclic heteroaryl.

In some other preferred embodiments of the present invention, each R¹⁻¹ is independently amino, hydroxy, hydroxy-substituted methyl, hydroxy -substituted ethyl, or 5- or 6-membered nitrogen-containing monocyclic heteroaryl (eg, pyridyl).

In a preferred embodiment of the present invention, R¹ is C₁₋₄ alkyl with one hydrogen atom replaced by R¹⁻¹, wherein R¹⁻¹ is amino, hydroxyl or 5- or 6-membered nitrogen-containing monocyclic heteroaryl. More preferably, R¹ is methyl with one hydrogen atom replaced by R¹⁻¹ or ethyl with one hydrogen atom replaced by R¹⁻¹, wherein R¹⁻¹ is amino, hydroxyl or 6-membered nitrogen-containing monocyclic heteroaryl. More preferably, R¹ is methyl with one hydrogen atom replaced by R¹⁻¹ or ethyl with one hydrogen atom replaced by R¹⁻¹, wherein each R¹⁻¹is independently amino, hydroxyl or pyridyl. For example

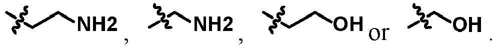

In a preferred embodiment of the present invention, R² is C₁₋₄ alkyl with one hydrogen atom replaced by R¹⁻¹, wherein R¹⁻¹ is amino, hydroxyl or 5- or 6-membered nitrogen-containing monocyclic heteroaryl. More preferably, R² is methyl with one hydrogen atom replaced by R¹⁻¹ or ethyl with one hydrogen atom replaced by R¹⁻¹, wherein R¹⁻¹ is amino, hydroxyl or 6-membered nitrogen-containing monocyclic heteroaryl. More preferably, R¹ is methyl with one hydrogen atom replaced by R¹⁻¹ or ethyl with one hydrogen atom replaced by R¹⁻¹, wherein each R¹⁻¹ is independently amino, hydroxyl or pyridyl. For example

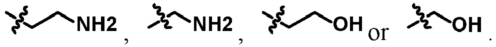

In a preferred embodiment of the present invention, R¹ and R² are bonded to each other to form a 4 to 6-membered ring. More preferably , when R¹ is ethyl and R² is 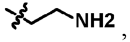 R¹ and R² are bonded to each other to form

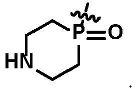

In a preferred embodiment of the present invention, R¹ and R² are bonded to each other to form a 4 to 6-membered ring with at least one hydrogen replaced by hydroxyl. For example, when R¹ is 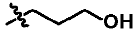 and R² is methyl, the carbon at the β position of R¹ is bonded to R² to form

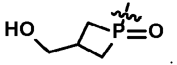

### Z

In a preferred embodiment of the present invention, Z is -CH₂-, - NH(CH₂)n-, -O(CH₂) n-, -S-, -SO-, or -SO₂- , and each n is independently an integer of 0 to 3.

In a preferred embodiment of the present invention, n is 1.

In a preferred embodiment of the present invention, Z -CH₂-, -NH(CH₂)-, - O(CH₂)-, -S-, -SO-, or -SO₂-.

In a preferred embodiment of the present invention, when Z contains a heteroatom (N, O or S), the heteroatom in Z is connected to ring A via a covalent bond. For example, when Z is -NH(CH₂)-, the N atom is connected to 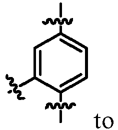 form 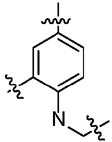 via a covalent bond. For example, when Z is -O(CH₂)-, the O atom is connected to 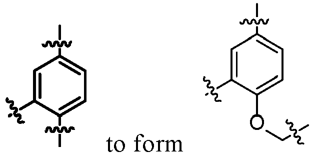 via a covalent bond.

### Ring A

In a preferred embodiment of the present invention, Ring A is

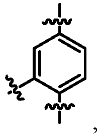

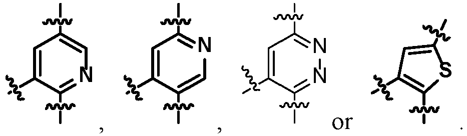

### Ring B

In a preferred embodiment of the present invention, Ring **B** is a 5- or 6-membered nitrogen-containing monocyclic heteroaryl, and the 5- or 6-membered nitrogen-containing monocyclic heteroaryl is 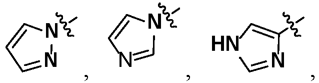 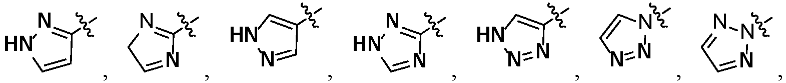 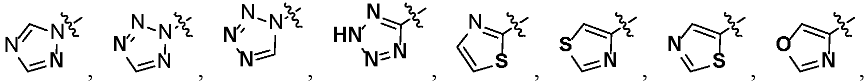 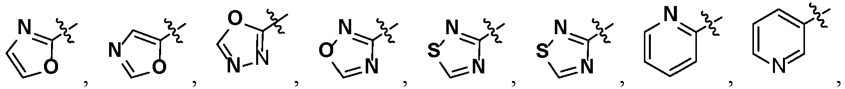 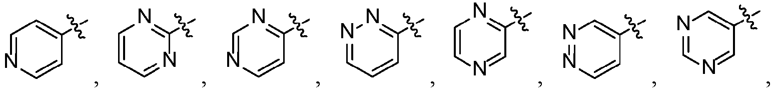 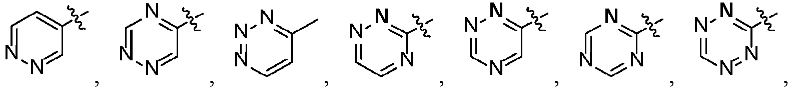 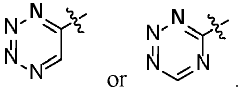 More preferably, the 5- or 6 - membered nitrogen-containing monocyclic heteroaryl is

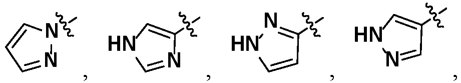

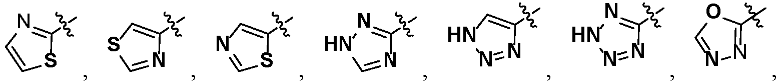

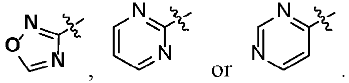

In a preferred embodiment of the present invention, Ring **B** is 5- or 6-membered nitrogen-containing monocyclic heteroaryl with at least one hydrogen atom replaced by

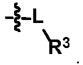

In a preferred embodiment of the present invention, Ring **B** is 5-membered nitrogen-containing monocyclic heteroaryl with at least one hydrogen replaced by 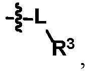 and the 5-membered nitrogen-containing monocyclic heteroaryl with at least one hydrogen replaced by 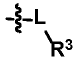 is preferably: 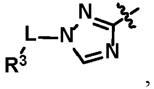 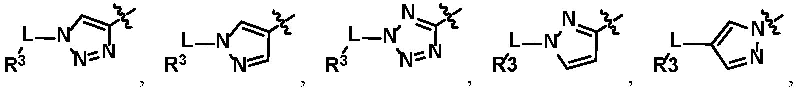 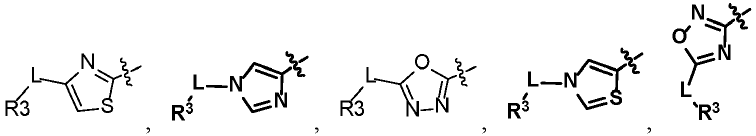 or 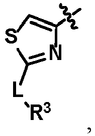 wherein each L is independently none, -CH₂-, -NH-, -O-, -S-, -SO-, or - SO₂-. When L is none, the 5-membered nitrogen-containing monocyclic heteroaryl with at least one hydrogen replaced by 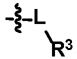 is preferably 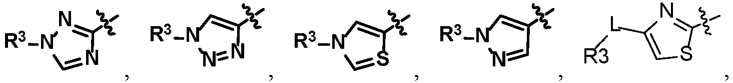 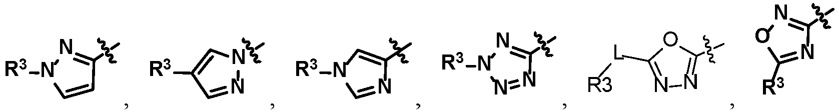 or

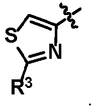

In a preferred embodiment of the present invention, Ring **B** is 6-membered nitrogen-containing monocyclic heteroaryl with at least one hydrogen replaced by 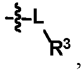 which is preferably 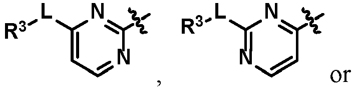 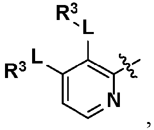 wherein each L is independently none, -CH₂-, -NH-, -O-, -S-, - SO-, or -SO₂-. When L is none, the 6-membered nitrogen-containing monocyclic heteroaryl with at least one hydrogen replaced by 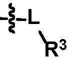 is preferably 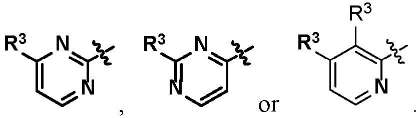 In a preferred example, when the 6-membered nitrogen-containing monocyclic heteroaryl with at least one hydrogen replaced by 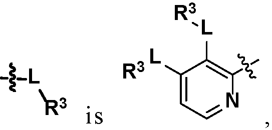 wherein are cyano 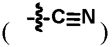 and 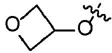 respectively, Ring B is

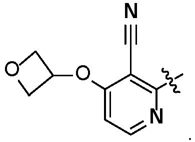

In a preferred embodiment of the present invention, Ring **B** is 8 to 10-membered nitrogen-containing fused ring heteroaryl, which is preferably 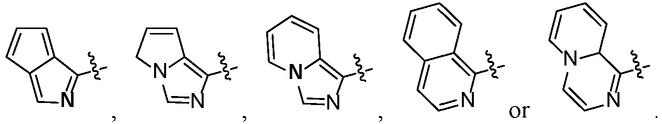 More preferably, the 8 to 10-membered nitrogen-containing fused ring heteroaryl is preferably a 9-membered nitrogen-containing fused ring heteroaryl, for example

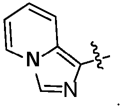

In a preferred embodiment of the present invention, Ring **B** is 8 to 10-membered nitrogen-containing fused ring heteroaryl with at least one hydrogen replaced by 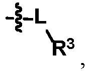 wherein each L is independently none, -CH₂-, -NH-, -O-, - S-, -SO-, or -SO₂-. More preferably, the 8 to 10-membered nitrogen-containing fused ring heteroaryl with at least one hydrogen replaced by 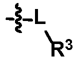 is 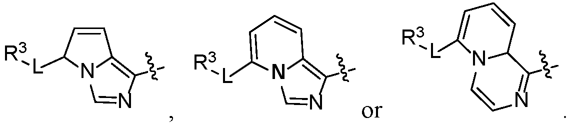 When L is none, the 8 to 10-membered nitrogen-containing fused ring heteroaryl with at least one hydrogen replaced by 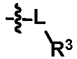 is preferably

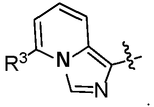

In a preferred embodiment of the present invention, Ring **B** is a 5 to 10-membered heteroaromatic ketone group. More preferably, Ring **B is** a 5- or 6-membered heteroaromatic ketone group. More preferably, Ring **B** is a 6-membered heteroaromatic ketone group. More preferably, Ring **B** is a pyridone. More preferably, Ring B is 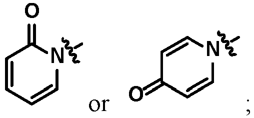 for example

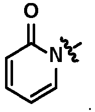

In a preferred embodiment of the present invention, Ring **B is** a 5 to 10-membered heteroaromatic ketone group with at least one hydrogen replaced by 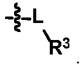 More preferably, Ring **B** is a 5- or 6-membered heteroaromatic ketone group with at least one hydrogen replaced by 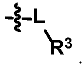 More preferably, Ring **B** is a 6-membered heteroaromatic ketone group with at least one hydrogen replaced by 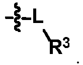 More preferably, Ring **B** is a pyridone with at least one hydrogen replaced by 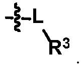 More preferably, Ring **B** is 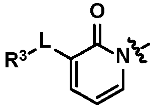 or 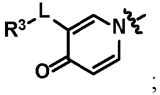 for example

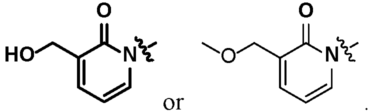

### R³

In the present invention, R³ is connected to Ring **B** by connecting with L to form 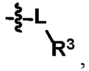 wherein each L is independently none, -CH₂-, -NH-, -O-, -CH₂O-, -S-, -SO-, or -SO₂-. It should be understood that various groups formed by the combination of each L and R³ are within the scope of the present invention

In a preferred embodiment of the present invention, R³ is C₁₋₆ alkyl, more preferably C₁₋₄ alkyl, more preferably methyl, ethyl, n-propyl, isopropyl, n-butyl, isobutyl ortert-butyl, for example, methyl.

In order to obtain a compound with higher affinity for TEAD protein, in a preferred embodiment of the present invention, R³ is 3 to 6-membered heterocycloalkyl, more preferably 3 to 6-membered nitrogen heterocycloalkyl or 3 to 6-membered oxygen heterocycloalkyl. More preferably, the 3 to 6-membered nitrogen heterocycloalkyl is preferably 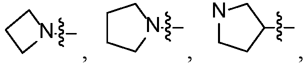 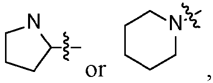 for example 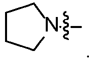 More preferably, the 3 to 6-membered oxygen heterocycloalkyl is preferably 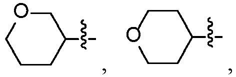 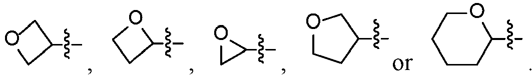 More preferably, the 3- to 6-membered oxygen heterocycloalkyl is O-heterocyclobutanyl and O-heterocyclopentanyl, for example 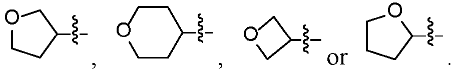 In addition, compared with nitrogen heterocycloalkyl, when R³ is 3- to 6-membered oxygen heterocycloalkyl, the compound has a stronger affinity for TEAD protein.

In a more preferred embodiment, when the L linker connected to R³ contains O and S, for example, when the L atom connected to R³ is -O-, -CH₂O-, -S-, -SO-, or -SO₂-, the compound has a stronger affinity for TEAD protein. More preferably, when the L linker connected to R³ is -O- or -CH₂O-, the compound has a stronger affinity for TEAD protein.

In a more preferred embodiment, when the L atom connected to R³ is -O- or -S-, the compound has a stronger affinity for TEAD protein. More preferably, when the L atom connected to R³ is -O-, that is, when 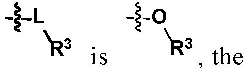 compound has a stronger affinity for TEAD protein.

In a more preferred embodiment, when the L atom connected to R ³ is O and R³ is 3 to 6-membered oxygen heterocycloalkyl, the compound has a stronger affinity for TEAD protein.

In a preferred embodiment of the present invention, R³ is 5- or 6-membered monocyclic heteroaryl, which is 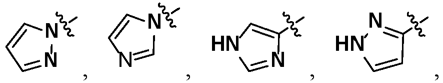 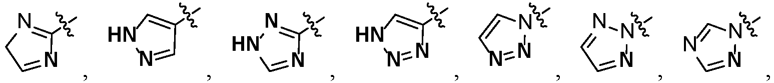 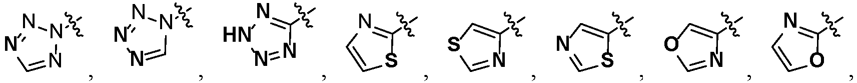 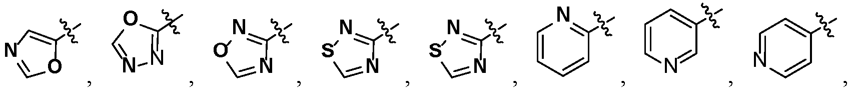 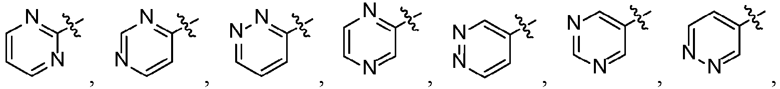 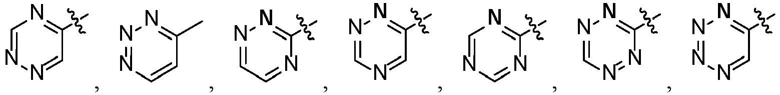 or 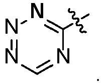 More preferably, R³ is 6-membered nitrogen-containing monocyclic heteroaryl, and more preferably, R³ is 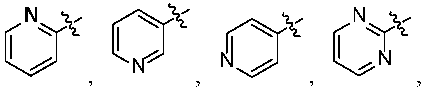 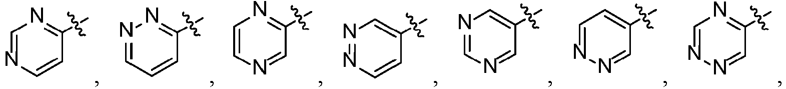 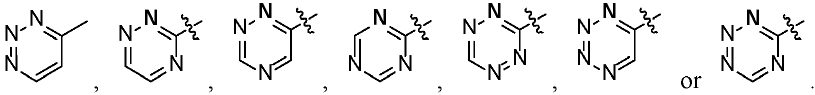 For example, ^{R3} is

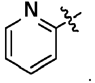

In a preferred embodiment of the present invention, R ³ is amino oramino with at least one hydrogen replaced by R³⁻¹ , for example

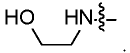

In a preferred embodiment of the present invention, R³ is hydroxyl.

In a preferred embodiment of the present invention, R³ is cyano

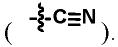

In a preferred embodiment of the present invention, R³ is C₁₋₆ alkyl with at least one hydrogen replaced by R³⁻¹. More preferably, R³ is C₁₋₄ alkyl with at least one hydrogen replaced by R³⁻¹. More preferably, R³ is methyl with at least one hydrogen replaced by R³⁻¹, ethyl with at least one hydrogen replaced by R³⁻¹, n-propyl with at least one hydrogen replaced by R³⁻¹, or isopropyl with at least one hydrogen replaced by R³⁻¹. More preferably, R³ is methyl with at least one hydrogen replaced by methoxy, ethyl with at least one hydrogen replaced by methoxy, or methyl with at least one hydrogen replaced by hydroxyl, or methyl with at least one hydrogen replaced by fluorine, for example 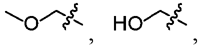 difluoromethyl or trifluoromethyl.

In a preferred embodiment of the present invention, R³ is 3 to 6-membered heterocycloalky with at least one hydrogen replaced by R³⁻¹. More preferably, R³ is 3 to 6-membered heterocycloalkyl with at least one hydrogen replaced by R³⁻¹. More preferably, R³ is 5 or 6-membered heterocycloalkyl with at least one hydrogen replaced by R³⁻¹. More preferably, R³ is 5-membered nitrogen heterocycloalkyl with at least one hydrogen replaced by R³⁻¹ or 5-membered oxygen heterocycloalkyl with at least one hydrogen replaced by R³⁻¹. More preferably, R ³ is 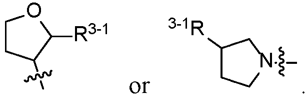 More preferably, R³ is 5-membered nitrogen heterocycloalkyl with at least one hydrogen replaced by hydroxyl or methyl or 5-membered oxygen heterocycloalkyl with at least one hydrogen replaced by hydroxyl or methyl; for example

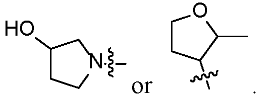

In a preferred embodiment of the present invention, R³ is 5- or 6-membered monocyclic heteroaryl with at least one hydrogen replaced by R³⁻¹. More preferably, R³ is 5- or 6-membered nitrogen-containing monocyclic heteroaryl with at least one hydrogen replaced by R³⁻¹. More preferably, R³ is 6-membered nitrogen-containing monocyclic heteroaryl group with at least one hydrogen replaced by R³⁻¹. More preferably, R³ is pyridyl with at least one hydrogen replaced by R³⁻¹ ; for example

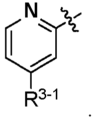

### R³⁻¹

In a preferred embodiment of the present invention, R³⁻¹ is hydroxyl.

In a preferred embodiment of the present invention, R³⁻¹ is halogen, more preferably fluorine, chlorine, bromine or iodine; for example, fluorine.

In a preferred embodiment of the present invention, R³⁻¹ is C₁₋₄ alkyl with at least one hydrogen replaced by hydroxyl. More preferably, R ³⁻¹ is methyl with one hydrogen replaced by hydroxyl or ethyl with one hydrogen replaced by hydroxyl, for example

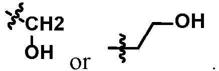

In a preferred embodiment of the present invention, R³⁻¹ is C₁₋₄ alkoxy, more preferably methoxy, ethoxy or propoxy, for example methoxy.

### Ring C

In a preferred embodiment of the present invention, Ring C is phenyl or phenyl with at least one hydrogen replaced by R⁴. More preferably, the phenyl with at least one hydrogen replaced by R^{4 is} 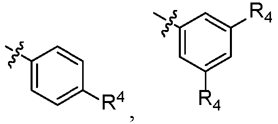 or 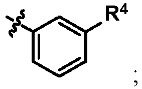 for example

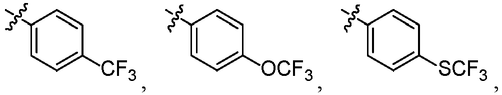

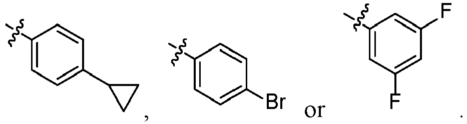

To obtain compounds with higher affinity for TEAD protein, in a preferred embodiment of the present invention, Z is -NH(CH₂ )n-, -O(CH₂ )n-, and Ring C is phenyl or phenyl with at least one hydrogen replaced by R⁴. More preferably, Z-ring C is 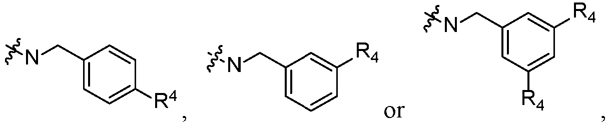 For example:

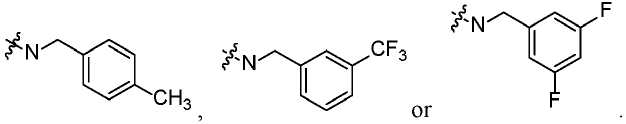

When Ring C is phenyl or phenyl with at least one hydrogen replaced by R⁴, the compound of the present invention has relatively high metabolic kinetics.

In a preferred embodiment of the present invention, Ring C is 3 to 7-membered monocycloalkyl. More preferably, Ring C is 4 to 6-membered monocycloalkyl. More preferably, Ring C is cyclohexyl, cyclopentyl or cyclobutyl; for example, cyclohexyl.

In a preferred embodiment of the present invention, Ring C is 3 to 7-membered monocycloalkyl with at least one hydrogen replaced by R⁴, more preferably, 4 to 6-membered monocycloalkyl with at least one hydrogen replaced by R⁴. More preferably, the 4 to 6-membered monocycloalkyl with at least one hydrogen replaced by R⁴ is 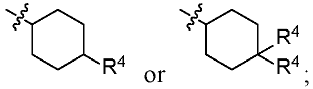 for example

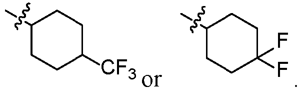

In order to obtain a compound with higher affinity for TEAD protein, in a preferred embodiment of the present invention, Z is -NH(CH₂ )n-, -O(CH₂ )n-, and Ring C is 3 to 7-membered monocycloalkyl or 3 to 7-membered monocycloalkyl with at least one hydrogen replaced by R⁴. More preferably, Ring C is 4 to 6-membered monocycloalkyl or 4 to 6-membered monocycloalkyl with one hydrogen replaced by R⁴. For example, Z-Ring C is 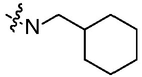 or

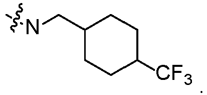

In a preferred embodiment of the present invention, Ring C is C₅₋₁₂ bridged bicycloalkyl or C₅₋₁₂ bridged bicycloalkyl with at least one hydrogen replaced by R⁴. More preferably, Ring C is C₅₋₈ bridged bicycloalkyl or C₅₋₁₂ with at least one hydrogen replaced by R⁴. More preferably, Ring C is bicyclo[1.1.1]pentyl, bicyclo[2.2.2]octyl, bicyclo[1.1.1]pentyl with at least one hydrogen replaced by R⁴, or bicyclo[2.2.2]octyl with at least one hydrogen replaced by R⁴, for example:

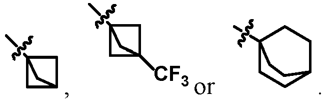

In a preferred embodiment of the present invention, Ring C is C ₁₀₋₂₀ bridged tricycloalkyl with at least one hydrogen replaced by R⁴. More preferably, Ring C is adamantyl or adamantyl with at least one hydrogen replaced by R⁴, for example:

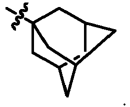

In a preferred embodiment of the present invention, Ring C is 8 to 10-membered benzocycloalkyl. More preferably, Ring C is benzocyclopentyl, benzocyclobutyl or benzocyclohexyl. For example, Ring C is

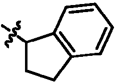

In a preferred embodiment of the present invention, Ring C is 8 to 10-membered benzocycloalkyl with at least one hydrogen replaced by R⁴, more preferably, benzocyclopentyl with at least one hydrogen replaced by R⁴, benzocyclobutyl with at least one hydrogen replaced by R⁴, or benzocyclohexyl with at least one hydrogen replaced by R⁴. More preferably, the 8 to 10-membered benzocycloalkyl with at least one hydrogen replaced by R⁴ is 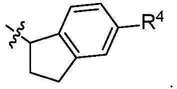 For example, Ring C is

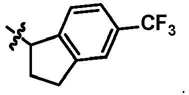

### R⁴

In a preferred embodiment of the present invention, R⁴ is C₁₋₄ alkyl or C₁₋₄ alkyl with at least one hydrogen replaced by halogen. More preferably, R⁴ is methyl, ethyl, n-propyl, isopropyl, n-butyl, isobutyl, tert-butyl, methyl with at least one hydrogen replaced by halogen, ethyl with at least one hydrogen replaced by halogen, n-propyl with at least one hydrogen replaced by halogen, or isopropyl with at least one hydrogen replaced by halogen. More preferably, R⁴ is trifluoromethyl or hexafluoroethyl, for example, trifluoromethyl.

In a preferred embodiment of the present invention, R⁴ is halogen. More preferably, R⁴ is fluorine, chlorine, bromine or iodine, for example, bromine or fluorine.

In a preferred embodiment of the present invention, R⁴ is 3 to 6-membered cycloalkyl. More preferably, R⁴ is cyclopropyl, cyclobutyl, cyclopentyl, or cyclohexyl, for example, cyclopropyl.

In a preferred embodiment of the present invention, R⁴ is C₁₋₄ alkoxy or C₁₋₄ alkoxy with at least one hydrogen replaced by halogen. More preferably, R⁴ is methoxy, ethoxy, n-propoxy, isopropoxy, trifluoromethoxy, or hexafluoroethoxy. More preferably, R⁴ is trifluoromethoxy or hexafluoroethoxy, for example, trifluoromethoxy.

In a preferred embodiment of the present invention, R4 is C₁₋₄ alkylthiol or C₁₋₄ alkylthiol with at least one hydrogen replaced by halogen. More preferably, R4 is methylthiol, ethylthiol, n-propylthiol, isopropylthiol, methylthiol with at least one hydrogen replaced by fluorine or ethylthiol with at least one hydrogen replaced by fluorine. More preferably, R⁴ is trifluoromethylthiol or hexafluoroethylthiol, for example, trifluoromethylthiol.

In a preferred embodiment of the present invention, R⁴ is thiol or thiol replaced by halogen, preferably thiol replaced by fluorine, such as pentafluorothiol.

Based on the beneficial effect of showing excellent binding ability to TEAD protein, in the compound of general formula (I) of the present invention,
Z is -CH2- , -NH-, or -O-;
R¹ is as described herein;
R² is as described herein;
or, R ¹ and R ² are bonded to form a 4- or 6-membered ring or a 4- or 6-membered ring with at least one hydrogen replaced by R¹⁻¹ ; R ¹⁻¹ is independently as described herein; (preferably, R¹ and R² are bonded to each other to form 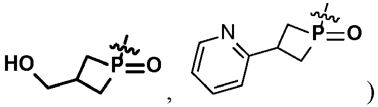
Ring A is 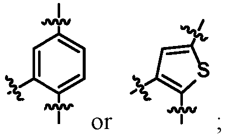
Ring B is 5-membered nitrogen-containing heterocycle, 6-membered nitrogen-containing heterocycle, 5-membered nitrogen-containing heterocycle with one hydrogen replaced by methyl, 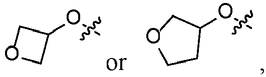 or 6-membered nitrogen-containing heterocycle with one hydrogen replaced by methyl, 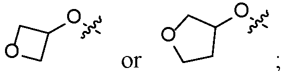
wherein the 5-membered nitrogen-containing heterocycle is 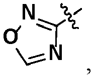 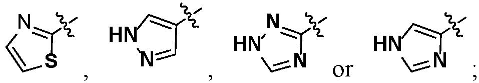 the 6-membered nitrogen-containing heterocycle is 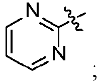 (more preferably, Ring B is 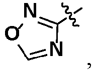 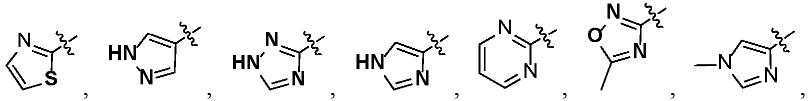 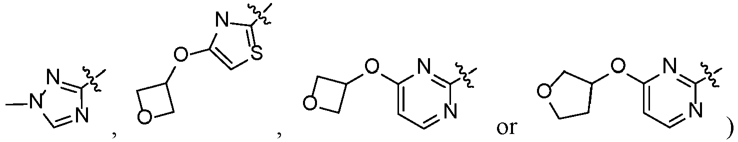
Ring C is phenyl, phenyl with at least one hydrogen replaced by R⁴, C₅₋₈ bridged bicycloalkyl , C₅₋₁₂ bridged bicycloalkyl with at least one hydrogen is replaced by R⁴, C₁₀₋₂₀ bridged tricycloalkyl, C₁₀₋₂₀ bridged tricycloalkyl with at least one hydrogen replaced by R⁴, 4 to 6-membered monocycloalkyl (preferably 6-membered monocycloalkyl), 4 to 6-membered monocycloalkyl with at least one hydrogen replaced by R⁴ ( preferably 6-membered monocycloalkyl with at least one hydrogen replaced by R⁴); each R⁴ is independently as described herein (preferably, R⁴ is trifluoromethyl, pentafluorosulfanyl, fluorine, chlorine, bromine, -SCF3 , -OCF3 or cyclopropyl).

As used herein, the term "alkyl" refers to a linear or branched saturated monovalent hydrocarbonyl, wherein the alkyl may be optionally substituted with one or more substituents. In a specific embodiment, the alkyl is a linear saturated monovalent hydrocarbonyl having 1 to 20 (C₁₋₂₀ ), 1 to 15 (C₁₋₁₅ ), 1 to 12 (C₁₋₁₂), 1 to 10 (C₁₋₁₀ ) or 1 to 6 (C₁₋₆ ) carbon atoms, or a branched saturated monovalent hydrocarbonyl having 3 to 20 (C ₃₋₂₀ ), 3 to 15 (C₃₋₁₅ ), 3 to 12 (C₃₋₁₂), 3 to 10 (C₃₋₁₀ ) or 3 to 6 (C₃₋₆ ) carbon atoms. The linear C₁₋₆ and branched C₃₋₆ alkyl groups used herein are also referred to as "low alkyl groups". Examples of alkyl groups include, but are not limited to, methyl, ethyl, propyl (including all isomeric forms), n-propyl, isopropyl, butyl (including all isomeric forms), n-butyl, isobutyl, tert-butyl, pentyl (including all isomeric forms) and hexyl (including all isomeric forms). For example, C₁₋₆ alkyl refers to a linear saturated monovalent hydrocarbon group having 1 to 6 carbon atoms or a branched saturated monovalent hydrocarbonyl having 3 to 6 carbon atoms. In one embodiment, the alkyl is an optionally substituted alkyl described elsewhere herein.

As used herein, the term "cycloalkyl" refers to a cyclic fully or partially saturated bridged and/or non-bridged hydrocarbonyl or ring system, which may be optionally substituted with one or more substituents. In specific embodiments, the cycloalkyl has 3 to 20 (C₃₋₂₀ ), 3 to 15 (C₃₋₁₅ ), 3 to 12 (C₃₋₁₂ ), 3 to 10 (C₃₋₁₀ ) or 3 to 7 (C₃₋₇ ) carbon atoms. Examples of cycloalkyl groups include, but are not limited to, cyclopropyl, cyclobutyl, cyclopentyl, cyclohexyl, cycloheptyl, decahydronaphthyl and adamantyl. In one embodiment, the cycloalkyl is an optionally substituted cycloalkyl described elsewhere herein.

As used herein, the term "monocycloalkyl" refers to a cycloalkyl group of a single ring. In some embodiments of the present invention, the monocycloalkyl is cyclopropyl (same as cyclopropane group), cyclobutyl (same as cyclobutane group), cyclopentyl (same as cyclopentane group), cyclohexyl (same as cyclohexane group).

As used herein, the term "bridged bicycloalkyl" refers to a ring system formed by two monocycloalkyl groups being bridged together. In some embodiments of the invention, the bridged bicycloalkyl is bicyclo[1.1.1] pentanyl or bicyclo[2.2.2] octanyl. In some embodiments of the invention, the bridged bicycloalkyl is optionally substituted with a substituent.

As used herein, the term "bridged tricycloalkyl" refers to a ring system formed by three monocycloalkyl groups being bridged together. In some embodiments of the present invention, the bridged tricycloalkyl is adamantyl. In some embodiments of the present invention, the bridged tricycloalkyl is optionally substituted with a substituent.

As used herein, the term "heterocycloalkyl" refers to a ring system formed by at least one ring carbon atom in a saturated or partially unsaturated monocyclic or polycyclic cyclic hydrocarbonyl system being substituted with at least one heteroatom selected from N, O and or S(=O)m' (wherein m' is an integer from 0 to 2). For example, it includes monocyclic heterocycloalkyl, spiro heterocycloalkyl, fused heterocycloalkyl and bridged heterocycloalkyl. Preferably, the heterocycloalkyl is 3 to 20-membered heterocycloalkyl; more preferably, the heterocycloalkyl is 3 to 10-membered heterocycloalkyl; more preferably, the heterocycloalkyl is 3 to 6-membered monocyclic heterocycloalkyl. The term "oxygen heterocycloalkyl" refers to heterocycloalkyl in which the heteroatom is O. The term "nitrogen heterocycloalkyl" refers to heterocycloalkyl in which the heteroatom is N. In some embodiments of the present invention, the heterocycloalkyl is

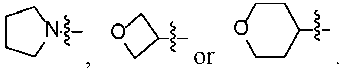

As used herein, the terms "aryl", "aryl ring" and "aromatic ring" are used interchangeably and refer to all-carbon monocyclic, all-carbon non-fused polycyclic (rings are connected by covalent bonds, not fused) or all-carbon fused polycyclic (i.e., rings that share adjacent carbon atom pairs) groups, at least one ring in the group is aromatic, i.e., has a conjugated π electron system. The term "C₆₋₁₄ aryl" refers to an aryl having 6 to 14 ring atoms, preferably C₆₋₁₀ aryl. In the present invention, C₆₋₁₄ aryl includes monocyclic aryl, non-fused polycyclic aryl and aromatic fused polycyclic, wherein examples of monocyclic aryl include phenyl, and examples of non-fused polycyclic aryl include biphenyl and the like.

As used herein, the term "heteroaryl" refers to an optionally substituted monocyclic or polycyclic group or ring system containing at least one aromatic ring, wherein the aromatic ring has one or more heteroatoms independently selected from O, S and N. In one embodiment, each ring of the heteroaryl group may have one or two O atoms, one or two S atoms and/or one to four N atoms, provided that the total number of heteroatoms in each ring is less than or equal to four and each ring has at least one carbon atom. In specific embodiments, the heteroaryl group has 5 to 20, 5 to 15 or 5 to 10 ring atoms. In specific embodiments, the heteroaryl group refers to a bicyclic, tricyclic or tetracyclic ring, wherein one of the rings is an aromatic ring having one or more heteroatoms independently selected from O, S and N, and the other rings may be saturated, partially unsaturated or aromatic rings, and may be a carbon ring or contain one or more heteroatoms independently selected from O, S and N. Examples of monocyclic heteroaryl groups include, but are not limited to, furanyl, imidazolyl, isothiazolyl, isoxazolyl, oxadiazolyl, oxazolyl, pyrazinyl, pyrazolyl, pyridazinyl, pyridinyl, pyrimidinyl, pyrrolyl, thiadiazolyl, thiazolyl, thienyl, tetrazolyl, triazinyl, and triazolyl. Examples of bicyclic heteroaryl groups include, but are not limited to, benzofuranyl, benzimidazolyl, benzisoxazolyl, benzopyranyl, benzothiadiazolyl, benzothiazolyl, benzothiophenyl, benzothiophenyl, benzothiophenyl, benzotriazolyl, benzoxazolyl, fluoropyridyl, imidazopyridyl, imidazothiazolyl, indolizinyl, indolyl, indazolyl, isobenzofuranyl, isobenzothiophenyl, isoindolyl, isoquinolyl, isothiazolyl, naphthyridinyl, oxazolopyridinyl, phthalazinyl, pteridinyl, purinyl, pyridopyridinyl, pyrrolopyridinyl, quinolyl, quinoxalinyl, quinazolinyl, thiadiazolopyrimidinyl, and thienopyridinyl. Examples of tricyclic heteroaryl groups include, but are not limited to, acridinyl, benzindolyl, carbazolyl, bibenzofuranyl, pyridinyl, phenanthrolinyl, phenanthridinyl, phenpyrazinyl, phenazinyl, phenothiazinyl, phenoxazinyl and xanthenyl. In a specific embodiment, the heteroaryl is optionally substituted with one or more substituents described elsewhere herein.

As used herein, the term "5- or 6-membered monocyclic heteroaryl" refers to a monocyclic heteroaryl having 5 or 6 ring atoms, wherein 1, 2, 3 or 4 ring atoms are heteroatoms selected from N, O or S(=O)m' (wherein m' is an integer of 0 to 2). Specific examples of monocyclic heteroaryl include, but are not limited to, thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine, etc. The term "5 or 6-membered nitrogen-containing monocyclic heteroaryl" refers to a 5 or 6-membered monocyclic heteroaryl in which at least one ring atom is N.

As used herein, the term "8 to10-membered bicyclic heteroaryl" refers to a fused bicyclic heteroaryl having 8 to 10 ring atoms, wherein 1, 2, 3, 4 or 5 ring atoms are heteroatoms selected from N, O or S(=O)m' (wherein m' is an integer of 0 to 2). The fused bicyclic heteroaryl can be a bicyclic group (preferably a 9- or 10-membered bicyclic heteroaryl ring) formed by the fusion of a monocyclic heteroaryl ring (such as phenyl) and a monocyclic heteroaryl ring (preferably a 5- or 6-membered monocyclic heteroaryl ring), or a bicyclic group formed by the fusion of a monocyclic heteroaryl ring (preferably a 5 or 6 membered monocyclic heteroaryl ring) and a monocyclic heteroaryl ring (preferably a 5 or 6 membered monocyclic heteroaryl ring). In some embodiments of the present invention, the 8 to 10-membered bicyclic heteroaryl is

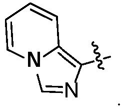

As used herein, the term "alkoxyl" refers to a stable straight or branched, or cyclic hydrocarbonyl group, or a combination thereof, which consists of the indicated number of carbon atoms and one or more (in one embodiment, one to three) O atoms. Examples of alkoxyl include, but are not limited to -O-CH3 (methoxyl), -O-CH₂-CH₃(ethoxyl), -O-CH₂-CH₂-CH₃ (n-propoxyl), -O-CH-(CH₃)₂ (isopropoxyl) and -O-CH₂-CH₂-O-CH₃. In one embodiment, the alkoxyl is an optionally substituted alkoxyl described elsewhere herein.

As used herein, the term "alkylthiol" refers to a stable straight or branched, or cyclic hydrocarbonyl group, or a combination thereof, consisting of the indicated number of carbon atoms and one or more (in one embodiment, 1 to 3) S atoms. Examples of alkylthiol include -S-CH₃ , -S-CH₂ -CH₃ , -S-CH₂ -CH₂ - CH₃ , -S-CH-(CH₃ )₂ , and -S-CH₂ -CH₂ -S-CH₃ .

As used herein, the term "bonded" refers to the chemically operative formation of a covalent bond between any two atoms in one or more groups. Chemically operative means that the covalent bond formed conforms to the classical valence bond theory. In one embodiment of the present invention, the β carbon atoms in -CH₂C_{(β)}H₃ and the nitrogen atom in 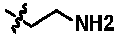 are bonded to each other to form 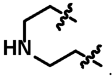 In another embodiment of the present invention, the β carbon atoms in 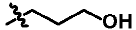 and the carbon atom of the methyl are bonded to each other to form

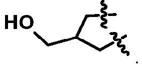

As used herein, the term "heteroaryl ketone group" refers to a group in which carbonyl is directly attached to a heteroaryl ring. In one embodiment of the present invention, the heteroaryl ketone group is a pyridone group, for example

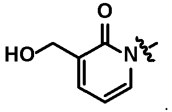

As used herein, the term "amino" refers to -NH₂.

As used herein, the term "hydroxyl" refers to -OH.

As used herein, "-SO₂ -" refers to

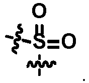

As used herein, the term "benzyl" refers to -CH₂- benzene.

As used herein, the term "halogen" refers to fluorine, chlorine, bromine or iodine.

As used herein, the term "substituted" refers to any one or more hydrogen atoms on a particular atom being replaced by a substituent, which may include variants of deuterium and hydrogen, as long as the valence of the particular atom is normal and the substituted compound is stable. When the substituent is an oxo group (i.e., =O), it means that two hydrogen atoms are replaced. Oxo substitution does not occur on aromatic groups. The term "optionally substituted" or "optionally substituted" means that it may be substituted or not substituted, and unless otherwise specified, the type and number of the substituent may be arbitrary on a chemically achievable basis.

As used herein, "at least one hydrogen is replaced by a substituent" means that one hydrogen is replaced by a substituent or multiple (eg, 2, 3, 4) hydrogens are replaced by the same or different substituents.

The above groups are connected to other parts of the molecule via the ring atoms marked with " 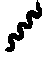 ".

### Pharmaceutical compositions

On the other hand, an embodiment of the present invention further provides a pharmaceutical composition containing the compounds of the present invention or pharmaceutically acceptable salts, stereoisomers, solvates or prodrugs thereof, and a pharmaceutically acceptable carrier.

As used herein, the term "stereoisomer" includes conformational isomers and configurational isomers, wherein configurational isomers mainly include cis-trans isomers and optical isomers. The compounds of the present invention may exist in the form of stereoisomers, and therefore cover all possible stereoisomer forms, including but not limited to cis-trans isomers, tautomers, enantiomers, diastereomers, atropisomers, etc. The compounds of the present invention may also exist in the form of any combination or any mixture of the aforementioned stereoisomers, such as equal mixtures of mesomer, racemate, atropisomers, etc, for example, a single enantiomer, a single diastereomer or a mixture thereof, or a single atropisomer or a mixture thereof. When the compounds of the present invention contain an olefin double bond, unless otherwise specified, it includes cis-isomers and trans-isomers, and any combination thereof. The atropisomers of the present invention are stereoisomers of axial or planar chirality generated based on restricted intramolecular rotation. And as a drug, stereoisomers with excellent activity are preferred. The compound of general formula (I) has optical isomers arising from asymmetric carbon etc., and if necessary, a single isomer can be separated and obtained by methods known in the art, such as crystallization or chiral chromatography.

As used herein, the term "pharmaceutically acceptable salts" refers to salts that are suitable for contact with tissues of humans and lower animals without abnormal toxicity, irritation, allergic reactions, etc., within the scope of reasonable medical judgment, and are commensurate with a reasonable benefit/risk ratio. Pharmaceutically acceptable salts are well known in the prior art. For example, SM Berge et al. describe pharmaceutically acceptable salts in detail in J. Pharmaceutical Sciences, 1977, 66, 1-19, which are incorporated herein by reference. Pharmaceutically acceptable salts of the compounds of the present invention include those derived from suitable inorganic acids and bases and suitable organic acids and bases. Examples of pharmaceutically acceptable non-toxic acid addition salts are salts formed of amino groups with inorganic acids such as hydrochloric acid, hydrobromic acid, phosphoric acid, sulfuric acid and perchloric acid, or with organic acids such as acetic acid, oxalic acid, maleic acid, tartaric acid, citric acid, succinic acid or malonic acid, or salts formed by using other methods used in the art such as ion exchange. Other pharmaceutically acceptable salts include adipate, alginate, ascorbate, aspartate, benzenesulfonate, benzoate, bisulfate, borate, butyrate, camphorate, camphorate sulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy - ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectate, persulfate, 3-phenylpropionate, phosphate, pivalate, propionate, stearate, succinate, sulfate, tartrate, thiocyanate, p-toluenesulfonate, undecanoate, valerate and the like. Salts derived from appropriate bases include alkali metal salts, alkaline earth metal salts, ammonium salts and N ⁺ (C ₁₋₄ alkyl)₄ salts. Representative alkali metal salts or alkaline earth metal salts include sodium salts, lithium salts, potassium salts, calcium salts, magnesium salts, etc. Other pharmaceutically acceptable salts (when applicable) include non-toxic ammonium salts, quaternary ammonium salts and amine cations formed by using counterions such as halides, hydroxides, carboxylates, sulfates, phosphates, nitrates, lower alkyl sulfonates and aryl sulfonates.

As used herein, the terms "solvent compounds" and "solvates" refer to a substance formed by the combination of a compound of the present invention and a pharmaceutically acceptable solvent. Pharmaceutically acceptable solvents include acetic acid and the like. Solvates include stoichiometric solvents and non-stoichiometric solvents. Certain compounds of the present invention may exist in a non-solvated form or a solvated form. Generally speaking, the solvated form is comparable to the non-solvated form and is included within the scope of the present invention.

### Uses

On the other hand, an embodiment of the present invention further provides uses of the compounds of the present invention or pharmaceutically acceptable salt, stereoisomer, solvate or prodrug thereof, or the pharmaceutical compositions of the present invention for:
(i) binding to TEAD, preferably to the palmitoyl pocket of TEAD;
(ii) inhibiting TEAD transcription levels;
(iii) inhibiting/blocking YAP-TEAD binding;
(iv) inhibiting/blocking YAP-TEAD transcriptional function;
(v) regulating the Hippo signaling pathway;
(vi) preventing and/or treating diseases associated with increased TEAD transcription levels and/or YAP phosphorylation disorders and/or Hippo signaling pathway disorders; and/or
(vii) preparing drugs for preventing and/or treating diseases associated with increased TEAD transcription levels and/or YAP phosphorylation disorders and/or Hippo signaling pathway disorders.

### Treatment method

On the other hand, embodiments of the present invention further provide a method for treating diseases associated with increased TEAD transcription levels and/or YAP phosphorylation disorders and/or Hippo signaling pathway disorders, comprising the steps of:
administering a therapeutically effective amount of the compounds of the present invention or pharmaceutically acceptable salts, stereoisomers, solvates or prodrugs thereof to a subject;
or, administering a therapeutically effective amount of the pharmaceutical compositions of the present invention to a subject.

As used herein, the term "preventing" refers to preventing the onset, recurrence or spread of a disease or condition, or one or more symptoms associated with the disease or condition. In one embodiment, such symptoms are known to those skilled in the art to be associated with the disease or condition to be prevented. In a specific embodiment, the term refers to the administration of the compounds provided herein in conjunction with or without other additional active agents before the onset of symptoms to patients with a risk of suffering from a disease or disorder described herein. The term includes the suppression and reduction of symptoms of a particular disease. In a specific embodiment, patients with a family history of a disease are specifically selected as candidates. In addition, patients with a history of recurrent symptoms are also potential candidates for prevention.

As used herein, the term "treating" refers to eradicating or ameliorating a disease or condition, or one or more symptoms associated with the disease or condition. In one embodiment, such symptoms are known to those skilled in the art to be associated with the disease or condition to be treated. In specific embodiments, the term refers to minimizing the spread or worsening of a disease or condition by administering one or more preventive or therapeutic agents to a subject suffering from the disease or condition. In some embodiments, the term refers to administering a compound of the invention after the onset of symptoms of a particular disease, in conjunction with or without other additional active agents.

As used herein, the term "subject" is defined herein to include animals, such as mammals, including, but not limited to, primates (eg, humans), cows, sheep, goats, horses, dogs, cats, rabbits, rats, mice, and the like.

The compounds of the present invention are included in a pharmaceutically acceptable vector or diluent in an amount sufficient to deliver to a patient a therapeutically effective amount for the desired indication without causing serious toxic effects in the treated patient.

As used herein, the term "therapeutically effective amount" refers to an amount of a compound sufficient to provide a therapeutic effect in the treatment or management of a disease or disorder, or sufficient to delay or minimize one or more symptoms associated with the disease or disorder. A therapeutically effective amount of a compound refers to an amount of a therapeutic agent that, when used alone or in combination with other therapies, can provide a therapeutic effect in the treatment or management of a disease or disorder. The term "therapeutically effective amount" can include an amount that improves overall therapy, reduces or avoids symptoms or causes of a disease or disorder, or enhances the therapeutic efficacy of another therapeutic agent.

### Preparation of compounds

On the other hand, the present invention also provides general synthetic routes of the compounds, including general synthetic routes 1, 2 and 3.

### General synthetic route 1:

SM-1 reacts with SM-2 to undergo an aromatic nucleophilic substitution reaction under the condition of NaH or K₂ CO₃ as a base to obtain Int-1. The Br atom in Int-1 is converted into a borate ester using SM-3 by a Pd-catalyzed reaction to obtain Int-2. Int-2 then reacts with an aromatic chloride or bromide Int-3 by a Pd-catalyzed Suzuki reaction to obtain Int-4. When Z in Int-4 is NH, it must first be protected with Boc to obtain Int-5. The nitro group in Int-5 is then reduced to an amino group to obtain Int-6. The amino group in Int-6 is diazotized with isoamyl nitrite and iodinated with cuprous iodide to obtain Int-7. The iodide Int-7 undergoes carbon-phosphorus coupling with a dialkylphosphine oxide SM-4 under Pd catalysis to obtain Int-8. When Z of Int-8 is protected by the Boc, a step of TFA of the Boc deprotection is performed to obtain the final compound.

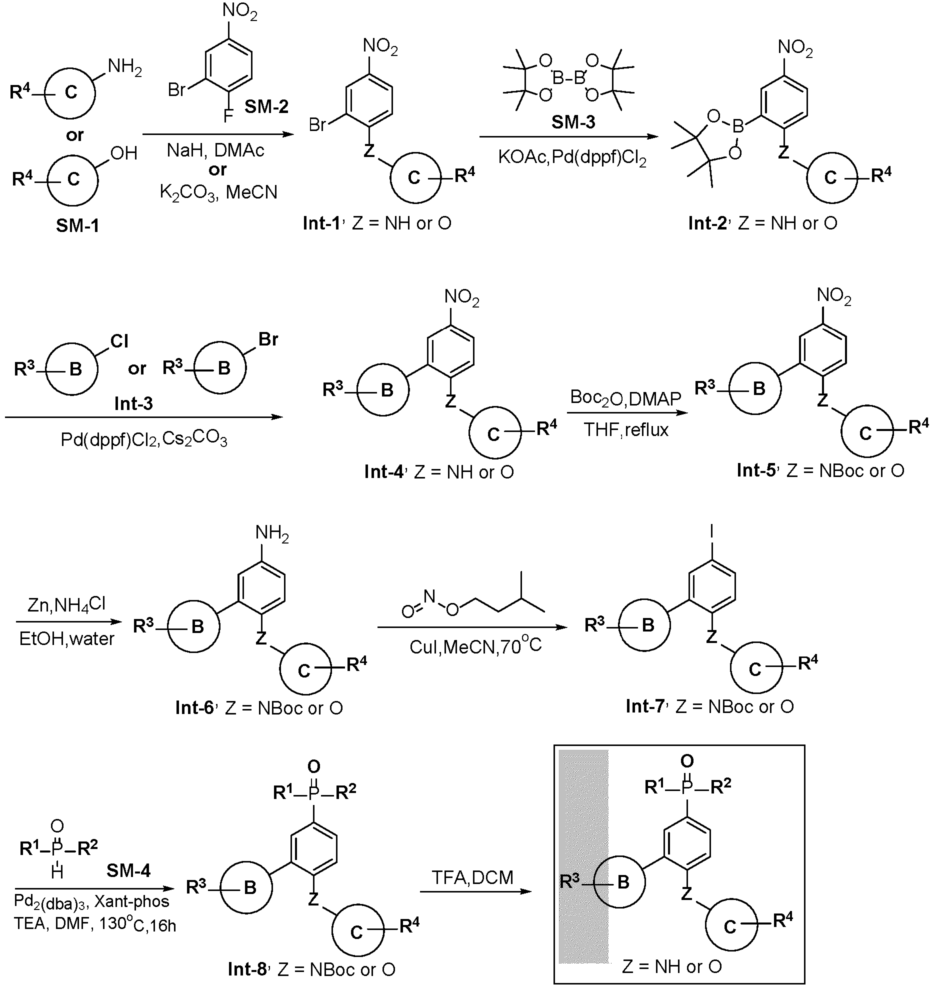

### General synthetic route 2:

According to the general synthetic route 1, Int-8-1 is synthesized from 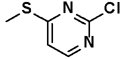 as Int-3. The methylthio group of Int-8-1 is oxidized to methyl sulfone using mCPBA to obtain Int-9. SM-5 reacts with Int-9 to undergo aromatic nucleophilic substitution reaction under the condition of NaH or K₂CO₃ as a base to obtain Int-10. When the Z of Int-10 is protected by the Boc, a further step of TFA of the Boc deprotection is performed to obtain the final compound.

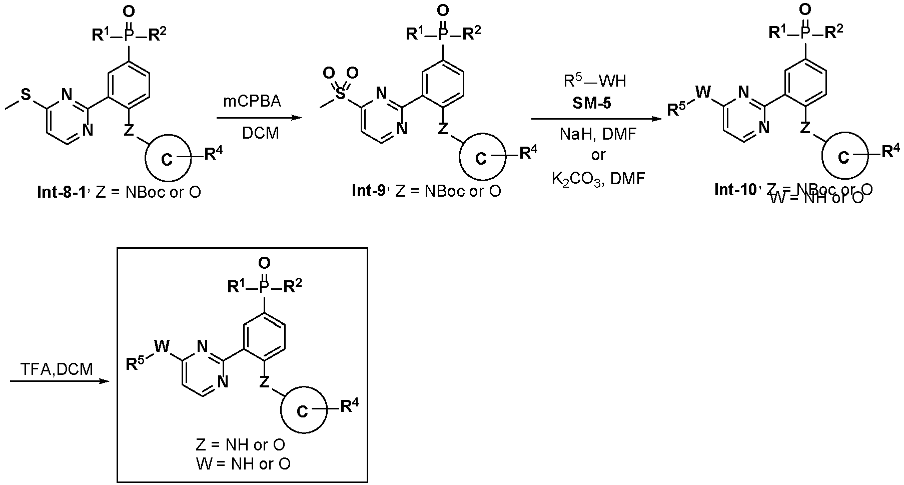

### General synthetic route three:

SM-6 undergoes carbon-phosphorus coupling with dimethylphosphine oxide under Pd catalysis, and then undergoes aromatic nucleophilic substitution reaction with SM-1 under the condition of NaH or K₂ CO₃ as base to obtain Int-11. The cyano group of Int-11 reacts with hydroxylamine, and the obtained condensation product is treated with acetyl chloride in pyridine to form the oxadiazole heterocycle to obtain the final compound.

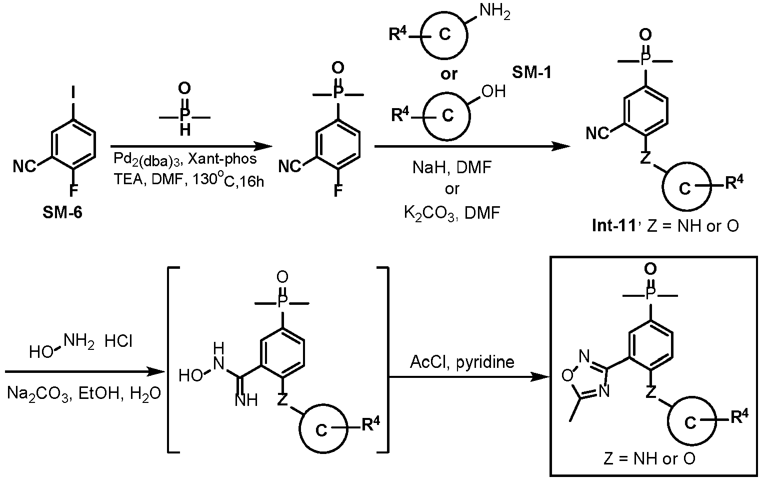

In a specific embodiment, the subject is a human. In order to make the purpose, technical scheme and advantages of the embodiments of the present invention clearer, the present invention is further described below in conjunction with specific examples. It should be understood that these examples are only used to illustrate the present invention and are not intended to limit the scope of the present invention. The experimental methods in the following examples that do not specify specific conditions are usually carried out under conventional conditions or under conditions recommended by the manufacturer. Unless otherwise specified, percentages and parts are weight percentages and weight parts. The experimental materials and reagents used in the following examples can be obtained from commercial channels unless otherwise specified.

Unless otherwise specified, the technical and scientific terms used herein have the same meaning as commonly understood by ordinary technicians in the technical field to which the application belongs. It should be noted that the terms used herein are only for describing specific embodiments and are not intended to limit the exemplary embodiments of the present application.

### Embodiment 1: Synthesis and characterization of compound 8

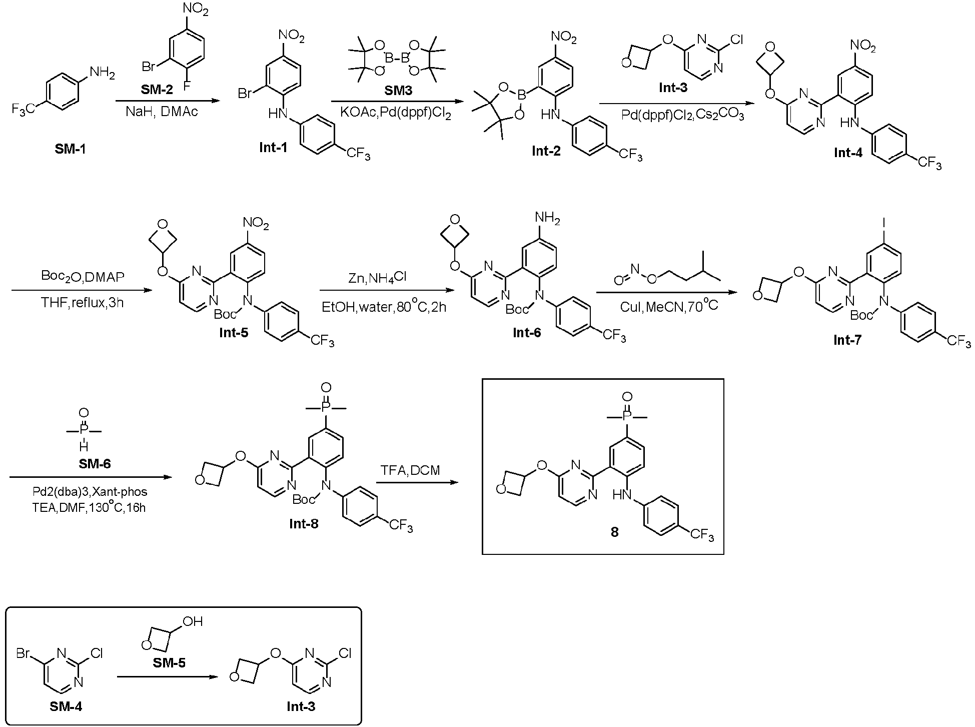

### Synthesis of intermediate Int-1

Dissolving Compound SM-1 (8.06 g, 50.00 mmol, 1 eq) in DMAc (100 mL), and adding NaH (4.0 g, 100.00 mmol, 2 eq) at 0 °C, after the addition, stirring the mixture for 0.5 h, dissolving Compound SM-2 (11.0 g, 50.00 mmol, 1 eq) in DMAc (15 mL) and added at 0 °C. After the dropwise addition is completed, heating to 130 °C and stirring overnight, after monitoring the completion of the reaction by LCMS, cooling to room temperature, adding 100 mL of saturated NH4Cl aqueous solution and 150 mL of EA, filtering, and separating the filtrate to obtain an organic phase, washing with brine, drying with anhydrous sodium sulfate, concentrating the organic phase, and purifying by silica gel column (PE: EA = 15:1) to obtain intermediate Int-1 (3.6 g, purity 89%, yield 17.74%) as a yellow solid. LCMS [M+H] ⁺ = 361/363.

### Synthesis of intermediate Int-2

adding Compound Int-1 (1 g, 2.77 mmol, 1 eq), compound SM-3 (0.844 g, 3.32 mmol, 1.2 eq), Pd(dppf)Cl ₂ (0.2 g, 0.277 mmol, 0.1 eq) and potassium acetate (0.54 g, 5.54 mmol, 2 eq) to 1,4-dioxane (10 mL) in sequence, replacing with N₂, heating to 100 °C and stirring overnight, after monitoring the completion of the reaction by LCMS, cooling to room temperature, adding 100 mL of water and 150 mL of EA, filtering, and separating the filtrate to obtain an organic phase, drying with anhydrous sodium sulfate, concentrating the organic phase to obtain intermediate **Int-2** (1.7 g, crude product) as a black solid. LCMS [M+H] ⁺ = 409.

### Synthesis of intermediate Int-3

Dissolving Compound SM-4 (2.2 g, 11.37 mmol, 1 eq) and compound SM-5 (0.84 g, 11.37 mmol, 1 eq) in THF (10 mL), and dissolving potassium tertbutoxide (1.4 g, 12.51 mmol, 1.1 eq) in THF (10 mL), and adding dropwise to the reaction system at -50 °C, after the dropwise addition is complete, stirring at -50 °C for 1 hour, after monitoring the completion of the reaction by LCMS, adding100 mL of saturated ammonium chloride aqueous solution, extracting with EA , drying with anhydrous sodium sulfate, concentrating the organic phase, and preparing the intermediate **Int-3** (0.56 g, purity 99%, yield 26.12%) by reverse phase as a white solid. LCMS [M+H] ⁺ = 187.

### Synthesis of intermediate Int-4

Adding Intermediate Int-2 (1.7 g, 2.77 mmol, crude product), intermediate Int-3 (0.517 g, 2.77 mmol, 1 eq), Pd(dppf)Cl ₂(0.2 g, 0.277 mmol, 0.1 eq) and cesium carbonate (1.8 g, 5.54 mmol, 2 eq) to a mixed solvent of DMF (15 mL) and water (3 mL) in sequence, and replacing with N₂, heating to 100 °C and stirring overnight, after monitoring the completion of the reaction by LCMS, cooling to room temperature, adding 100 mL of water and 150 mL of EA, filtering, separating the filtrate to obtain an organic phase, drying with anhydrous sodium sulfate, concentrating the organic phase, and purifying by silica gel column (DCM: MeOH = 10:1) to obtain intermediate Int-4 (0.5 g, purity 89%, yield 37.18%) as a light yellow solid. LCMS [M+H] ⁺ = 433.

### Synthesis of intermediate Int-5

Dissolving the intermediate Int-4 (500 mg, 1.16 mmol, 1 eq), Boc₂O (505 mg, 2.31 mmol, 2 eq) and DMAP (71 mg, 0.578 mmol, 0.5 eq) in THF (8 mL), heating to reflux and stirring for 3 hours, after monitoring the completion of the reaction by LCMS, adding 100 mL of aqueous solution, extracting with EA, drying with anhydrous sodium sulfate, concentrating the organic phas, and then purifying by silica gel column (DCM: MeOH = 12:1) to obtain intermediate Int-5 ( 540 mg, purity 89%, yield 78%) as a light yellow solid.

### Synthesis of intermediate Int-6

Dissolving the intermediate Int-5 (540 mg, 1.01 mmol, 1 eq), Zn powder (398 mg, 6.06 mmol, 6 eq) and ammonium chloride solid (542 mg, 10.14 mmol, 10 eq) in a mixed solvent of EtOH (10 mL) and water (2 mL), heating to reflux and stirring for 2 hours, after monitoring the completion of the reaction by LCMS, adding 50 mL of aqueous solution, extracting with EA, drying with anhydrous sodium sulfate, concentrating the organic phase, and then purifying by silica gel column (DCM: MeOH = 10:1) to obtain the intermediate Int-6 (400 mg, purity of 73.7%, yield of 57.85%) as a light yellow solid.

### Synthesis of intermediate Int-7

Dissolving the intermediate Int-6 (400 mg, 0.796 mmol, 1 eq), isoamyl nitrite (112 mg, 0.955 mmol, 1.2 eq) and cuprous iodide (227 mg, 1.19 mmol, 1.5 eq) in acetonitrile (8 mL), heating to reflux and stirring for 2 hours, after monitoring the completion of the reaction by LCMS, adding 50 mL of aqueous solution, extracting with EA, drying with anhydrous sodium sulfate, concentrating the organic phase, and then purifying by silica gel column (DCM: MeOH = 9:1) to obtain the intermediate Int-7 (120 mg, purity of 84.5%, yield of 20.77%) as a light yellow solid.

### Synthesis of intermediate Int-8:

Adding the intermediate Int-7 (120 mg, 0.196 mmol, 1 eq), compound SM-6 (23 mg, 0.293 mmol, 1.5 eq), **Pd ₂ (dba) ₃** (36 mg, 0.039 mmol, 0.2 eq), **XantPhos** (23 mg, 0.039 mmol, 0.2 eq) and **TEA** (39 mg, 0.391 mmol, 2 eq) to DMF (5 mL) in sequence, replacing with N₂, heating to 100 °C and stirring overnight, after monitoring the completion of the reaction by LCMS, adding 30 mL of water, extracting with EA, drying with anhydrous sodium sulfate, concentrating the organic phase, and then purifying by TLC (DCM: MeOH = 10:1) to obtain intermediate Int-8 (70 mg, purity 91%, yield 28.89%) as a light yellow solid.

### Synthesis of Compound 8

Dissolving the intermediate Int-8 (70 mg, 0.12 mmol, 1 eq) in DCM (4 ml), adding TFA (1 ml) at 0°C, and the mixture is reacted at 25°C for 1 hour. After the reaction is completed, the mixture is cooled to 0°C and diluted with water (30 ml), and a saturated sodium carbonate aqueous solution is added dropwise to adjust the mixture to alkalinity, the aqueous layer is extracted with ethyl acetate (15 mL×3 times), and the organic layers are combined and washed with saturated brine (25 mL×1 time), dried over anhydrous sodium sulfate, and concentrated under reduced pressure, the residue is purified by prep-HPLC (ammonia/acetonitrile system) and lyophilized to obtain compound 8 (25.0 mg, 0.054 mmol, purity of 99.0%, yield of 45%) as a yellow solid. LCMS[M+1] ⁺ = 463.8. ¹ H-NMR (400 MHz, DMSO-d6) δ 11.35 (s, 1 H), 8.73 (d, *J =* 6.0 Hz, 1 H), 8.68 (dd, *J =* 12.4, 1.6 Hz, 1 H), 7.76-7.70 (m, 1 H), 7.67 (d, *J =* 8.4 Hz, 2 H), 7.54 (dd, *J =* 8.4, 2.4 Hz, 1 H), 7.45 (d, *J =* 8.8 Hz, 2 H), 7.02 (d, 5.6 Hz, 1H), 5.79-5.73 (m, 1 H), 4.95 (t, *J =* 7.2 Hz, 2 H), 4.69-4.66 (m, 2 H), 1.67 (d, *J* = 13.2 Hz, 6 H).

### Embodiment 2: Synthesis and characterization of compound 1

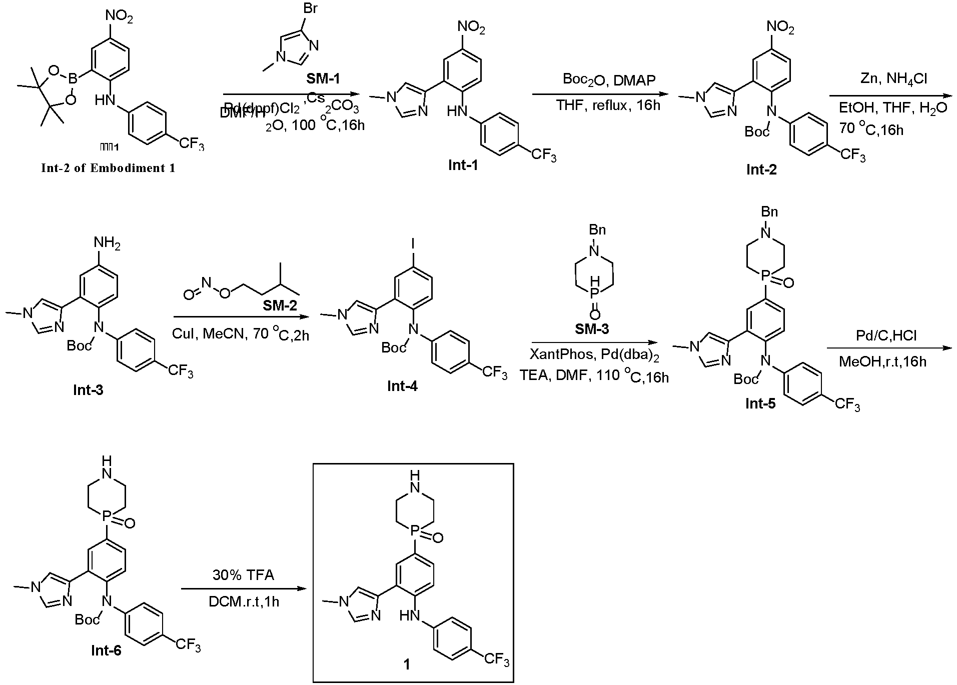

### Synthesis of Intermediate Int-1

Adding the intermediate Int-2 (2 g, 4.9 mmol, 1 eq) of Embodiment 1, compound SM-1 (1.5 g, 9.8 mmol, 2 eq), Pd(dppf)Cl₂ (118 mg, 0.24 mmol, 0.05 eq), and Cs₂CO₃ (3.2 g, 9.8 mmol, 2 eq) to a three-necked flask. Replacing with N₂ , and then adding DMF (20 ml) and H₂O (4 ml). The mixture is reacted at 100 °C for 16 hours. After the reaction is completed, cooling to room temperature and filtering, the obtained filtrate is diluted with ethyl acetate (150 mL) and washed with water (50 mL × 3 times), washed with saturated brine (50 mL × 1 time), then dried with anhydrous sodium sulfate, and finally concentrated under reduced pressure. The obtained residue is purified by silica gel column chromatography (petroleum ether:ethyl acetate = 5:1) to obtain intermediate **Int-1** (830 mg, 2.3 m mol, purity 92%, yield 46.89%) as a yellow solid. LCMS [M+H] ⁺ =363.

### Synthesis of Intermediate Int-2

Dissolving the intermediate **Int-1** (830 mg, 2.3 mmol, 1 eq), Boc₂O (1 g, 4.6 mmol, 2 eq), DMAP (140 mg, 1.1 mmol, 0.5 eq) in THF (20 ml), refluxing and reacting at 70 °C for 16 hours. After the reaction, adding ethyl acetate (100 mL) to dilute, and washed with 1 mol/L hydrochloric acid aqueous solution (30 mL), washed with saturated brine (30 mL), and then dried with anhydrous sodium sulfate, and finally concentrated under reduced pressure. The residue is purified by silica gel column chromatography (petroleum ether: ethyl acetate = 5:1) to obtain intermediate **Int-2** (550 mg, 1.2 mmol, purity of 91%, yield of 51.54%) as a yellow oil. LCMS [M+H] ⁺ = 462.9.

### Synthesis of intermediate Int-3

Dissolving the intermediate **Int-2** (550 mg, 1.2 mmol, 1 eq) in EtOH (10 mL), THF (2 mL) and H ₂ O (2 ml), then adding Zn powder (630 mg, 9.6 mmol, 8 eq) and NH ₄ Cl (650 mg, 12 mmol, 10 eq), replacing with N₂, refluxing and reacting at 70 °C for 16 hours (Zn powder is added every three hours, twice in total, 630 mg×2). After the reaction, filtering the reaction solution through diatomaceous earth, and diluting the filtrate with water (50 mL), extracting the aqueous layer with ethyl acetate (30 mL×3 times), and washing with saturated brine (50 mL×1 time), then drying with anhydrous sodium sulfate, and finally concentrating under reduced pressure. The obtained residue is purified by silica gel column chromatography (eluent: petroleum ether/ethyl acetate = 2/1) to obtain intermediate **Int-3** (300 mg, purity 90%, 0.7 mmol, yield 57.47%) as a yellow oil. LCMS [M-Boc] ⁺ = 333.2.

### Synthesis of intermediate Int-4

Dissolving the intermediate **Int-3** (300 mg, 0.7 mmol, 1 eq) and **SM-2** (90 mg, 0.8 mmol, 1.1 eq) in MeCN (15 ml), and adding CuI (200 mg, 1.05 mmol, 1.5 eq) under N₂ protection, and reacting the mixture at 70°C for 2 hours. After the reaction , cooling to room temperature, and adding water (50 mL) to dilute, extracting the aqueous layer with ethyl acetate (30 mL×3 times), and washing with saturated brine (50 mL×1 time), then drying with anhydrous sodium sulfate, and finally concentrating under reduced pressure. The obtained residue is purified by silica gel column chromatography (eluent: petroleum ether/ethyl acetate = 8/1) to obtain intermediate **Int-4** (180 mg, 0.33 mmol, yield 47.74%) as a yellow oil. LCMS[M+H] ⁺ = 543.7.

### Synthesis of intermediate Int-5 :

Dissolving XantPhos (12 mg, 0.02 mmol, 0.05 eq), Pd(dba) ₂ (12 mg, 0.02 mmol, 0.05 eq), TEA (100 mg, 1 mmol, 3 eq) in anhydrous DMF (5 ml) under N₂ protection, and reacting at room temperature for 20 minutes. Then, adding the intermediate **Int-4** (180 mg, 0.33 mmol, 1 eq) and **SM-3** (105 mg, 0.5 mmol, 1.5 eq), and continuing to react at 110°C for 16 hours. After the reaction, cooling to room temperature. Adding water (50 mL) to dilute, and extracting the aqueous layer with ethyl acetate (30 mL × 3 times), and washing with saturated brine (50 mL × 1 time), then drying with anhydrous sodium sulfate, and finally concentrating under reduced pressure. The obtained residue is purified by silica gel column chromatography (eluent: dichloromethane /methanol = 20/1) to obtain intermediate **Int-5** (80 mg, 0.13 mol, purity 90%, yield 38.8%) as a yellow solid. LCMS [M+H] ⁺ = 625.5.

### Synthesis of intermediate Int-6:

Dissolving the intermediate **Int-5** (80 mg, 0.13 mmol, 1 eq) in MeOH (5 ml) at room temperature, adding Pd/C (10 mg), conc.HCl (0.2 ml), and reacting under a hydrogen atmosphere for 16 hours. After the reaction, filtering and spin-drying to obtain intermediate **Int-6** (45 mg, 0.08 mol, purity 90%, yield 65.71%) as a yellow solid, which is used directly in the next reaction without further purification. LCMS [M+H] ⁺ = 534.9.

### Synthesis of Compound 1

Dissolving the intermediate **Int-6** (40 mg, 0.08 mmol, 1 eq) in DCM (3.5 ml), adding TFA (1.5 ml), and reacting the mixture at 25°C for 1 hour. After the reaction, cooling the mixture to 0°C, and adding water (30 ml) to dilute, and adding a saturated sodium carbonate aqueous solution dropwise to adjust the mixture to alkalinity, extracting the aqueous layer with ethyl acetate (15 mL×3 times), and combining the organic layers, and washing with saturated brine (25 mL×1 time), drying with anhydrous sodium sulfate, and concentrating under reduced pressure. The residue is purified by prep-HPLC (ammonia/acetonitrile system) and lyophilized to obtain compound 1 (16.2 mg, 0.037 mmol, purity of 96.45%, yield of 46.25%) as a yellow solid. LCMS[M+H] ⁺ = 434.9. ¹ H-NMR (400 MHz, DMSO-d6) δ 10.78 (s, 1 H), 7.99 (d, *J =* 11.2 Hz, 1 H), 7.83 (d, *J* =10.0 Hz, 2 H), 7.59 (d, *J =* 8.8 Hz, 2 H), 7.53-7.50 (m, 2 H), 7.30 (d, *J =* 8.8 Hz, 2 H), 3.73 (s, 3 H), 3.32-3.02 (m, 2 H), 2.96-2.87 (m, 2 H), 2.17-2.13 (m, 2 H), 1.80-1.76 (m 2 H).

### Embodiment 3: Synthesis and characterization of compound 9

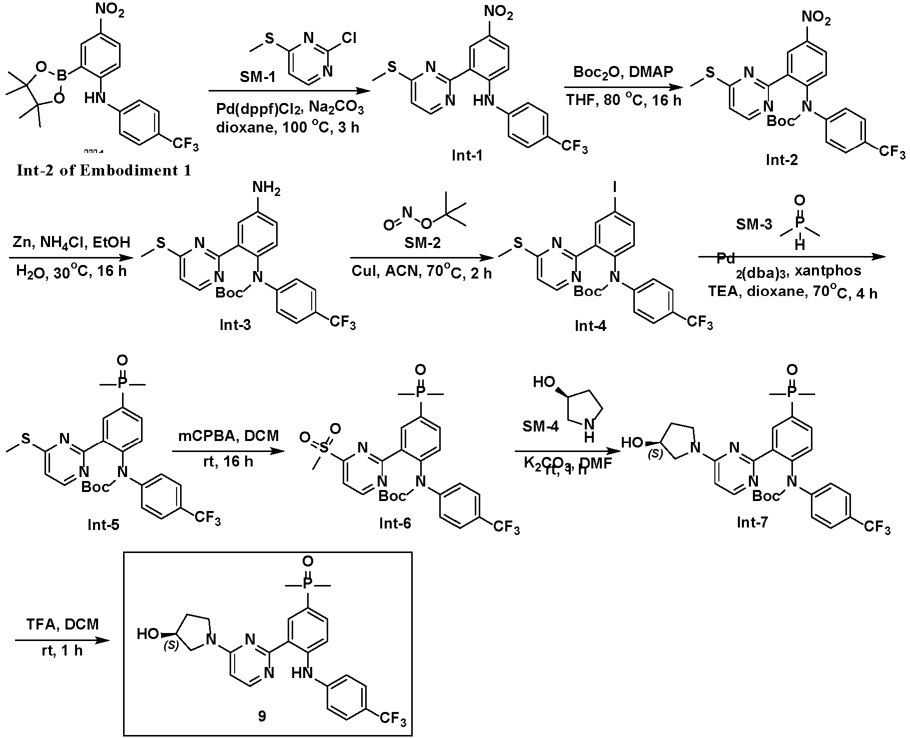

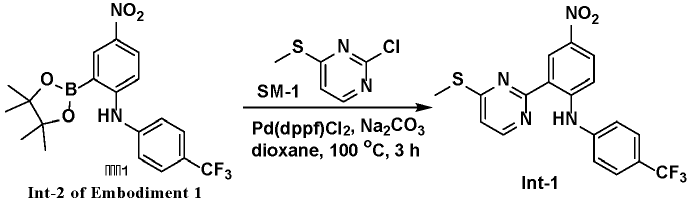

| **Starting Materials** | **Molecular weight** | **quantity** | **mole** | **equivalent** |
|---|---|---|---|---|
| **Int-2 in Embodiment 1** | **408** | **6.0 g** | **14.7 mmol** | **1.0** |
| **SM-1** | **160.5** | **2.4 g** | **14.7 mmol** | **1.0** |
| **Pd(dppf)Cl₂** | **731** | **1.1 g** | **1.5 mmol** | **0.1** |
| **Na₂CO₃** | **106** | **3.1 g** | **29.4 mmol** | **2** |
| **dioxane** | / | **100 mL** | / | / |
| **H₂O** | / | **10 mL** | / | / |

1) Under N₂ protection, adding Int-2, SM-1, Pd(dppf)Cl 2, and Na ₂ CO ₃ from Embodiment 1 to dioxane and H₂O.
2) Reacting at 100 ^{°} C for 3 h. Monitoring by TLC.
3) Cooling to room temperature.
4) Spin-drying the reaction solution, and passing through a column (PE:EA=3:1) and spin-drying to obtain 5.5 g (92.1%) of yellow solid intermediate Int-1.

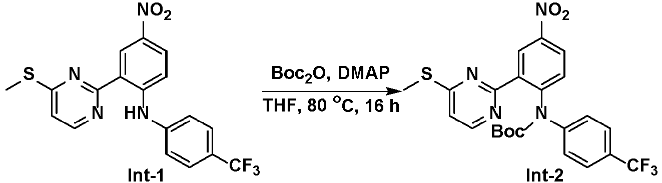

| **Starting Materials** | **Molecular weight** | **quantity** | **mole** | **equivalent** |
|---|---|---|---|---|
| **Int-1** | **406** | **5.5 g** | **13.5 mmol** | **1.0** |
| **Boc2O** | **218** | **3.54 g** | **16.2 mmol** | **1.2** |
| **DMAP** | **122** | **3.31 g** | **27.1 mmol** | **2.0** |
| **THF** | / | **100 mL** | / | / |

1) Adding Int-1, Boc₂O, and DMAP to THF.
2) Reacting at 80 ^{°} C for 16 h. Monitoring by TLC.
3) Spin-drying the reaction solution, and passing through a column (PE:EA=3:1) and spin-drying to obtain 4.2 g (61.3%) of white solid intermediate Int-2.

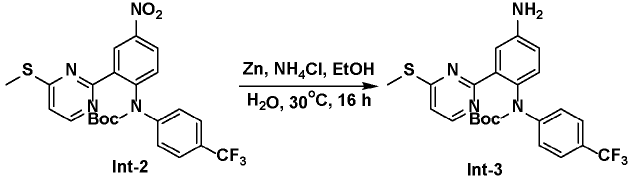

| **Starting Materials** | **Molecular weight** | **quantity** | **mole** | **equivale nt** |
|---|---|---|---|---|
| **Int-2** | **506.5** | **4.2 g** | **8.29 mmol** | **1.0** |
| **Zn** | **65** | **2.16 g** | **33.2 mmol** | **4.0** |
| **NH4Cl** | **58.5** | **1.94 g** | **33.2 mmol** | **4.0** |
| **EtOH** | / | **40 mL** | / | / |
| **H2O** | / | **40 mL** | / | / |

1) Adding Int-2, Zn, and NH ₄ Cl to EtOH and H₂O.
2) Reacting at 30 ^{°} C for 16 h, monitoring by TLC.
3) Spin-drying the reaction solution, and passing through a column (PE:EA=1:1) and spin-drying to obtain 2.7 g (68.3%) of a yellow compound intermediate Int-3.

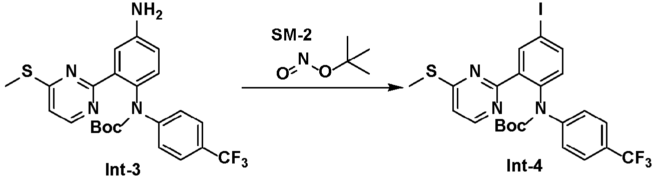

| **Starting Materials** | **Molecular weight** | **quantity** | **mole** | **equivalent** |
|---|---|---|---|---|
| **Int-3** | **476.5** | **3.6 g** | **5.67 mmol** | **1.0** |
| **SM-2** | **103** | | **6.80 mmol** | **1.2** |
| **CuI** | **190.5** | | **17.0 mmol** | **3.0** |
| **ACN** | / | **20 mL** | / | / |

1) Adding Int-3 to acetonitrile and cooling to 0 ^{°} C.
2 ) Adding SM-2.
3) Reacting at 25 ° C for 2 h.
4) Adding CuI.
5) Reacting at 70 ° C for 2 h. Monitoring by TLC.
6) Spin-drying the reaction solution, and passing through a column (PE:EA=3:1) and spin-drying to obtain 1.2 g (36.1%) of a yellow compound intermediate Int-4.

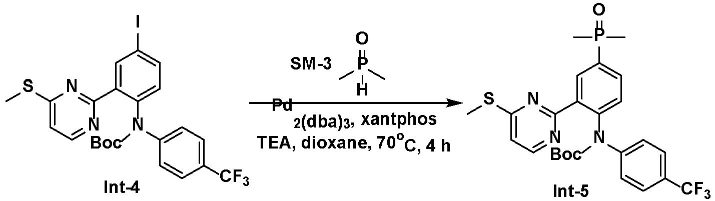

| **Starting Materials** | **Molecular weight** | **quantity** | **mole** | **equivalent** |
|---|---|---|---|---|
| **Int-4** | **587** | **1.2 g** | **2.0 mmol** | **1.0** |

| | | | | |
|---|---|---|---|---|
| **SM-3** | **78** | **0.48 g** | **6.1 mmol** | **3.0** |
| **Pd ₂ (dba) ₃** | **1035** | **0.21 g** | **0.20 mmol** | **0.1** |
| **Xantphos** | **578.5** | **0.24 g** | **0.41 mmol** | **0.2** |
| **TEA** | **101** | **0.62 g** | **6.1 mmol** | **3.0** |
| **dioxane** | / | **24 mL** | / | / |

1) Adding Int-4, SM-3, Pd₂(dba)₃ , Xantphos, and TEA to dioxane.
2) Reacting at 70 ^{°} C for 4 hours and monitoring by TLC.
3) Spin-drying the reaction solution, and passing through a column (DCM:MeOH=10:1) and spin-drying to obtain 0.95 g (86.5%) of a yellow compound intermediate Int-5.

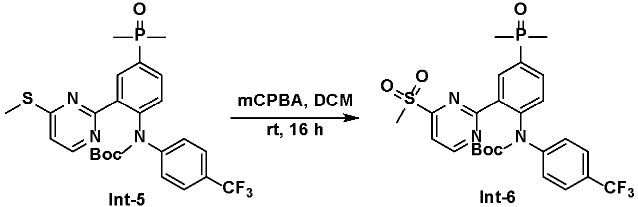

| **Starting Materials** | **Molecular weight** | **quantity** | **mole** | **equivalent** |
|---|---|---|---|---|
| **Int-5** | **537.5** | **950 mg** | **1.77 mmol** | **1.0** |
| **mCPBA** | **172.5** | **915 mg** | **5.30 mmol** | **3.0** |
| **DCM** | / | **20 mL** | / | / |

1) Adding Int-5 and mCPBA to DCM.
2) Reacting at 25 ^{°} C for 16 h and monitoring by TLC.
3) Spin-drying the reaction solution, and passing through a column (DCM:MeOH=10:1) and spin-drying to obtain 480 mg (47.7%) of a light yellow solid intermediate Int-6.

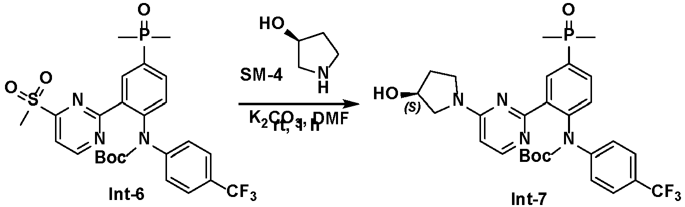

| **Starting Materials** | **Molecular weight** | **quantity** | **mole** | **equivalent** |
|---|---|---|---|---|
| **Int-6** | **569.5** | **150 mg** | **0.26 mmol** | **1.0** |
| **SM-4** | **87** | **46 mg** | **0.53 mmol** | **2.0** |
| **K ₂ CO ₃** | **138** | **109 mg** | **0.79 mmol** | **3.0** |
| **DMF** | / | **2.0 mL** | / | / |

1) Adding Int-6, SM-4, K₂ CO₃ into DMF.
2) Reacting at 25 ^{°} C for 1 hour and monitoring by TLC.
3) Spin-drying the reaction solution, and passing through a column (DCM:MeOH=10:1) and spin-drying to obtain 125 mg (82.3%) of a light yellow compound intermediate Int-7.

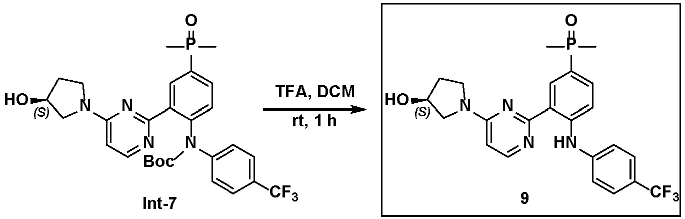

| **Starting Materials** | **Molecular weight** | **quantity** | **mole** | **equivalent** |
|---|---|---|---|---|
| **Int-7** | **576.5** | **120 mg** | **0.21 mmol** | **1.0** |
| **TFA** | **114** | **71.2 mg** | **0.62 mmol** | **3.0** |
| **DCM** | / | **5.0 mL** | / | / |

1) Adding Int-7 and TFA to DCM.
2) Reacting at 25 ^{°} C for 1 h. Monitoring by TLC.
3) Spin-drying the reaction solution, and passing through a column (DCM:MeOH=10:1) and spin-drying to obtain 34.0 mg (34.3%) of a light yellow solid compound 9 . ¹ H NMR (400 MHz, DMSO-d6) δ 11.91 (s, 1H), 8.86 (dd, J = 12.3, 2.0 Hz, 1H), 8.34 (d, J = 6.1 Hz, 1H), 7.72 - 7.63 (m, 3H), 7.54 (dd, J = 8.5, 2.5 Hz, 1H), 7.43 (d, J = 8.4 Hz, 2H), 6.52 (br s, 1H), 5.10 (d, J = 13.6 Hz, 1H), 4.45 (br s, 1H), 3.84 - 3.45 (m, 3H), 2.58 - 2.53 (m, 1H), 2.15 - 2.02 (m, 1H), 2.01 - 1.90 (m, 1H), 1.66 (s, 3H), 1.63 (s, 3H).

### Embodiment 4: Synthesis and characterization of compound 10

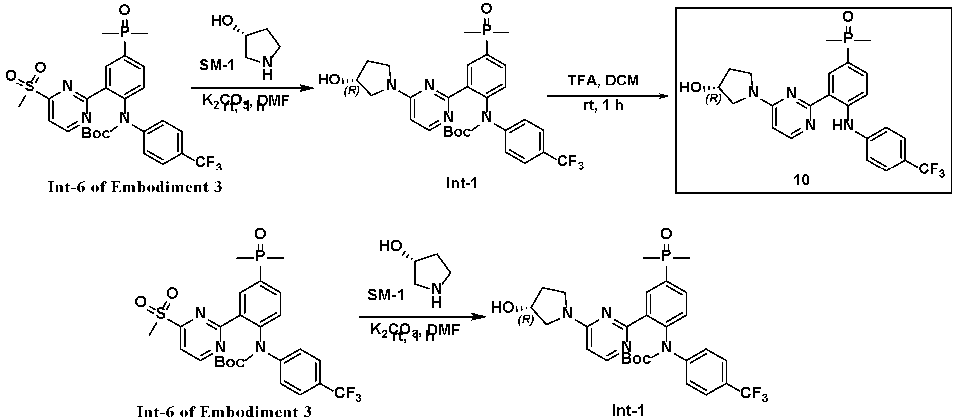

| **Starting Materials** | **Molecular weight** | **quantity** | **mole** | **equivalent** |
|---|---|---|---|---|
| **Int-6 in Embodiment 3** | **569.5** | **150 mg** | **0.26 mmol** | **1.0** |
| **SM-1** | **87** | **46 mg** | **0.53 mmol** | **2.0** |
| **K ₂ CO ₃** | **138** | **109 mg** | **0.79 mmol** | **3.0** |

| | | | | |
|---|---|---|---|---|
| **DMF** | / | **5.0 mL** | / | / |

1) Adding Int-6, SM-1, K₂CO₃ from Embodiment 3 to DMF.
2) Reacting at 25 ^{°} C for 1 hour and monitoring by TLC.
3) Spin-drying the reaction solution, and passing through a column (DCM:MeOH=10:1) and spin-drying to obtain 130 mg (85.6%) of a light yellow compound intermediate Int-1.

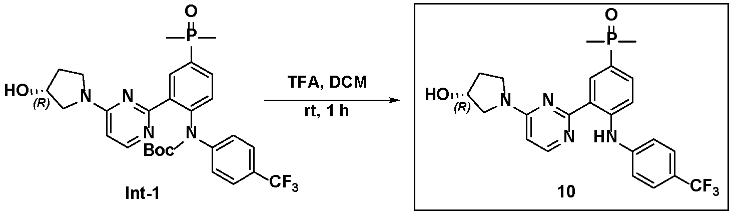

| **Starting Materials** | **Molecular weight** | **quantity** | **mole** | **equivalent** |
|---|---|---|---|---|
| **Int-1** | **576.5** | **120 mg** | **0.21 mmol** | **1.0** |
| **TFA** | **114** | **71.2 mg** | **0.62 mmol** | **3.0** |
| **DCM** | / | **5.0 mL** | / | / |

1) Adding Int-1 and TFA to DCM.
2 ) Reacting at 25 ^{°} C for 1 h. Monitoring by TLC.
3) Spin-drying the reaction solution, and passing through a column (DCM:MeOH=10:1) and spin-drying to obtain 62.3 mg (62.8%) of a light yellow solid compound **10**. ¹ H NMR (400 MHz, DMSO-d6) δ 11.91 (s, 1H), 8.86 (dd, J = 12.3, 2.0 Hz, 1H), 8.34 (d, J = 6.1 Hz, 1H), 7.72 - 7.63 (m, 3H), 7.54 (dd, J = 8.5, 2.5 Hz, 1H), 7.43 (d, J = 8.4 Hz, 2H), 6.52 (br s, 1H), 5.10 (d, J = 13.6 Hz, 1H), 4.45 (br s, 1H), 3.84 - 3.45 (m, 3H), 2.58 - 2.53 (m, 1H), 2.15 - 2.02 (m, 1H), 2.01 - 1.90 (m, 1H), 1.66 (s, 3H), 1.63 (s, 3H).

### Embodiment 5: Synthesis and characterization of compound 2

According to the general synthetic route 1 , compound 2 is synthesized by using 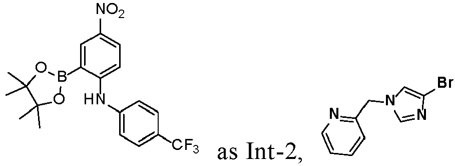 as Int-3 and dimethylphosphine oxide as SM-4. LCMS [M+H] ⁺ =471.

### Embodiment 6: Synthesis and characterization of compound 3

According to the general synthetic route 1 , compound **3** is synthesized by using 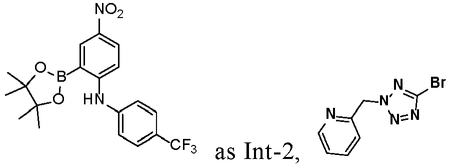 as Int-3 and dimethylphosphine oxide as SM-4 . LCMS [M+H] ⁺ = 473.

### Embodiment 7: Synthesis and characterization of compound 4

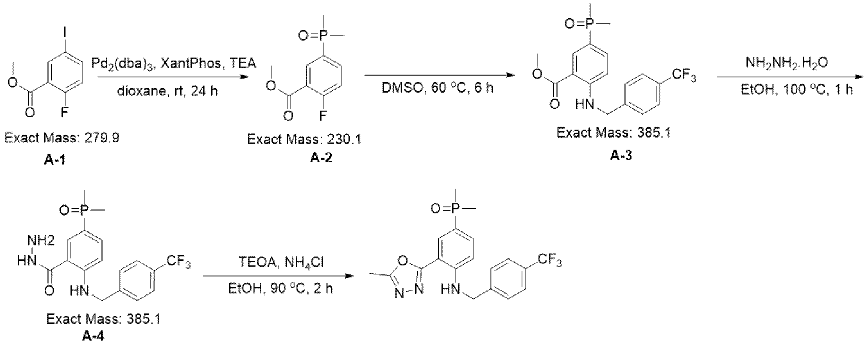

### Synthesis of intermediate A-2:

Adding raw material **A-1** (500 mg, 1.79 mmol), **Pd ₂ (dba) ₃** (164 mg, 0.18 mmol), **XantPhos** (207 mg, 0.36 mmol), dimethylphosphine oxide (167 mg, 2.14 mmol) and **TEA** (271 mg, 2.68 mmol) to dioxane (8 mL) in sequence, replacing with N₂, and stirring at room temperature overnight. Adding 50 mL of water, extracting with EA, drying with anhydrous sodium sulfate, concentrating the organic phase, and purifying with flash column chromatography DCM-MeOH (0 ~ 10 %) to obtain intermediate **A-2** (300 mg, yield 73.00 %) as a red solid. LCMS: m/z = 231.1 (M+H ⁺ , ESI)

### Synthesis of intermediate A-3:

Dissolving Compound **A-2** (300 mg, 1.30 mmol) in DMSO (5 mL), and adding p-trifluoromethylbenzylamine (457 mg, 2.61 mmol), after the adding, heating to 60°C and reacting for 6 hours. After the reaction is completed, cooling to room temperature, adding 50 mL of water , extracting with EA, combining the organic phases and washing with brine, drying with anhydrous sodium sulfate, concentrating the organic phase and then purifying by silica gel column DCM: MeOH (0 ~ 10 %) (1 % TEA) to obtain intermediate **A-3** (340 mg, yield 67.70 %) as a yellow solid. LCMS: m/z = 386.1 (M+H ⁺ , ESI)

### Synthesis of intermediate A-4:

Adding hydrazine hydrate (1.01 g, 20.24 mmol) to the ethanol (5 mL) solution of compound **A-3** (300 mg, 0.78 mmol), after the adding, heating to 100 °C and reacting for 4 hours, after the reaction, cooling to room temperature and concentrating to obtain intermediate **A-4** (200 mg, yield 66.67%) as a white solid. The product is directly used for the next step without further purification. LCMS: m/z = 386.1 (M+H ⁺ , ESI)

### Synthesis of compound 4:

Adding triethyl orthoacetat (38 mg, 0.23 mmol) to the ethanol (1 mL) suspension of compounds **A-4** (30 mg, 0.08 mmol) and ammonium chloride (1 mg, 0.02 mmol), after the adding, heating to 100 °C and reacting for 2 hours, after the reaction, cooling to room temperature, concentrating, extracting with EA, combining the organic phases, washing with brine, drying with anhydrous sodium sulfate, and concentrating the organic phases, then purifying by TLC DCM: MeOH (10%) to obtain compound 4 (3.5 mg, yield was 10.98%) as a white solid. ¹ H NMR (400 MHz, DMSO- *d* ₆ ) δ 8.37 (t, *J =* 6.1 Hz, 1H), 8.12 (dd, *J =* 11.8, 1.9 Hz, 1H), 7.71 (d, *J =* 8.2 Hz, 2H), 7.64 - 7.56 (m, 3H), 6.84 (dd, *J =* 8.8, 2.2 Hz, 1H), 4.79 (d, *J =* 6.0 Hz, 2H), 2.62 (s, 3H), 1.60 (s, 3H), 1.57 (s, 3H). LCMS: m/z = 410.1 (M+H ⁺ , ESI).

### Embodiment 8: Synthesis and characterization of compound 5

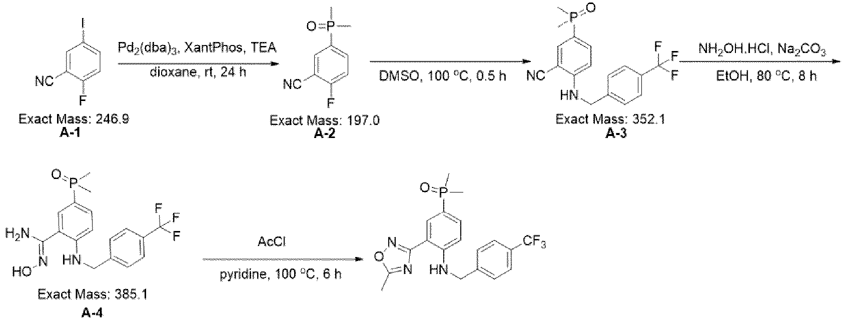

### Synthesis of intermediate A-2:

Adding raw material **A-1** (500 mg, 2.02 mmol), **Pd ₂ (dba)** ₃ (185 mg, 0.20 mmol), **XantPhos** (234 mg, 0.40 mmol), dimethylphosphine oxide (190 mg, 2.43 mmol) and **TEA** (307 mg, 3.04 mmol) to dioxane (8 mL) in sequence, replacing with N₂, and stirring at room temperature overnight. Adding 50 mL of water, extracting with EA, drying with anhydrous sodium sulfate, concentrating the organic phase, and purifying with flash column chromatography DCM- MeOH (0 ~ 10 %) to obtain intermediate A-2 (300 mg, yield 75. 17 %) as a red solid. LCMS: m/z = 198.0 (M+H ⁺ , ESI)

### Synthesis of intermediate A-3:

Dissolving Compound **A-2** (300 mg, 1.52 mmol) in DMSO (5 mL), and adding p-trifluoromethylbenzylamine (533 mg, 3.04 mmol), after the adding, heating to 100°C and reacting for 3 hours. After the reaction, cooling to room temperature, adding 50 mL of water , extracting with EA, drying with anhydrous sodium sulfate, concentrating the organic phase, and then purifying by silica gel column DCM: MeOH (0 ~ 10 %) (1 % TEA) to obtain intermediate **A-3** (600 mg, yield 111.92 %) as a yellow solid. LCMS: m/z = 353.1 (M+H ⁺ , ESI)

### Synthesis of intermediate A-4:

Adding Sodium carbonate (481 mg, 4.54 mmol) to a ethanol (15 mL) suspension of compound **A-3** (400 mg, 1.14 mmol) and hydroxylamine hydrochloride (394 mg, 5.68 mmol), after the adding, heating to 80°C and reacting for 8 hours, after the reaction, cooling to room temperature, evaporating to dryness, extracting with EA, combining the organic phases, and washing with brine, drying with anhydrous sodium sulfate, and concentrating the organic phases to obtain intermediate **A-4** (400 mg, yield 88.22%) as a yellow solid. The product is directly used for the next step without further purification. LCMS: m/z = 400.1 (M+H ⁺ , ESI)

### Synthesis of compound 5:

Under ice bath conditions, adding acetyl chloride (118 mg, 1.50 mmol) to a pyridine (5 mL) solution of compound **A-4** (400 mg, 1.14 mmol), after the adding, heating to 100°C and reacting for 6 hours, after the reaction, cooling to room temperature. Adding an appropriate amount of water to quench the reaction. Directly purify with C18 column water: acetonitrile (0 ~ 50 %) (0.11 % formic acid) to obtain compound 5 (70 mg, yield 17.07 %) as a white solid. ¹ H NMR (400 MHz, DMSO- d ₆ ) δ 8.36 (dd, *J =* 11.9, 1.9 Hz, 1H), 7.71 (d, *J =* 8.1 Hz, 2H), 7.67 - 7.62 (m, 1H), 7.57 (dd, *J =* 8.2, 5.8 Hz, 3H), 6.79 (dd, *J =* 8.7, 2.3 Hz, 1H), 4.77 (d, *J =* 6.0 Hz, 2H), 2.71 (s, 3H), 1.59 (s, 3H), 1.56 (s, 3H). LCMS: m/z = 410.1 (M+H ⁺ , ESI).

### Embodiment 9: Synthesis and characterization of compound 6

According to the general synthetic route 3 , compound **6** is synthesized using 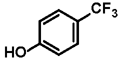 as SM-1. LCMS [M+H] ⁺ =397.

### Embodiment 10: Synthesis and characterization of compound 7

According to the general synthetic route 1, compound 7 is synthesized using 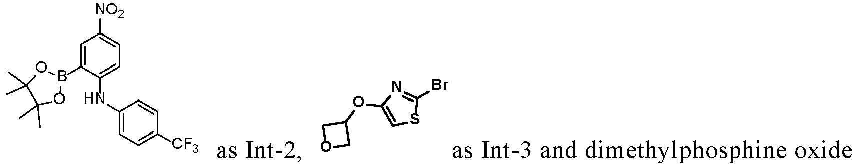 as SM-4 . LCMS [M+H] ⁺ = 473.

### Embodiment 11: Synthesis and characterization of compound 11

According to the general synthetic route 1 , compound 11 is synthesized using 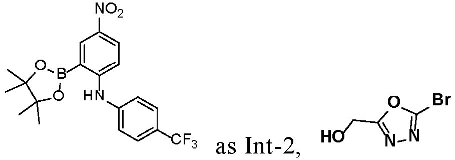 as Int-3 and dimethylphosphine oxide as SM-4 . LCMS [M+H] ⁺ =412.

### Embodiment 12: Synthesis and characterization of compound 12

According to the general synthetic route 1 , compound **12** is synthesized using 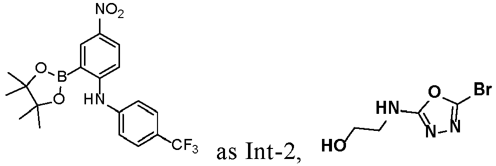 as Int-3 and dimethylphosphine oxide as SM-4 . LCMS [M+H] ⁺ =441.

### Embodiment 13: Synthesis and characterization of compound 13

According to the general synthetic route 2 , compound **13** is synthesized using 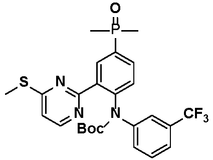 as Int-8-1 and 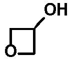 as SM-5 . LCMS [M+H] ⁺ = 464. ¹ H-NMR (400 MHz, DMSO-d ₆ ) δ 11.29 (s, 1H), 8.75 (d, *J =* 6.0 Hz, 1H), 8.70 (dd, *J =* 12.4, 2.0 Hz, 1H), 7.74-7.69 (m, 1H), 7.63-7.57 (m, 3H), 7.41-7.39 (m, 2H), 7.02 (d, *J =* 6.0 Hz, 1H), 5.81-5.75 (m, 1H), 4.97 (t, *J =* 7.2 Hz, 2H), 4.70 (dd, *J =* 8.0, 5.6 Hz, 2H), 1.68 (s, 3H), 1.63 (s, 3H).

### Embodiment 14: Synthesis and characterization of compound 14

According to the general synthetic route 2 , compound **14** is synthesized using 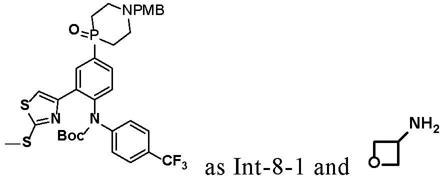 as SM-5 . LCMS [M+H] ⁺ = 509.

### Embodiment 15: Synthesis and characterization of compound 15

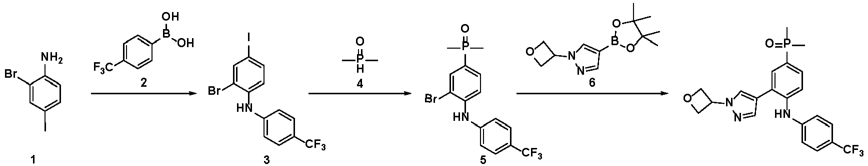

### Step 1: Synthesis of compound 3

Adding compound **1** (3 g, 10.07 mmol), compound 2 (1.91 g, 10.07 mmol), DBU (3.07 g, 20.14 mmol), anhydrous copper acetate (915 mg, 5.03 mmol) to acetonitrile (20 mL), and then adding 4A molecular sieves, replacing with O₂ three times and stirring at 25°C for 16 hours. Filtering, concentrating, and purifying by column (pure PE) to obtain a brown liquid compound **3** (570 mg, 1.29 mmol, yield 12.81%).

### Step 2: Synthesis of compound 5

Adding compound **3** (1.7 g, 3.85 mmol), compound **4** (451 mg, 5.77 mmol), Pd₂dba₃ (353 mg, 384.60 µmol), Xantphos (446 mg, 769.21 µmol), triethylamine (1.17 g, 11.54 mmol) to anhydrous N,N-dimethylformamide (5 mL), replacing with N₂ three times, and reacting at 110°C for 16 hours. Diluting with water, extracting with ethyl acetate, concentrating the organic phase, and purifying by column to obtain the product brown solid compound **5** (800 mg, 2.04 mmol, yield 33.72%, purity 63.64%). LCMS [M+1] ⁺ = 392.0.

### Step 3: Synthesis of compound 15

Adding compound **5** (100 mg, 255.00 µmol), compound **6** (77 mg, 306.01 µmol), cesium carbonate (250 mg, 765.01 µmol), PdCl2(dppf) (19 mg, 25.50 µmol) to 1,4-dioxane (2 mL) and water (0.5 mL), and reacting at 90°C for 2 hours under N₂ protection. Diluting with water, extracting with ethyl acetate, and concentrating the organic phase, preparing and purifying by Pre-HPLC to obtain an off-white solid compound 15 (38 mg, 82.92 umol, yield 32.52%, purity 95.66%). LCMS [M+1] ⁺ = 435.8. ¹ H-NMR (400 MHz, DMSO-d ₆ ) δ 8.23 (d, *J* = 41.2 Hz, 2H), 7.83 (d, *J =* 15.0 Hz, 2H), 7.61 - 7.40 (m, 4H), 6.96 (s, 2H), 5.56 (s, 1H), 4.96 - 4.76 (m, 4H), 1.65 (d, *J =* 13.4 Hz, 6 H).

### Embodiment 16: Synthesis and characterization of compound 16

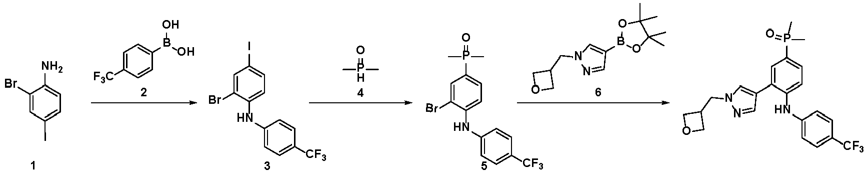

### Step 1: Synthesis of compound 3

Adding compound 1 (3 g, 10.07 mmol), compound 2 (1.91 g, 10.07 mmol), DBU (3.07 g, 20.14 mmol), anhydrous copper acetate (915 mg, 5.03 mmol) to acetonitrile (20 mL), and adding 4A molecular sieves, replacing with O₂ three times and stirring at 25°C for 16 hours, filtering, concentrating, and purifying by column (pure PE) to obtain the product, brown liquid compound 3 (570 mg, 1.29 mmol, yield 12.81%).

### Step 2: Synthesis of compound 5

Adding compound **3** (1.7 g, 3.85 mmol), compound **4** (451 mg, 5.77 mmol), Pd ₂ dba ₃ (353 mg, 384.60 µmol), Xantphos (446 mg, 769.21 µmol), triethylamine (1.17 g, 11.54 mmol) to N,N-dimethylformamide (5 mL), replacing with N₂ three times, and reacting at 110°C for 16 hours. Diluting with water, extracting with ethyl acetate, concentrating the organic phase, and purifying by column to obtain the product brown solid compound **5** (800 mg, 2.04 mmol, yield 33.72%, purity 63.64%). LCMS [M+1] ⁺ = 392.0.

### Step 3: Synthesis of compound 16

Adding compound **5** (100 mg, 0.255 mmol), compound **6** (81 mg, 0.306 mmol), cesium carbonate (250 mg, 0.765 mmol), PdCl2(dppf) (19 mg, 0.02550 mmol) to 1,4-dioxane (2 mL) and water (0.5 mL) and reacting at 90°C for 2 hours. Diluting with water, extracting with ethyl acetate, concentrating the organic phase, preparing and purifying by Pre-HPLC to obtain an off-white solid compound 16 (10.6 mg, 0.0229 mmol, yield 8.97%, purity 97.98%). LCMS [M+1] ⁺ = 449.9. ¹ H NMR (400 MHz, DMSO-d6) δ 8.31 (s, 1H), 8.00 (s, 1H), 7.86 - 7.70 (m, 2H), 7.58 (d, *J =* 11.4 Hz, 2H), 7.42 (d, *J =* 8.0 Hz, 3H), 6.88 (s, 2H), 4.53 (s, 2H), 4.32 (d, *J =* 21.6 Hz, 4H), 1.65 (d, *J =* 13.4 Hz, 7H).

### Embodiment 17: Synthesis and characterization of compound 17

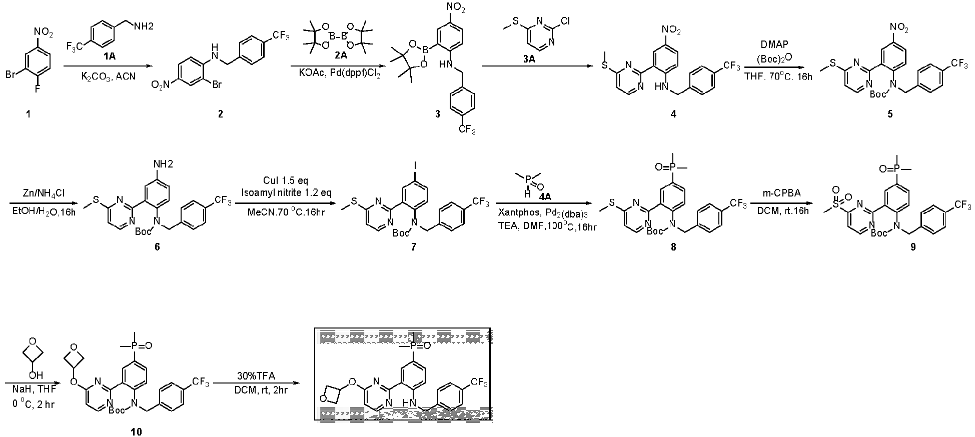

### Synthesis of intermediate 2

Dissolving Compound **1** (3.60 g, 16.37 mmol, 1 eq) in acetonitrile (50 mL), adding K₂CO₃ (9.05 g, 65.47 mmol, 2 eq) and compound **1A** (4.3 g, 24.55 mmol, 1 eq) at 25°C, and then heating to 70°C and stirring overnight, after monitoring the completion of the reaction by LCMS, cooling to room temperature, filtering, adding 200 mL of saturated NH ₄ Cl aqueous solution and 100 mL of EA to the filtrate, separating the filtrate to obtain an organic phase, washing with brine and drying with anhydrous sodium sulfate, concentrating the organic phase, and purifying the crude product by silica gel column (PE:EA = 10:1) to obtain intermediate **2** (3.0 g, purity of 89%, yield of 43.48%) as a yellow solid. LCMS [M+H] ⁺ = 372.7/374.7.

### Synthesis of intermediate 3

Adding intermediate **2** (6.5 g, 17.33 mmol, 1 eq), B ₂ Pin ₂ (4.84 g, 19.06 mmo, 1.2 eq), Pd(dppf)Cl ₂ (1.01 g, 1.39 mmol, 0.1 eq) and potassium acetate (6.93 g, 51.98 mmol, 2 eq) to 1,4-dioxane (100 mL) in sequence, replacing with N₂, heating to 100 °C and stirring overnight, after monitoring the completion of the reaction by LCMS, cooling to room temperature, adding 100 mL of water and 150 mL of EA, filtering, separating the filtrate to obtain an organic phase, drying with anhydrous sodium sulfate, and concentrating the organic phase to obtain Intermediate **3** (6 g, crude product) as a black solid. LCMS [M+H] ⁺ = 422.9.

### Synthesis of intermediate 4

Adding intermediate **3** (9.5 g, 22.50 mmol, crude product), intermediate **3A** (4.34 g, 27.00 mmol, 1.2 eq), Pd(dppf)Cl₂ (1.31 g, 1.8 mmol, 0.08 eq) and cesium carbonate (18.33 g, 56.25 mmol , 2.5 eq) to a mixed solvent of 1,4-dioxane (150 mL) and water (30 mL) in sequence, replacing with N₂, heating to 100 °C and stirring overnight, after monitoring the completion of the reaction by LCMS, cooling to room temperature, adding 100 mL of water and 150 mL of EA, filtering, separating the filtrate to obtain an organic phase, extracting with EA, combining the organic phases, washing with brine, drying with anhydrous sodium sulfate, and concentrating the organic phase to obtain Intermediate **4** (8 g, crude) as a black solid. LCMS [M+H] ⁺ = 420.8.

### Synthesis of intermediate 5

Dissolving intermediate **4** (2 g, 4.76 mmol, 1 eq) in THF (30 mL), and adding Boc ₂ O (2.08 g, 9.51 mmol, 2.19 mL, 2 eq) and DMAP (290.59 mg, 2.38 mmol, 0.5 eq) , and heating to reflux and stirring for 3 hours. After monitoring the completion of the reaction by LCMS, adding 100 mL of aqueous solution, extracting with EA, washing with brine, drying with anhydrous sodium sulfate, and concentrating the organic phase, and then purifying by silica gel column (PE:EA = 5:1) to obtain intermediate 5 (1 g, 1.92 mmol, 40.34% yield, 90% purity) as a yellow solid. LCMS [M + H] ⁺ = 520.8.

### Synthesis of intermediate 6

Dissolving intermediate **5** (2.7 g, 5.19 mmol, 1 eq) in a mixed solvent of EtOH (30 mL) and water (6 mL), and adding Zn powder (2.71 g, 41.50 mmol, 6 eq) and ammonium chloride solid (2.77 g, 51.87 mmol, 10 eq), and heating to reflux and stirring for 3 hours. After monitoring the completion of the reaction by LCMS, filtering, adding 50 mL of aqueous solution, extracting with EA, drying with anhydrous sodium sulfate, and concentrating the organic phase, and then purifying by silica gel column (PE: EA = 1:1) to obtain intermediate **6** (1.70 g, 3.12 mmol, 60.13% yield, 90% purity) as a light yellow solid. LCMS [M + H ⁺ ] = 490.8.

### Synthesis of intermediate 7

Adding intermediate **6** (1 g, 2.04 mmol, 1 eq), isoamyl nitrite (286.58 mg, 2.45 mmol, 1.2 eq) and cuprous iodide (582.37 mg, 3.06 mmol, 1.5 eq) to acetonitrile (20 mL), heating to reflux and stirring for 16 hours. After monitoring the completion of the reaction by LCMS, filtering, adding 50 mL of aqueous solution, extracting with EA, drying with anhydrous sodium sulfate, and concentrating, and purifying by silica gel column (PE: EA = 15:1) to obtain intermediate 7 (300 mg, 0.5 mmol, 24.51% yield, purity 91%) as a light yellow solid. LCMS [M + H] ⁺ = 601.8.

### Synthesis of intermediate 8

Adding intermediate 7 (120 mg, 0.196 mmol, 1 eq), compound **4A** (23 mg, 0.293 mmol, 1.5 eq), Pd ₂ (dba) ₃ (36 mg, 0.039 mmol, 0.2 eq), XantPhos (23 mg, 0.039 mmol , 0.2 eq) and TEA (39 mg, 0.391 mmol, 2 eq) to DMF (5 mL) in sequence, replacing with N₂, and heating to 100 ° C and stirringovernight. After monitoring the completion of the reaction by LCMS, cooling to room temperature, adding 30 mL of water and extracting with EA, drying with anhydrous sodium sulfate, and concentrating the organic phase, and then purifying by TLC (DCM: MeOH = 10:1) to obtain intermediate **8** (95 mg, 0.172 mmol, purity of 91%, yield of 87.84%) as a light yellow solid. LCMS [M+H] ⁺ = 551.8.

### Synthesis of intermediate 9

Dissolving intermediate **8** (1.2 g, 2.18 mmol, 1 eq) in DCM (20 mL), adding mCPBA (750.90 mg, 4.35 mmol, 2 eq) dropwise in DCM solution at 0°C, and stirring at 25°C for 16 hours under N₂ protection. LCMS shows that the reaction is completed. Washing with sodium bicarbonate aqueous solution, washing with brine, drying with anhydrous sodium sulfate, and concentrating to obtain intermediate **9** (1.2 g, crude product) as a light yellow solid. The product is used directly for the next step without further purification. LCMS [M + H] ⁺ = 583.8.

### Synthesis of intermediate 10

Dissolving intermediate **9** (1.6 g, 2.82 mmol, 1 eq) in anhydrous THF (20 ml), replacing with N₂ three times, and adding NaH (246.76 mg, 6.17 mmol, 60% purity, 3 eq) under ice bath, and stirring the mixture for 0.5 hours. Then adding compound **5A** (456.99 mg, 6.17 mmol, 3 eq). The reaction is continued at 0 °C for 2 hours, after monitoring the completion of the reaction by LCMS, adding 50 ml of ice water to quench the reaction, and extracting with EA, drying with anhydrous sodium sulfate, and concentrating the organic phase to obtain intermediate **10** (1.2 g, crude product) as a light yellow solid. LCMS [M + H] ⁺ = 557.8.

### Synthesis of compound 17

Dissolving intermediate 10 (1.6 g, 2.90 mmol, 1 eq) in DCM (20 ml), adding TFA (5 ml) at 0°C, and the mixture is reacted at 25°C for 1 hour. After the reaction, cooling the mixture to 0°C and diluting with water (30 ml), and adding a saturated sodium carbonate aqueous solution dropwise to adjust the mixture to alkalinity, and extracting the aqueous layer with ethyl acetate (25 mL×3 times), and combining the organic layers, washing with saturated brine (25 mL×1 time), drying with anhydrous sodium sulfate, concentrating under reduced pressure, and purifying and freeze-drying by prep-HPLC (ammonia/acetonitrile system) to obtain compound 17 (565 mg, 0.98 mmol, purity of 98.20%, yield of 33.7%) as a light yellow solid. ¹ H NMR (400 MHz, DMSO- *d* ₆ ) δ 9.95 (t, *J =* 6.0 Hz, 1H), 8.72-8.68 (m, 2H), 7.73-7.71 (m, 2H), 7.59-7.51 (m, 3H), 6.96 (d, *J =* 5.6 Hz, 1H), 6.75-6.72 (m, 1H), 5.75-5.69 (m, 1H), 4.96 (t, *J =* 7.2 Hz, 2H), 4.74 (d, *J =* 6.0 Hz, 2 H), 4.70-4.67 (m, 2 H), 1.59 (d, *J* = 13.2 Hz, 6 H). LCMS[M+H] ⁺ = 477.8.

### Embodiment 18: Synthesis and characterization of compound 18

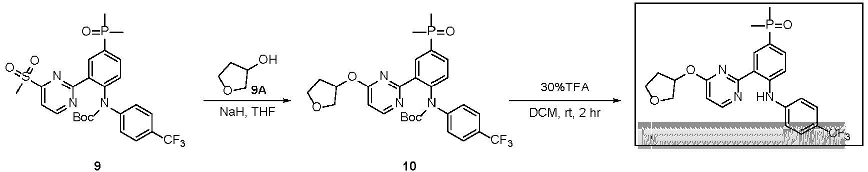

### Synthesis of intermediate 10

Dissolving intermediate **9** (101.87 mg, 1.16 mmol, 1 eq, see the synthesis of compound 8) in anhydrous THF (10 ml), replacing with N₂ three times, and adding NaH (86.72 mg, 2.17 mmol, 60% purity, 6 eq) under ice bath, and stirring the mixture for 0.5 hours. Then, adding a THF solution (5 ml) of compound **9A** (456.99 mg, 6.17 mmol, 3 eq) . The reaction is continued at 0 °C for 2 hours, after monitoring the completion of the reaction by LCMS, adding 50 ml of ice water to quench the reaction, extracting with EA, washing with brine, drying with anhydrous sodium sulfate, and concentrating the organic phase to obtain intermediate **10** (1.2 g, crude product) as a light yellow solid. LCMS [M + H] ⁺ = 577.8.

### Synthesis of compound 18

Dissolving intermediate **10** (200 mg, 346.30 µmol) in 30% TFA of DCM (10 ml), and the mixture is reacted at 25°C for 2 hours. After the reaction, cooling the mixture to 0°C and diluting with EA (20 ml), and adding a saturated sodium carbonate aqueous solution dropwise to adjust the mixture to alkalinity, extracting the aqueous layer with ethyl acetate (20 mL×3 times), combining the organic layers, and washing with saturated brine (25 mL×1 time), drying with anhydrous sodium sulfate, and concentrating under reduced pressure, purifying the crude product by Prep-HPLC (ammonia/acetonitrile system) and lyophilizing to obtain compound 18 (66.7 mg, 139.71 µmol, purity of 96.52%, yield of 40.34%) as a light yellow solid. ¹ H NMR (400 MHz, DMSO- *d* ₆ ) δ 11.35 (s, 1H), 8.79-8.75 (m, 1H), 8.70 (d, *J =* 6.0 Hz, 1H), 7.77-7.72 (m, 1 H), 7.68 (d, *J =* 8.0 Hz, 2 H), 7.58-7.55 (m, 1 H), 7.46 (d, *J =* 8.0 Hz, 2H), 6.95 (d, *J =* 5.6 Hz, 1H), 5.70-5.66 (m, 1H), 4.00 - 3.96 (m, 1H), 3.92-3.85 (m, 2 H), 3.81-3.76 (m, 1 H), 2.35-2.28 (m, 1H), 2.15 - 2.08 (m, 1H), 1.67 (d, *J =* 13.2 Hz, 6H). LCMS[M+H] ⁺ =477.9.

### Embodiment 19: Synthesis and characterization of compound 19

According to the general synthetic route 2 , compound **19** is synthesized using 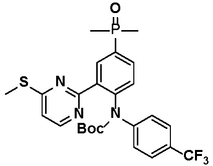 as Int-8-1 and 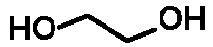 as SM-5. LCMS [M+H] ⁺ = 452.

### Embodiment 20: Synthesis and characterization of compound 20

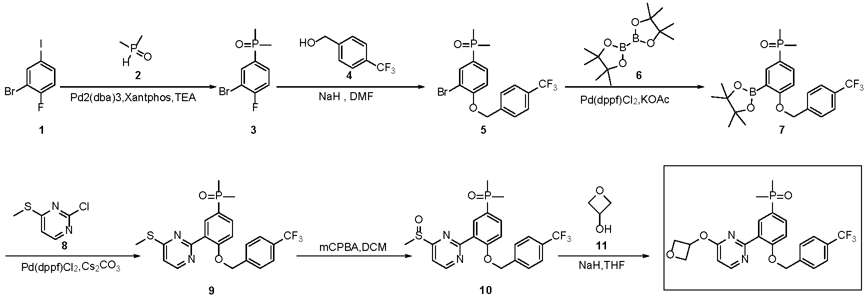

### Synthesis of intermediate 3

At 25 ° C, adding Xantphos (961.50 mg, 1.66 mmol) and Pd2(dba)3 (1.52 g, 1.66 mmol) to a 100 ml three-necked flask respectively, replacing with N₂ three times, and then adding 1,4-dioxance (20 mL) and TEA (2.52 g, 24.93 mmol). The mixture is reacted at 25°C for ten minutes under N₂ protection, and then adding compound **1** (5 g, 16.62 mmol) and compound **2** (1.30 g, 16.62 mmol) and continuing to react for 24 hr. LCMS monitoring shows that the raw material reaction is complete and the product is generated. Adding 100 ml of ethyl acetate to dilute, and washing with 50 ml of water and 50 ml of saturated brine respectively, drying the organic phase with anhydrous sodium sulfate and concentrating under reduced pressure. The obtained crude product is purified by silica gel column (developing solvent: dichloromethane: methanol = 10:1) to obtain intermediate 3 (2.20 g, 7.89 mmol, purity 90%, yield 47.47% ) as a yellow oil. LCMS [M+1] ⁺ = 251/253.

### Synthesis of intermediate 5

Dissolvin compound **4** (2.3 g, 5.65 mmol) in 1,4-dioxane, replacing with N₂, adding NaH (637 mg, 15.93 mmol) at 0 ^{°} C and stirring at this temperature for 0.5 h, then adding compound **3** (2 g, 7.97 mmol), reacting and heating to 80 °C and stirred for 0.5 h, after monitoring the completion of the reaction by LCMS, cooling to room temperature, adding 100 mL of ethyl acetate, and then washing with 100 mL of saturated ammonium chloride aqueous solution and 100 mL of saturated brine, respectively, drying the organic phase with anhydrous sodium sulfate, and concentrating the organic phase to obtain intermediate **5** (2.5 g, crude product) as a yellow oil. LCMS [M+1] ⁺ = 407/409.

### Synthesis of intermediate 7

Adding intermediate **5** (2.3 g, 5.65 mmol, crude product), compound **6** (1.72 g, 6.78 mmol), Pd(dppf)Cl₂ (0.328 g, 0.452 mmol) and anhydrous potassium acetate (1.66 g, 16.95 mmol) to 1,4-dioxane (25 mL) in sequence, replacing with N₂, heating to 100 °C and stirring overnight, after monitoring the completion of the reaction by LCMS, cooling to room temperature, adding 100 mL of water and 150 mL of EA, filtering, separating the filtrate to obtain an organic phase, drying with anhydrous sodium sulfate, and concentrating the organic phase to obtain Intermediate 7 (2.5 g, crude product) as a black oil. LCMS [M + H ⁺ ] = 455.

### Synthesis of intermediate 9

Adding intermediate **7** (1.4 g, 3.08 mmol), compound **8** (0.594 g, 3.7 mmol), Pd(dppf)Cl ₂ (0.224 g, 0.308 mmol) and cesium carbonate (3.01 g, 9.25 mmol) to a mixed solvent of 1,4-dioxane (15 mL) and water (3 mL) in sequence, replacing with N₂, heating to 100 °C and stirring overnight, after monitoring the completion of the reaction by LCMS, cooling to room temperature, adding 100 mL of water and 150 mL of EA,filtering, separating the filtrate to obtain an organic phase, drying with anhydrous sodium sulfate, and concentrating the organic phase, and purifying by silica gel column (DCM: MeOH = 10:1) to obtain intermediate **9** (0.73 g, purity 91%, yield 47.64%) as a light yellow oil. LCMS [M + H ⁺ ] = 453.

### Synthesis of intermediate 10

Dissolving intermediate **9** (350 mg, 0.77 mmol) in anhydrous dichloromethane (5 mL), replacing with N₂, dissolving mCPBA (267 mg, 1.55 mmol) in anhydrous dichloromethane (5 mL), slowly adding to the reaction system at 0°C with a syringe, stirring for 10 min, heating to room temperature and continue stirring for 2 hours. After monitoring the completion of the reaction by LCMS, adding 100 mL of aqueous solution, extracting with EA, drying with anhydrous sodium sulfate, and concentrating the organic phase to obtain intermediate **10** (350 mg, crude product) as a light yellow oil. LCMS[M+H ⁺ ]= 469.

### Synthesis of compound 20

Dissolving compound **11** (166 mg, 2.24 mmol) in anhydrous THF (5 mL), and adding NaH (166 mg, 2.24 mmol) at 0°C, stirring at this temperature for 0.5 hours, dissolving intermediate **10** (350 mg, 0.747 mmol) in anhydrous THF (5 mL) and adding to the reaction, continue to stir at 0°C for 2 hours. After monitoring the completion of the reaction by LCMS, adding 50 mL of aqueous ammonium chloride solution, extracting with EA , drying with anhydrous sodium sulfate, concentrating the organic phase, and then purifying by prep-HPLC to obtain compound 20 (95 mg, purity of 96.7%, yield of 25.0%) as a light yellow solid. LCMS [MH ⁺ ] = 479. ¹ H-NMR (400 MHz, CDCl ₃ ) δ 8.63 (d, *J =* 6.0 Hz, 1 H), 8.03 (dd, *J =* 11.6, 2.0 Hz, 1 H), 7.87-7.82 (m, 1 H), 7.63 (d, *J =* 8.4 Hz, 2 H), 7.52 (d, *J* = 8.4 Hz, 2 H), 7.14 (dd, *J* = 8.8*,* 1.6 Hz, 1 H), 6.77 (d, *J =* 6.0 Hz, 1 H), 5.57-5.51 (m, 1 H), 5.28 (s, 2 H), 4.88-4.85 (m, 2 H), 4.71-4.68 (m, 2 H), 1.75 (d, *J* = 12.8 Hz, 6 H).

### Embodiment 21: Synthesis and characterization of compound 21

According to the general synthetic route 1 , compound **21** is synthesized using 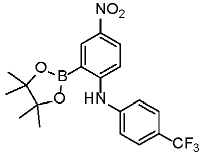 as Int-2, 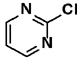 as Int-3, and 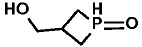 as SM-4. LCMS [M+H] ⁺ = 434.

### Embodiment 22: Synthesis and characterization of compound 22

According to the general synthetic route 1 , compound 22 is synthesized using 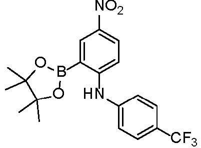 as Int-2, 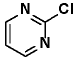 as Int-3, and 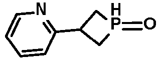 as SM-4. LCMS [M+H] ⁺ =481.

### Embodiment 23: Synthesis and characterization of compound 23

According to the general synthetic route 1 , intermediate 23-A is synthesized using 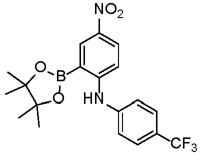 as Int-2, 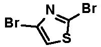 as Int-3 and dimethylphosphine oxide as SM-4. LCMS [M+H] ⁺ = 475, 477.

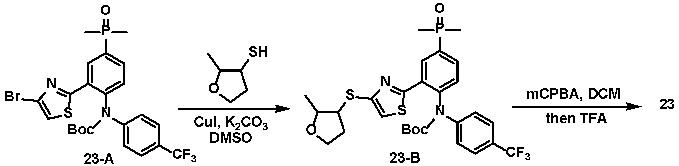

Placing intermediate 23-A (57 mg, 0.1 mmol, 1 eq), 2-methyl-3-thioltetrahydrofuran (59 mg, 0.5 mmol, 5 eq), CuI (4 mg, 0.02 mmol, 0.2 eq), potassium carbonate (69 mg, 0.5 mmol, 5 eq) in a reaction tube, adding 0.5 mL DMSO, replacing with N₂, stirring and placing in a 100°C oil bath to heat for 24 hours. After the reaction, cooling the reaction tube to room temperature, adding water to dilute the system, extracting the aqueous layer with ethyl acetate (15 mL×3 times), combining the organic layers, and washing with saturated brine (25 mL×1 time), drying with anhydrous sodium sulfate, concentrating under reduced pressure, and the residue is purified by prep-HPLC (water/acetonitrile system) and lyophilized to obtain intermediate 23-B (16 mg, 0.026 mmol, purity of 95.4%, yield of 26%) as a brown solid. LCMS [M+H] ⁺ = 513.

Dissolving intermediate 23-B (16 mg, 0.026 mmol, 1 eq) in 0.5 mL DCM and stirring, adding 75% mCPBA (18 mg, 0.078 mmol, 3 eq) under ice bath cooling, naturally returning to room temperature and stirring for 16 hours, monitoring the completion of the reaction by LCMS. Adding 0.1 mL TFA to the system and stirring for 1 hour, monitoring complete removal of Boc by LCMS. Adding water to dilute the system, extracting the aqueous layer with DCM (8 mL × 3 times), combining the organic layers, washing with saturated brine (15 mL × 1 time), drying with anhydrous sodium sulfate, concentrating under reduced pressure, and the residue is purified by prep-HPLC (water/acetonitrile system) and lyophilized to obtain compound 23 (4 mg, 0.0073 mmol, purity 97%, yield 28%) as a yellow solid. LCMS [M+H] ⁺ = 545.

### Embodiment 24: Synthesis and characterization of compound 24

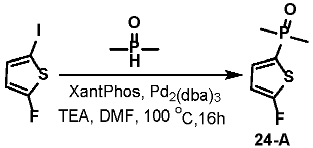

Adding 2-iodo-5-fluorothiophene (1 eq), dimethylphosphine oxide (1.5 eq), **Pd ₂ (dba) ₃** (0.2 eq), **XantPhos** (0.2 eq) and **TEA** (2 eq) to DMF in sequence, replacing with N₂, heating to 100°C and stirring overnight, after monitoring the completion of the reaction by LCM, cooling to room temperature, adding water to dilute, extracting with EA, drying with anhydrous sodium sulfate, concentrating the organic phase, and then purifying by silica gel column chromatography to obtain intermediate 24-A (yield 34%). LCMS [M + H] ⁺ = 179.

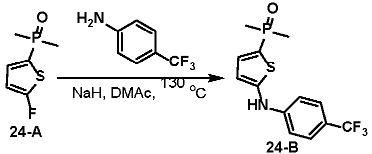

Dissolving p-trifluoromethylaniline (1.5 eq) in DMAc, adding NaH (2 eq) at 0 ° C, stirring for 0.5 hours after addition, dissolving intermediate 24-A (1 eq) in DMAc and adding at 0 °C, after the dropwise addition, heating to 130°C and stirring overnight, after monitoring the completion of the reaction by LCM, cooling to room temperature, adding saturated NH ₄ Cl aqueous solution and EA, filtering, separating the filtrate to obtain organic phase, washing with brine, drying with anhydrous sodium sulfate, concentrating the organic phase, and purifying by silica gel column to obtain intermediate 24-B (yield 23%) as brown oil. LCMS [M+H] ⁺ = 320.

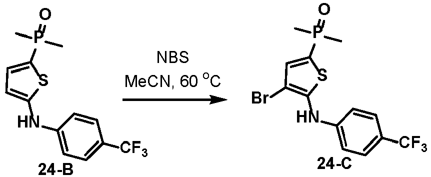

Dissolving intermediate 24-B (1 eq) in acetonitrile, stirring, adding NBS (1.2 eq) at room temperature, and heating to 60 ^{°} C in an oil bath. After monitoring the completion of the reaction by LCM, cooling to room temperature, adding saturated NaHCO3 aqueous solution and EA, filtering, separating the filtrate to obtain organic phase, washing with brine, drying with anhydrous sodium sulfate, concentrating the organic phase, and purifying by a silica gel column to obtain intermediate 24-C (yield 57%) as a reddish brown solid. LCMS [M+H] ⁺ = 398, 400.

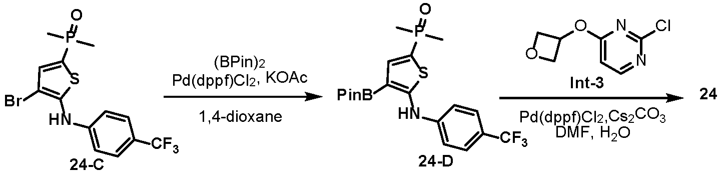

Adding intermediate 24-C (1 eq), (BPin) ₂ (1.2 eq), Pd(dppf)Cl ₂ (0.1 eq) and potassium acetate (2 eq) to 1,4-dioxane in sequence, replacing with N₂, heating to 100°C and stirring overnight, after monitoring the completion of the reaction by LCM, cooling to room temperature, adding water and EA, filtering, separating the filtrate to obtain an organic phase, and drying with anhydrous sodium sulfate, concentrating the organic phase to obtain a crude intermediate 24-D as a black solid.

Adding the crude intermediate 24-D, intermediate Int-3 (1 eq), Pd(dppf)Cl₂ (0.1 eq) and cesium carbonate (2 eq) to a mixed solvent of DMF and water of 5:1 in sequence, replacing with N₂, heating to 100°C and stirring overnight, after monitoring the completion of the reaction by LCM, cooling to room temperature, adding water and EA, filtering, separating the filtrate to obtain an organic phase, and drying with anhydrous sodium sulfate, concentrating the organic phase, and purifying by silica gel column chromatography and prep-HPLC to obtain compound 24 as a light brown solid. LCMS [M+H] ⁺ = 470.

### Embodiment 25: Synthesis and characterization of compound 25

According to the general synthetic route 1 , compound 25 is synthesized using 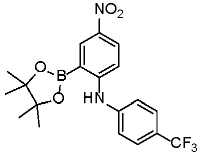 as Int-2, 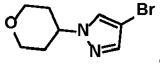 as Int-3 and dimethylphosphine oxide as SM-4. LCMS [M+H] ⁺ =464.

### Embodiment 26: Synthesis and characterization of compound 26

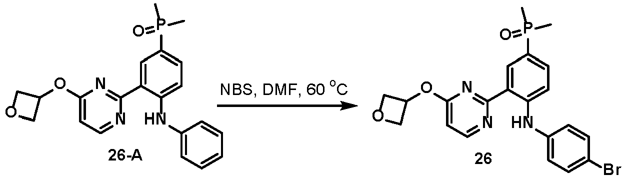

According to the general synthetic route 1 , intermediate 26-A is synthesized using 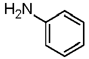 as SM-1, 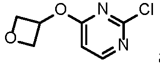 as Int-3 and dimethylphosphine oxide as SM-4.

Dissolving intermediate 26-A (1 eq) in DMF, adding NBS (1.2 eq) under stirring, and heating in an oil bath at 60 ^{°} C overnight. after monitoring the completion of the reaction by LCM, cooling to room temperature, adding water and EA, filtering, separating the filtrate to obtain an organic phase, and drying with anhydrous sodium sulfate, concentrating the organic phase, and purifying by silica gel column chromatography and prep-HPLC to obtain compound 26 as a light yellow solid. LCMS [M+H] ⁺ = 474, 476.

### Embodiments 27-31, Embodiments 37-38, Embodiments 42-46:

### Synthesis and Characterization of Compounds 27-31, 37-38, 42-46

According to the general synthetic route 1 , compound **27** is synthesized using 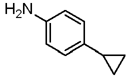 as SM-1, 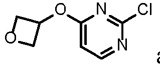 as Int-3 and dimethylphosphine oxide as SM-4 . LCMS [M+H] ⁺ =436.

According to the general synthetic route 1 , compound **28** is synthesized using 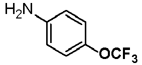 as SM-1, 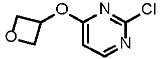 as Int-3 and dimethylphosphine oxide as SM-4. LCMS [M + H] ⁺ = 480. ¹ H-NMR (400 MHz, DMSO- d ₆ ) δ 11.27 (s, 1H), 8.74-8.69 (m, 2H), 7.70-7.65 (m, 1H), 7.46-7.33 (m, 5H), 7.02 (d, *J* = 6.0 Hz, 1H), 5.81-5.76 (m, 1H), 4.98 (t, *J =* 7.2 Hz, 2H), 4.70 (dd, *J =* 7.6, 5.6 Hz, 2H), 1.68-1.61 (m, 6H).

According to the general synthetic route 1 , compound **29** is synthesized using 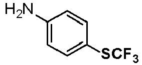 as SM-1, 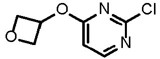 as Int-3 and dimethylphosphine oxide as SM-4 . LCMS [M+H] ⁺ =496. ¹ H-NMR (400 MHz, DMSO- *d* ₆ ) δ 11.34 (s, 1H), 8.74 (d, *J =* 6.0 Hz, 1H), 8.68 (dd, *J =* 12.4, 2.0 Hz, 1H), 7.77-7.72 (m, 1H), 7.66 (d, *J =* 8.4 Hz, 2H), 7.55 (dd, *J =* 8.4, 2.4 Hz, 1H), 7.42 (d, *J =* 8.4 Hz, 2H), 7.03 (d, *J =* 6.0 Hz, 1H), 5.80-5.74 (m, 1H), 4.96 (t, *J =* 7.2 Hz, 2H), 4.69 (dd, *J* = 7.6*,* 5.6 Hz, 2H), 1.70 (s, 3H), 1.66 (s, 3H).

According to the general synthetic route 1 , compound **30** is synthesized using 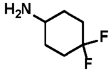 as SM-1, 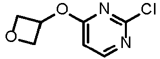 as Int-3 and dimethylphosphine oxide as SM-4 . LCMS [M+H] ⁺ = 438. ¹ H-NMR (400 MHz, DMSO-d ₆ ) δ 9.62 (d, *J =* 8.0 Hz, 1H), 8.67-8.64 (m, 2H), 7.65-7.59 (m, 1H), 7.00-6.92 (m, 2H), 5.77-5.71 (m, 1H), 4.99 (t, *J =* 7.2 Hz, 2H), 4.70-4.68 (m, 2H), 3.90-3.70 (m, 1H), 2.08-2.02 (m, 6H), 1.64-1.57 (m, 8H).

According to the general synthetic route 1, compound **31** is synthesized using 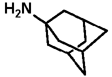 as SM-1, 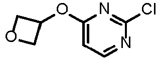 as Int-3 and dimethylphosphine oxide as SM-4. LCMS [M+H] ⁺ = 454.

According to the general synthetic route 1, compound **37** is synthesized using 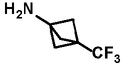 as SM-1, 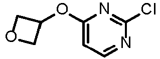 as Int-3 and dimethylphosphine oxide as SM-4. LCMS [M+H] ⁺ = 454.

According to the general synthetic route 1, compound **38** is synthesized using 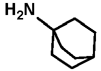 as SM-1, 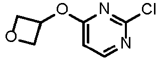 as Int-3 and dimethylphosphine oxide as SM-4 . LCMS [M+H] ⁺ =428.

According to the general synthetic route 1, compound **42** is synthesized using 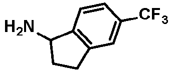 as SM-1, 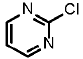 as Int-3 and dimethylphosphine oxide as SM-4. LCMS [M+H] ⁺ = 432.

According to the general synthetic route 1, compound **43** is synthesized using 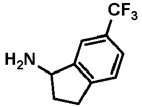 as SM-1, 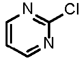 as Int-3 and dimethylphosphine oxide as SM-4. LCMS [M+H] ⁺ =432.

According to the general synthetic route 1 , compound **44** is synthesized using 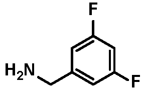 as SM-1, 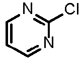 as Int-3 and dimethylphosphine oxide as SM-4. LCMS [M+H] ⁺ = 374.

According to the general synthetic route 1, compound **45** is synthesized using 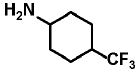 as SM-1, 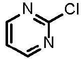 as Int-3 and dimethylphosphine oxide as SM-4 . LCMS [M+H] ⁺ =398.

According to the general synthetic route 1, compound **46** is synthesized using 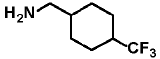 as SM-1, 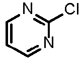 as Int-3 and dimethylphosphine oxide as SM-4. LCMS [M+H] ⁺ =412.

### Embodiment 32: Synthesis and characterization of compound 32

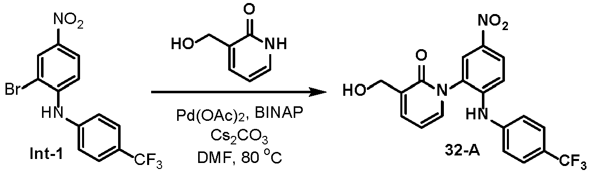

Adding intermediate Int-1 (1 eq), 3-hydroxymethylpyridone (1.2 eq), Pd(OAc) ₂ (0.1 eq), BINAP (0.1 eq) and cesium carbonate (2 eq) to DMF in sequence, replacing with N₂, heating to 80°C and stirring overnight, after monitoring the completion of the reaction by LCM, cooling to room temperature, adding water and EA, filtering, separating the filtrate to obtain an organic phase, and drying with anhydrous sodium sulfate, concentrating the organic phase, and then purifying by silica gel column chromatography to obtain the intermediate 32-A as a yellow solid. LCMS [M + H] ⁺ = 406.

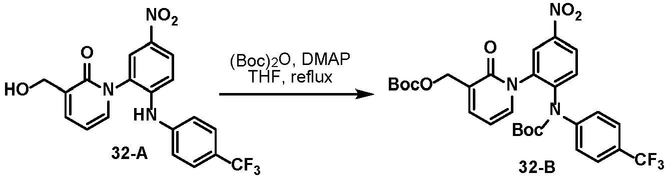

Dissolving intermediate 32-A (1 eq), Boc ₂ O (4 eq), DMAP (0.5 eq) in THF, refluxing and reacting at 70°C for 16 hours, and concentrating under reduced pressure after the reaction. The residue is purified by silica gel column chromatography to obtain intermediate 32-B (yield 61%) as a yellow oil. LCMS [M+Na] ⁺ = 628.

According to the general synthetic route 1, compound 32 is synthesized using 32-B as Int-5 and dimethylphosphine oxide as SM-4. LCMS [M+H] ⁺ =437.

### Embodiment 33: Synthesis and characterization of compound 33

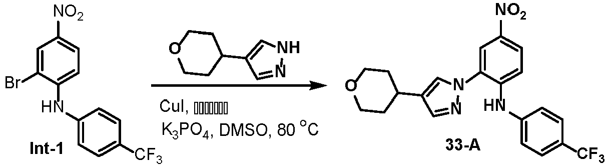

Adding the intermediate Int-1 (1 eq), 4-(4-pyranyl)pyrazole (1.5 eq), CuI (0.2 eq), N,N'-dimethyl trans-cyclohexanediamine (0.2 eq) and potassium phosphate (2 eq) to DMSO in sequence, replacing with N₂, heating to 80°C and stirring overnight, after monitoring the completion of the reaction by LCM, cooling to room temperature, adding water and EA, filtering, separating the filtrate to obtain an organic phase,and drying with anhydrous sodium sulfate, concentrating the organic phase, and then purifying by silica gel column chromatography to obtain the intermediate 33-A as a yellow solid. LCMS [M + H]⁺ = 433.

According to the general synthetic route 1, compound **33** is synthesized using 33-A as Int-4 and dimethylphosphine oxide as SM-4. LCMS [M+H] ⁺ =464.

### Embodiment 34: Synthesis and characterization of compound 34

According to the general synthetic route 1, compound **34** is synthesized using 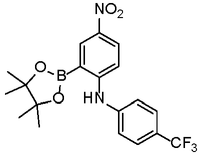 as Int-2, 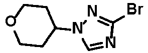 as Int-3 and dimethylphosphine oxide as SM-4. LCMS [M+H] ⁺ =465. ¹ H NMR (400 MHz, DMSO-d6) δ 8.66 (s, 1H), 8.14 (d, J = 0.6 Hz, 1H), 7.86 (ddd, J = 10.4, 8.4, 1.9 Hz, 1H), 7.78 (dd, J = 11.3, 1.9 Hz, 1H), 7.61 (dd, J = 8.4, 2.1 Hz, 1H), 7.58 - 7.52 (m, 2H), 7.22 (d, J = 8.5 Hz, 2H), 4.15 (ddd, J = 11.5, 7.4, 4.0 Hz, 1H), 3.79 - 3.69 (m, 2H), 2.97 (td, J = 12.1, 1.8 Hz, 2H), 2.02 - 1.92 (m, 2H), 1.69 (s, 3H), 1.66 (s, 3H), 1.64 - 1.58 (m, 2H).

### Embodiment 35: Synthesis and characterization of compound 35

According to the general synthetic route 1, compound **35** is synthesized using 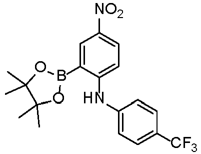 as Int-2, 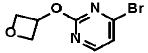 as Int-3 and dimethylphosphine oxide as SM-4. LCMS [M+H] ⁺ =464. ¹ H-NMR (400 MHz, DMSO- *d* ₆ ) δ 9.39 (s, 1H), 8.64 (d, *J* = 5.2 Hz, 1H), 8.03 (dd, *J* = 11.6, 2.0 Hz, 1H), 7.82-7.77 (m, 1H), 7.60-7.53 (m, 4H), 7.22 (d, *J =* 8.4 Hz, 2H), 5.59-5.54 (m, 1H), 4.82 (t, *J =* 7.2 Hz, 2H), 4.61 (dd, *J* = 8.0, 5.2 Hz, 2H), 1.70 (s, 3H), 1.67 (s, 3H).

### Embodiment 36: Synthesis and characterization of compound 36

According to the general synthetic route 1, compound **36** is synthesized using 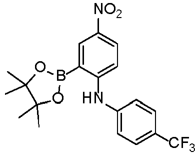 as Int-2, 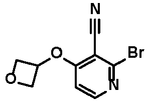 as Int-3 and dimethylphosphine oxide as SM-4. LCMS [M+H] ⁺ = 488.

### Embodiment 39: Synthesis and characterization of compound 39

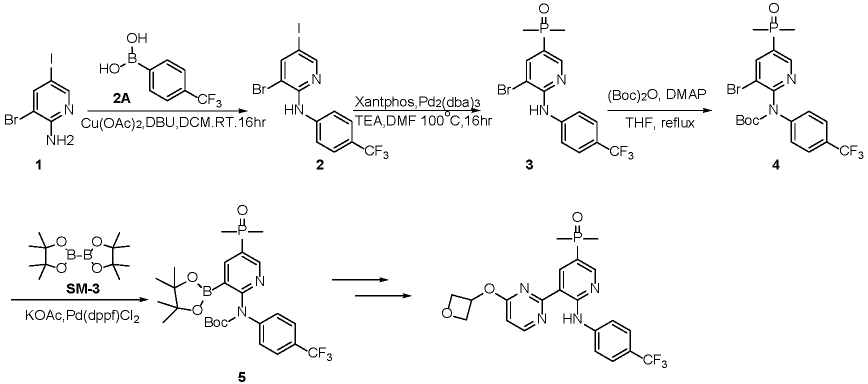

### Synthesis of intermediate 2:

Adding raw material **1** (2.5 g, 8.4 mmol), raw material 2A (2.4 g, 12.5 mmol), **Cu(OAc) ₂** (763 mg, 4.2 mmol) and **DBU** (2.56 g, 16.8 mmol) to DCM (45 mL) in sequence and stirring at room temperature overnight. Concentrating, adding 100 mL of water, extracting with EA, drying with anhydrous sodium sulfate, concentrating the organic phase, and purifying by flash column chromatography to obtain intermediate **2** (307 mg, yield 8.3%) as a brown solid. LCMS: m/z = 443, 445 (M+H ⁺ , ESI).

Starting from intermediate 2, combined with the route in the above figure, according to the method of general synthetic route 1, compound **39** is obtained. LCMS [M+H] ⁺ =465. ¹ H-NMR (400 MHz, DMSO- *d* ₆ ) δ 12.59 (s, 1H), 9.01 (dd, *J* = 11.6, 2.0 Hz, 1H), 8.86 (d, *J* = 6.4 Hz, 1H), 8.72 (q, *J* = 2.4 Hz, 1H), 8.10 (d, *J* = 8.8 Hz, 2H), 7.72 (d, *J* = 8.8 Hz, 2H), 7.12 (d, *J* = 6.0 Hz, 1H), 5.87-5.81 (m, 1H), 4.99 (t, *J* = 6.8 Hz, 2H), 4.71 (dd, *J* = 8.0, 5.2 Hz, 2H), 1.77 (s, 3H), 1.74 (s, 3H).

### Embodiment 40: Synthesis and characterization of compound 40

According to the general synthetic route 1, compound **40** is synthesized using 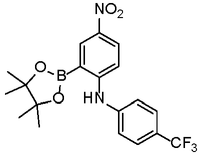 as Int-2, 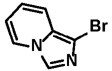 as Int-3 and dimethylphosphine oxide as SM-4. LCMS [M+H] ⁺ =430.

### Embodiment 41: Synthesis and characterization of compound 41

According to the general synthetic route 1 , compound **41** is synthesized using 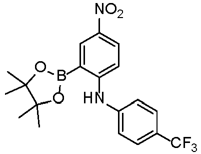 as Int-2, 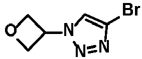 as Int-3 and dimethylphosphine oxide as SM-4. LCMS [M+H] ⁺ =437.

### Embodiment 47: Synthesis and characterization of compound 47

According to the general synthetic route 1, compound **47** is synthesized using 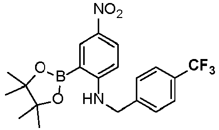 as Int-2, 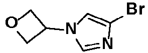 as Int-3 and dimethylphosphine oxide as SM-4. LCMS [M+H] ⁺ =450.

### Embodiment 48: Synthesis and characterization of compound 48

Referring to the synthesis method of compound **32, compound48** may be synthesized using 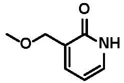 as raw material.

### Embodiment 49: Synthesis and characterization of compound 49

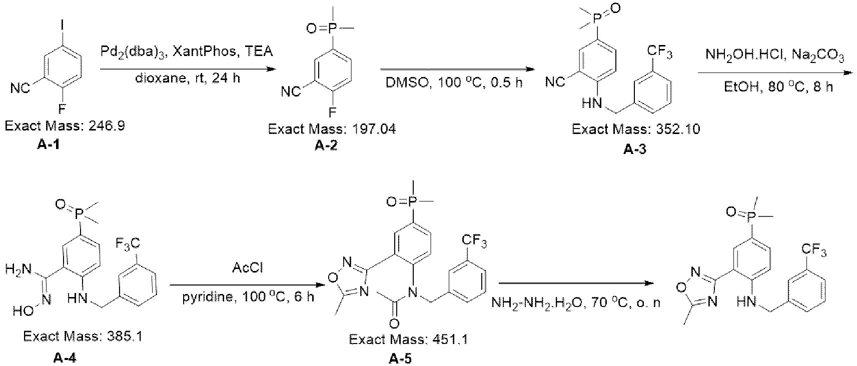

### Synthesis of intermediate A-2:

Adding raw material **A-1** (5.00 g, 20.24 mmol), **Pd ₂ (dba) ₃** (1.85 g, 2.02 mmol), **XantPhos** (2.34 g, 4.05 mmol), dimethylphosphine oxide (1.90 g, 24.29 mmol) and **TEA** (3.07 g, 30.36 mmol) to dioxane (75 mL) in sequence, replacing with nitrogen, and stirring at room temperature overnight. Concentrating, adding 100 mL of water, extracting with EA, drying with anhydrous sodium sulfate, concentrating the organic phase, and purifying by flash column chromatography DCM- MeOH (0 ~ 10 %) to obtain intermediate **A-2** (1.70 g, yield 42.60 %) as a yellow solid. LCMS: m/z = 198.0 (M+H ⁺ , ESI)

### Synthesis of intermediate A-3:

Dissolving compound **A-2** (500 mg, 2.54 mmol) in DMSO (5 mL), and adding m-trifluoromethylbenzylamine (883 mg, 5.07 mmol), after the adding, heating to 100°C and reacting for 0.5 hours. After the reaction, cooling to room temperature, adding 50 mL of water, extracting with EA, combining the organic phases, washing with brine, dried over anhydrous sodium sulfate, drying with anhydrous sodium sulfate, concentrating the organic phase, and purifying by silica gel column DCM: MeOH (0 ~ 10 %) (1 % TEA) to obtain intermediate **A-3** (630 mg, yield 70.51 %) as a yellow solid. LCMS: m/z = 353.1 (M+H ⁺ , ESI)

### Synthesis of intermediate A-4:

Adding sodium carbonate (746 mg, 7.04 mmol) to a ethanol (20 mL) suspension of compound **A-3** (630 mg, 1.76 mmol) and hydroxylamine hydrochloride (611 mg, 8.80 mmol), after the adding, heating to 80°C and reacting for 8 hours, after the reaction, cooling to room temperature, evaporating to dryness, extracting with EA, combining the organic phases, washing with brine, drying with anhydrous sodium sulfate, and concentrating the organic phases to obtain intermediate **A-4** (500 mg, yield 71.14%) as a yellow solid. The product is directly used for the next step without further purification. LCMS: m/z = 386.1 (M+H ⁺ , ESI)

### Synthesis of intermediate A-5:

Under ice bath conditions, adding acetyl chloride (118 mg, 1.50 mmol) to a pyridine (5 mL) solution of compound **A-4** (400 mg, 1.14 mmol), after the adding, heating to 100°C and reacting overnight, after the reaction, cooling to room temperature. Adding an appropriate amount of water to quench the reaction. Directly purify by C18 column at water: acetonitrile (0 ~ 50 %) (0.1 % formic acid) to obtain A-5 (230 mg, yield 50.87 %) as a white solid. LCMS: m/z = 410.1 (M+H ⁺ , ESI)

### Synthesis of compound 49:

Dissolving compound **A-5** (200 mg, 0.44 mmol) in hydrazine hydrate (15 mL, 5.07 mmol), after the addition, heating to 70°C and reacting overnight. After the reaction, cooling to room temperature, and after concentration, directly purifying by C18 column at water: acetonitrile (0 ~ 50 %) (0.1 % formic acid) to obtain compound 49 (30 mg, yield 16.54 %) as a white solid. ¹ H NMR (400 MHz, DMSO- *d* ₆ ) δ 8.36 (dd, *J* = 11.9, 1.9 Hz, 1H), 7.75 (d, *J* = 1.8 Hz, 1H), 7.67 - 7.57 (m, 5H), 6.84 (dd, *J* = 8.7, 2.3 Hz, 1H), 4.75 (d, *J* = 6.0 Hz, 2H), 2.70 (s, 3H), 1.59 (s, 3H), 1.56 (s, 3H). LCMS: m/z = 410.1 (M+H ⁺ , ESI).

### Embodiment 50: Synthesis and characterization of compound 50

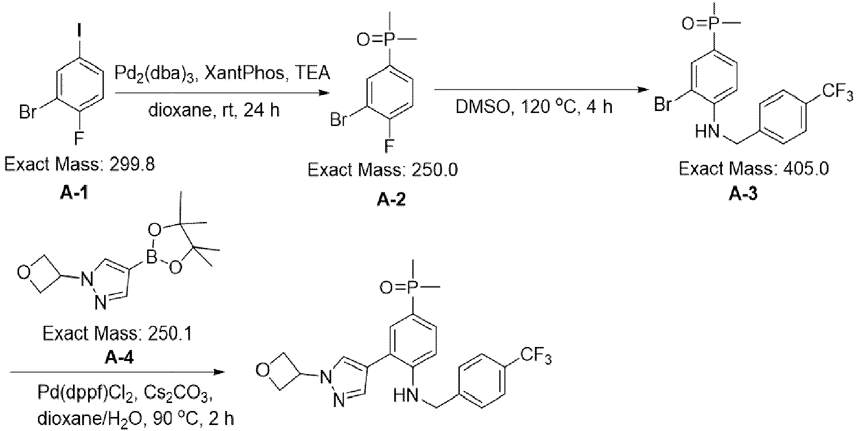

### Synthesis of intermediate A-2:

Adding raw material **A-1** (2.00 g, 6.65 mmol), **Pd₂ (dba)₃** (304 mg, 0.33 mmol), **XantPhos** (385 mg, 0.66 mmol), dimethylphosphine oxide (623 mg, 7.98 mmol) and **TEA** (1.01 g, 9.97 mmol) to dioxane (50 mL) in sequence, replacing with N₂, and stirring at room temperature overnight. Concentrating, adding 100 mL of water, extracting with EA, drying with anhydrous sodium sulfate, concentrating the organic phase, and purifying by flash column chromatography DCM-MeOH (0 ~10 %) to obtain intermediate **A-2** (150 mg, yield 8.99 %) as a red solid. LCMS: m/z = 251.0 (M+H ⁺ , ESI).

### Synthesis of intermediate A-3:

Dissolving compound **A-2** (120 mg, 0.48 mmol) in DMSO (3 mL), and adding p-trifluoromethylbenzylamine (167 mg, 0.96 mmol), after the addition, heating to 120°C and reacting overnight. After the reaction, cooling to room temperature, adding 30 mL of water, extracting with EA, drying with anhydrous sodium sulfate, concentrating the organic phase, and purifying by silica gel column DCM: MeOH (0 ~ 10 %) (1 % TEA) to obtain intermediate **A-3** (100 mg, yield 51.50 %) as a yellow solid. LCMS: m/z = 406.0 (M+H ⁺ , ESI).

### Synthesis of compound 50:

Adding Pd(dppf)Cl₂ (13 mg , 0.002 mmol) and cesium carbonate (112 mg, 0.34 mmol) to a dioxane (0.8 mL) and water (0.2 mL) solution of compound A-3 (70 mg, 0.17 mmol) and A-4 (43 mg, 0.17 mmol). After the addition, replacing with N₂, heating to 90 ° C and reacting for 2 hours. Concentrating, adding 50 mL of water, EA was extracted, extracting with EA, drying with anhydrous sodium sulfate, concentrating the organic phase, and purifying by flash column chromatography C18 at water: acetonitrile (0 ~ 50 %) (0.1 % formic acid) to obtain compound 50 (10 mg, yield 12.91 %) as a white solid. ¹ H NMR (400 MHz, DMSO- *d* ₆ ) δ 8.20 (s, 1H), 7.87 (s, 1H), 7.68 (d, *J* = 8.0 Hz, 2H), 7.57 (d, *J =* 8.0 Hz, 2H), 7.42 (dd, *J* = 11.5, 1.9 Hz, 1H), 7.33 (ddd, *J* = 10.8, 8.3, 2.0 Hz, 1H), 6.48 (dd, *J* = 8.4, 2.4 Hz, 1H), 6.22 (t, *J* = 6.2 Hz, 1H), 5.66 (t, *J* = 7.1 Hz, 1H), 5.02 - 4.94 (m, 4H), 4.50 (d, *J* = 6.0 Hz, 2H), 1.55 (s, 3H), 1.52 (s, 3H). LCMS: m/z = 450.1 (M+H ⁺ , ESI).

### Embodiment 51: Synthesis and characterization of compound 51

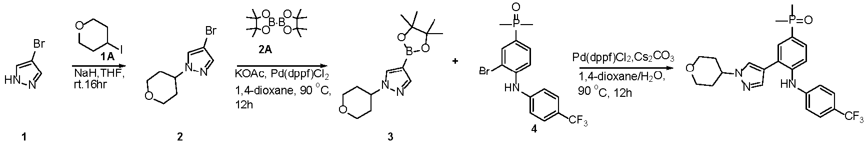

### Synthesis of intermediate 2

Dissolving compound **1** (200 mg, 1.36 mmol) in anhydrous THF (5 mL), and adding NaH (163 mg, 4.08 mmol) at 0°C, stirring at this temperature for 0.5 hours, dissolving compound **1A** (433 mg, 2.04 mmol) in anhydrous THF (5 mL) and adding to the reaction, continuing to stir at room temperature for 16 hours. after monitoring the completion of the reaction by LCMS, adding 50 mL of ammonium chloride aqueous solution, extracting with EA, drying with anhydrous sodium sulfate, concentrating the organic phase, and then purifying by a silica gel column (PE: EA = 1: 1) to obtain P065 (100 mg, purity of 90%, yield of 28.62%) as a yellow solid. LCMS [M+1] ⁺ = 230.9/232.8.

### Synthesis of Intermediate 3

Adding intermediate **3** (300 mg, 1.30 mmol,), compound **2A** (363 mg, 1.43 mmol), Pd(dppf)Cl ₂ (94 mg, 0.130 mmol) and anhydrous potassium acetate (382 mg, 3.89 mmol) to 1,4-dioxane (25 mL) in sequence, replacing with N₂, heating to 90 °C and stirring for 12 hours, and after monitoring the completion of the reaction by LCMS, cooling to room temperature, adding 100 mL of water and 150 mL of EA, filtering, separating the filtrate to obtain an organic phase, drying with anhydrous sodium sulfate, concentrating the organic phase, and purifying by silica gel column (PE: EA = 1: 1) to obtain intermediate 7 (205 mg, purity of 90%, yield of 51.09%) as a yellow solid. LCMS [M+1] ⁺ = 279.30.

### Synthesis of compound 51

Adding intermediate **3** (85 mg, 0.305 mmol), compound **4** (100 mg, 0.255 mmol), Pd(dppf)Cl ₂ (19 mg, 0.250 mmol) and cesium carbonate (250 mg, 0.760 mmol) to a mixed solvent of 1,4-dioxane (5 mL) and water (1 mL) in sequence, replacing with N₂, heating to 90 °C and stirring for 12 hours, and after monitoring the completion of the reaction by LCMS, cooling to room temperature, adding 50 mL of water and 75 mL of EA, filtering, separating the filtrate to obtain an organic phase, drying with anhydrous sodium sulfate, concentrating the organic phase, and purifying by silica gel column (DCM: MeOH = 10: 1) to obtain compound 51 (15.2 mg, purity of 96.47%, yield of 12.37%) as a yellow solid. LCMS[M+H ⁺ ]= 463.9. ¹ H-NMR (400 MHz, DMSO-d6) δ 8..35 (s, 1 H), 8.09 (s, 1 H), 7.86 (dd, *J* = 12, 2.0 Hz, 1 H), 7.76 (s, 1 H), 7.62-7.57 (m, 1H), 7.46-7.42 (m, 3 H), 6.93 (d, *J* = 8.8 Hz, 2 H), 4.42-4.34 (m, 1 H), 3.94-3.91 (m, 2 H), 3.48-3.41 (m, 2 H), 1.92-1.82 (m, 4 H), 1.68 (d, *J* = 13.6 Hz, 6H).

### Embodiment 52: Synthesis and characterization of compound 52

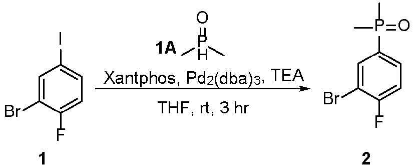

### Synthesis of Intermediate 2

Adding Xantphos (2.88 g, 4.99 mmol, 0.05 eq), Pd₂ (dba)₃ (4.56 g, 4.99 mmol, 0.05 eq), TEA (15.13 g, 149.55 mmol, 21.02 mL, 1.5 eq) to THF (200 mL) and replacing with N₂ three times. Stirring at 25 °C for 30 minutes, then adding a THF (100 mL) solution of compound **1** (30 g, 99.70 mmol, 1 eq) and compound **1A** (8 g, 102.50 mmol, 1.03 eq), and continuing to stir for 3 hours. LCMS monitoring showing that the raw material reaction is completed and product is generated. Filtering and concentrating, and purifying the obtained crude product by silica gel column (eluent: dichloromethane/methanol = 20/1) to obtain intermediate **2** (15.60 g, 59.04 mmol, 59.21% yield, 95% purity) as a yellow oil. LCMS [M+H] ⁺ = 250.8

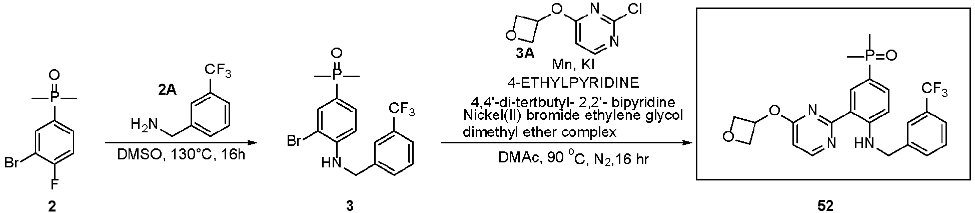

### Synthesis of Intermediate 3

Dissolving 2 (1 g, 3.98 mmol, 1 eq) was ed in DMSO (5 ml), and adding compound **2A** (1.40 g, 7.97 mmol, 2 eq), stirring the mixture at 130°C for 16 hours, LCMS shows that the product is generated, adding water to dilute, extracting with EA, washing with 1N hydrochloric acid aqueous solution and saturated brine, drying with anhydrous sodium sulfate, concentrating under reduced pressure, and purifying the residue by reverse phase column (acetonitrile/pure water) to obtain intermediate **3** (360.00 mg, 797.68 µmol, 20.02% yield, 90.00% purity) as a yellow oil. LCMS [M+H] ⁺ = 405.7

### Synthesis of compound 52

Dissolving intermediate **3A** (330.76 mg, 1.77 mmol, 2 eq), intermediate **3** (360 mg, 886.31 µmol, 1 eq), KI (176.55 mg, 1.06 mmol, 1.2 eq), Mn (194.77 mg, 3.55 mmol, 4 eq), 4-ETHYLPYRIDINE (94.97 mg, 886.31 µmol, 100.82 µL, 1 eq), 4,4'-di-tertbutyl- 2,2'- bipyridine (118.94 mg, 443.16 µmol, 0.5 eq), Nickel(II) bromide ethylene glycol dimethyl ether complex (136.77 mg, 443.16 µmol, 0.5 eq) in DMAc (10 mL), and stirring the mixture at 90°C for 16 h. LCMS shows that the reaction is completed, adding water (100 mL), extracting with EA (100 mL × 3), washing the organic phase with saturated brine (100 mL × 2), drying with anhydrous sodium sulfate, concentrating, and purifying by Prep-HPLC to obtain compound **52** (85.00 mg, 174.84 µmol, 19.73% yield, 98.74% purity) as a white solid. ¹ H-NMR (400 MHz, DMSO-d ₆ ) δ 9.96 (t, *J*= 6.0 Hz, 1H), 8.71-8.64 (m, 2H), 7.75 (s, 1H), 7.68-7.53 (m, 4H), 6.96 (d, *J* = 6.0 Hz, 1H), 6.79-6.74 (m, 1H), 5.76-5.68 (m, 1H), 4.96 (t, *J* = 7.2 Hz, 2H), 4.74-4.66 (m, 4H), 1.65-1.59 (m, 6H). LCMS[M+H] ⁺ = 477.8.

### Embodiment 53: Synthesis and characterization of compound 53

According to the general synthetic route 1 , compound **53** is synthesized by using 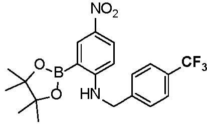 as Int-2, 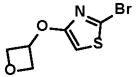 as Int-3 and dimethylphosphine oxide as SM-4 . LCMS [M+H] ⁺ =487.

### Embodiment 54: Synthesis and characterization of compound 54

According to the synthetic route of compound **17** , compound **54 is** synthesized by using the intermediate 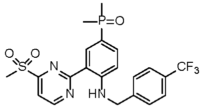 as the raw material and replacing 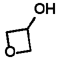 with 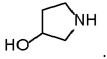 LCMS [M+H] ⁺ =491.

### Embodiment 55: Synthesis and characterization of compound 55

According to the general synthetic route 1 , compound **55** is synthesized by using 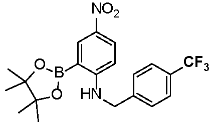 as Int-2, 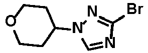 as Int-3 and dimethylphosphine oxide as SM-4. LCMS [M+H] ⁺ =479.

### Embodiment 56: Synthesis and characterization of compound 56

According to the general synthetic route 1 , compound **56** is synthesized by using 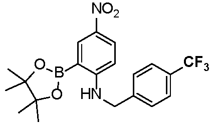 as Int-2, 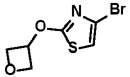 as Int-3 and dimethylphosphine oxide as SM-4. LCMS [M+H] ⁺ =487.

### Embodiment 57: Synthesis and characterization of compound 57

According to the general synthetic route 1, compound **57** is synthesized by using 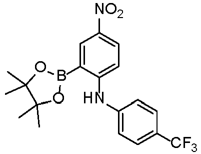 as Int-2, 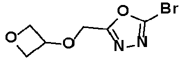 as Int-3 and dimethylphosphine oxide as SM-4. LCMS [M+H] ⁺ =468.

### Embodiment 58: Synthesis and characterization of compound 58

According to the synthetic route of compound **33** , the intermediate 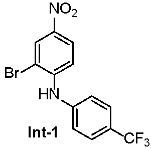 is replaced with 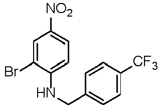 to synthesize compound 58. LCMS [M+H] ⁺ =478.

### Embodiment 59: Synthesis and characterization of compound 59

According to the synthetic route of compound **32** , the intermediate 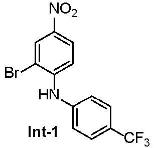 is replaced with 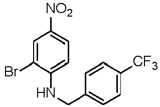 and the raw material 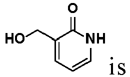 replaced by 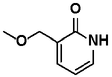 to synthesize compound **59.** LCMS [M+H] ⁺ =465.

### Embodiment 60: Synthesis and characterization of compound 60

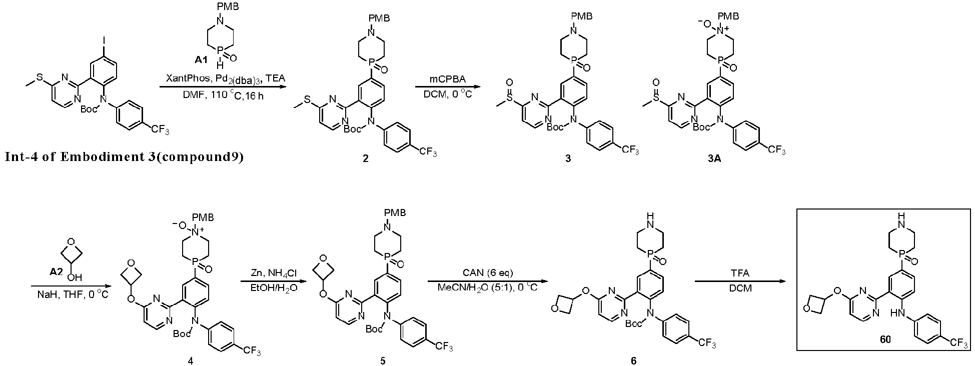

### Synthesis of Intermediate 2

Under N₂ protection, dissolving XantPhos (39.40 mg, 0.068 mmol, 0.2 eq), Pd _{**2** (dba) 3} (31.18 mg, 0.034 mmol, 0.1 eq), TEA (103.36 mg, 1.02 mmol, 3 eq) were ed in anhydrous DMF (5 ml), and stirring the mixture at room temperature for 30 minutes. Then adding a DMF ( 5 ml) solution of **Int4** (200 mg, 0.34 mmol, 1 eq) of Embodiment 3 and **A1** (122.19 mg, 0.51 mmol, 1.5 eq), and the mixture continues to react at 110°C for 16 hours, after monitoring the completion of the reaction by LCMS, cooling to room temperature, adding water (50 mL) to dilute, extracting with ethyl acetate (40 mL×3 times), and washing the organic phase with saturated brine (50 mL×2 times), then drying with anhydrous sodium sulfate, and finally concentrating under reduced pressure. The obtained crude product is purified by silica gel column (eluent: dichloromethane/methanol = 20/1) to obtain intermediate **2** (150 mg, 0.21 mmol, purity 86%, yield 61.8%) as a yellow solid. LCMS [M+H] ⁺ = 698.8.

### Synthesis of Intermediate 3

Dissolving intermediate **2** (200 mg, 0.29 mmol, 1 eq) in ultra-dry dichloromethane (10 mL), cooling to 0°C under N₂ protection, adding mCPBA (98.79 mg, 0.57 mmol, 2 eq), then removing the ice bath and naturally returning to room temperature (25°C). Continuing to stir until monitoring the completion of the reaction by LCMS, filtering, and slurrying with DCM to obtain a mixture of intermediate **3** and **3A** (180 mg, 0.25 mmol, purity of 79%, yield of 86%) as a yellow solid. Intermediate **3** : LCMS [M+H] ⁺ = 714.7, intermediate **3A** : LCMS [M+H] ⁺ = 730.7.

### Synthesis of Intermediate 4

Dissolving compound **A2** (40.55 mg, 0.55 mmol, 2 eq) in ultra-dry THF (10 ml), replacing with N₂ three times, and adding NaH (43.79 mg, 1.09 mmol, 60% purity, 4 eq) under ice bath, and stirring the mixture for 0.5 hours, then adding to Intermediate **3** (196 mg, 0.27 mmol, 1 eq), continuing to react at 0°C for 2 hours, after monitoring the completion of the reaction by LCMS, adding 10 ml of ice water to quench the reaction, extracting with EA (40 mL×3 times), and washing the organic phase with saturated brine (50 mL×2 times), then drying with anhydrous sodium sulfate, and concentrating the organic phase to obtain Intermediate **4** (180 mg, crude product) as a yellow solid. LCMS[M+H] ⁺ = 740.8.

### Synthesis of Intermediate 5

Dissolving intermediate **4** (600 mg, 810.04 µmol, 1 eq), Zn (529.69 mg, 8.10 mmol, 10 eq), NH ₄ Cl (649.95 mg, 12.15 mmol, 15 eq)) in EtOH (25 mL) and H ₂ O (5 mL), and stirring at 70°C for 16 hours under N₂ protection. LCMS shows that the reaction is complete, filtering, adding water (50 mL) and EA (50 mL) the separating, extracting the aqueous phase with EA, combining the organic phases, washing with brine, drying with anhydrous sodium sulfate, and concentrating to obtain Intermediate **5** (500 mg, crude product) as a yellow solid. LCMS [M+H] ⁺ = 724.8.

### Synthesis of Intermediate 6

Dissolving intermediate **5** (200 mg, 270.73 µmol, 1 eq) and CAN (861.23 mg, 1.62 mmol, 6 eq) in MeCN (10 mL) and stirring at 0°C for 3 hours under N₂ protection. LCMS shows that the reaction is completed, adding water (20 mL) and EA (20 mL) the separating, extracting the aqueous phase with EA (20mL × 3), combining the organic phases, washing with brine, drying with anhydrous sodium sulfate, and concentrating to obtain Intermediate **6** (150 mg, crude product) as a yellow solid. LCMS [M+H] ⁺ = 605.3.

### Synthesis of Compound 60

Dissolving intermediate **6** (50 mg, 80.83 µmol) in a 30% TFA solution of DCM (20 mL) and stirring at 25°C for 3 hours under N₂ protection. LCMS shows that the reaction is completed, adding EA to dilute, cooling to 0°C, adjusting the pH to alkaline with sodium bicarbonate aqueous solution, extracting with EA, washing the organic phase with brine, drying with anhydrous sodium sulfate, concentrating, and purifying by Prep-HPLC to obtain compound **60** (2.00 mg, 3.67 µmol, 4.54% yield, 95.09% purity) as a white solid. ¹ H-NMR (400 MHz, DMSO- *d* ₆ ) δ 11.39 (s, 1 H), 8.76 (d, *J* = 5.6 Hz, 1H), 8.71-8.66 (m, 1H), 7.76-7.68 (m, 3H), 7.58 (dd, *J* = 8.4, 2.4 Hz, 1H), 7.48 (d, *J* = 8.8 Hz, 2H), 7.04 (d, *J* = 6.0 Hz, 1H), 5.80-5.74 (m, 1H), 4.96 (t, *J* = 7.2 Hz, 2H), 4.71-4.67 (m, 2H), 3.68-3.57 (m, 1H), 3.16-2.93 (m, 3H), 2.16-1.97 (m, 2H), 1.86-1.72 (m, 2H). LCMS[M+H] ⁺ = 504.9.

### Embodiment 61: Synthesis and characterization of compound 61

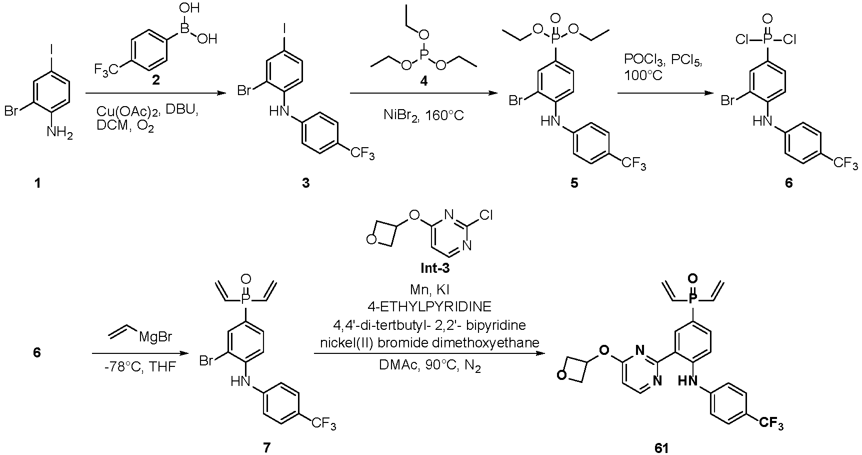

### Synthesis of intermediate 3

Adding compound 1 (3 g, 10.07 mmol), compound 2 (1.91 g, 10.07 mmol), DBU (3.07 g, 20.14 mmol), anhydrous copper acetate (915 mg, 5.03 mmol) to dichloromethane (20 mL), and then adding 4A molecular sieves, replacing with O₂ three times and stirring at 25°C for 16 hours. Filtering, concentrating, and purifying by column (pure PE) to obtain the brown liquid intermediate 3 (600 mg, 1.36 mmol, yield 13.5%).

### Synthesis of Intermediate 5

Adding compound 3 (3 g, 6.79 mmol), compound 4 (1.18 g, 7.13 mmol), and nickel bromide (149 mg, 678.71 µmol) into a single-necked bottle, replacing with N₂ three times, and reacting at 160°C for 6 hours. Adding water to dilute, extracting with ethyl acetate, concentrating the organic phase, and purifying by column to obtain the product brown solid intermediate 5 (1.5 g, 3.32 mmol, yield 48.89%). LCMS [M+1] + = 452.0.

### Synthesis of Intermediate 6

Adding compound 5 (1 g, 2.21 mmol) and phosphorus oxychloride (679 mg, 4.42 mmol) into a three-necked flask, replacing with N₂ three times, and adding phosphorus pentachloride (1.15 g, 5.53 mmol) under ice-water bath conditions, reacting at 100°C for 16 hours, concentrating to obtain a crude product brown solid compound 6 (900 mg), which is used directly in the next step.

### Synthesis of Intermediate 7

Adding compound 6 (900 mg) and tetrahydrofuran (10 mL) into a three-necked flask, replacing with N₂ three times, adding vinylmagnesium bromide (1.36 g, 10.39 mmol) dropwise at -78°C, and reacting for 2 hours at -78°C. Adding ice water to quench the reaction, extracting with ethyl acetate, concentrating the organic phase, and purifying by column chromatography to obtain a product, brown solid intermediate 7 (400 mg). LCMS [M+H] ⁺ = 415.7/417.7.

### Synthesis of compound 61

Dissolving intermediate Int-3 (269.02 mg, 1.44 mmol, 2 eq), intermediate 7 (300 mg, 720.86 µmol, 1 eq), 4-ETHYLPYRIDINE (77.24 mg, 720.86 µmol, 82.00 µL, 1 eq), Mn (237.62 mg, 4.33 mmol, 6 eq), KI (143.59 mg, 865.03 µmol, 1.2 eq) , 4,4'-di-tertbutyl- 2,2'- bipyridine (580.43 mg, 2.16 mmol, 3 eq), Nickel(II) bromide ethylene glycol dimethyl ether complex (667.41 mg, 2.16 mmol, 3 eq) in DMAc (20% MgSO4). mL), stirring at 100°C for 16 hours under N₂ protection. After LCMS showing that the reaction is complete, adding water (100 mL), extracting with EA (100 mL × 3), washing the organic phase with saturated brine (100 mL × 2), drying with anhydrous sodium sulfate, concentrating, and purifying by reverse phase column (H **₂** O (0.1% NH ₄ OH) / ACN) to obtain **compound 61** (7.20 mg, 14.77 µmol, 2.05% yield, 98.47% purity) as a yellow solid. ¹ H-NMR (400 MHz, DMSO- *d* ₆ ) δ 11.44 (s, 1H), 8.75 (d, *J* = 6.0 Hz, 1H), 8.62 (d, *J* = 12.8 Hz, 1H), 7.70-7.56 (m, 4H), 7.48 (d, *J* = 7.7 Hz, 2H), 7.04 (d, *J* = 6.0 Hz, 1H), 6.78-6.64 (m, 2H), 6.31 (d, *J* = 12.6 Hz, 1H), 6.22-6.12 (m, 3H), 5.75-5.70 (m, 1H), 4.95 (t, *J* = 6.6 Hz, 2H), 4.70-4.60 (m, 2H). LCMS[M+H] ⁺ = 487.5.

### Embodiment 62: Synthesis and characterization of compound 62

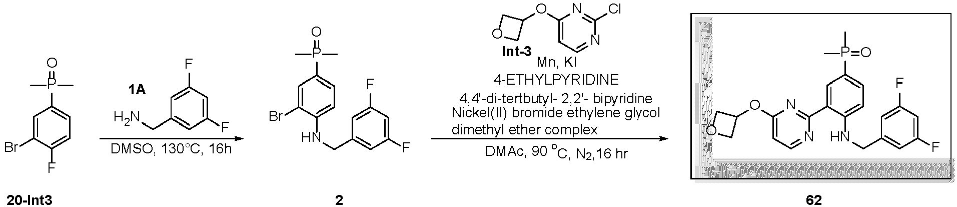

### Synthesis of Intermediate 2

Dissolving the intermediate 20-Int3 (300 mg, 1.20 mmol, 1 eq) of **compound 20** in DMSO (5 mL), and adding compound **1A** (342.11 mg, 2.39 mmol, 2 eq), stirring the mixture at 130°C for 16 hours, after LCMS showing that the product is generated, adding water to dilute, extracting with EA, washing with 1N hydrochloric acid aqueous solution and saturated brine, drying with anhydrous sodium sulfate, and concentrating under reduced pressure to obtain Intermediate **2** (120.00 mg, crude product) as a yellow solid. LCMS [M+H] ⁺ = 373.7.

### Synthesis of compound 62

Dissolving intermediate Int-3 (149.61 mg, 801.80 µmol, 2 eq), intermediate **2** (150 mg, 400.90 µmol, 1 eq), KI (79.86 mg, 481.08 µmol, 1.2 eq), Mn (44.05 mg, 801.80 µmol, 2 eq), 4-ETHYLPYRIDINE (42.96 mg, 400.90 µmol, 1 eq), 4,4'-di-tertbutyl-2,2'-bipyridine (53.80 mg, 200.45 µmol, 0.5 eq), Nickel(II) bromide ethylene glycol dimethyl ether complex (61.86 mg, 200.45 µmol, 0.5 eq) in DMAc, stirring the mixture at 90°C for 16 hours, after LCMS showing that the reaction is completed, adding water ( 100 mL), extracting with EA (100 mL × 3), washing the organic phase with saturated brine (100 mL × 2), drying with anhydrous sodium sulfate, concentrating, and purifying by Prep-HPLC to obtain **compound 62** (60.00 mg, 134.71 µmol, 33.60% yield, 100% purity) as a white solid. ¹ H-NMR (400 MHz, DMSO- *d* ₆ ) δ 9.92 (t, *J* = 6.0 Hz, 1H), 8.71-8.65 (m, 2H), 7.58-7.53 (m, 1H), 7.15-7.05 (m, 3H), 6.99-6.92 (m, 1H), 6.73 (dd, *J* = 8.7, 2.4 Hz, 1H), 5.77-5.71 (m, 1H), 4.97 (t, *J* = 7.2 Hz, 2H), 4.71-4.66 (m, 4H), 1.60 (dd, *J* = 28.2, 15.2 Hz, 6H). LCMS[M+H] ⁺ = 445.8.

### Embodiment 63: Synthesis and characterization of compound 63

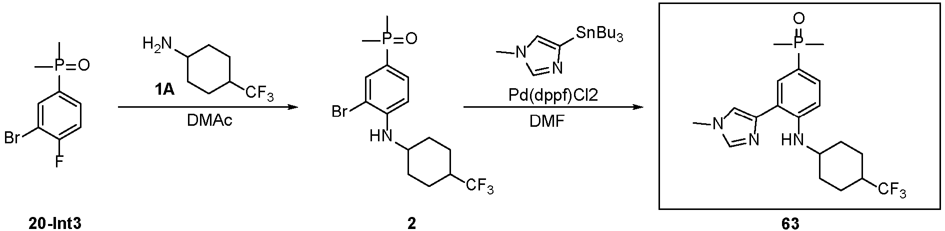

### Synthesis of Intermediate 2

Dissolving the intermediate 20-Int3 (240 mg, 956.05 µmol, 1 eq) of compound **20 and compound 1A** (1.04 g, 6.21 mmol, 6.5 eq) in DMAc (4 mL), and stirring the mixture in a microwave at 160°C for 4 hours, after LCMS monitoring the completion of the reaction, cooling to room temperature, adding water (20 mL) to dilute, extracting with ethyl acetate (20 mL×3 times), and washing the organic phase with saturated brine (20 mL×2 times), then drying with anhydrous sodium sulfate, and finally concentrating under reduced pressure. The obtained crude product is purified by silica gel column (eluent: dichloromethane/methanol = 20/1) to obtain intermediate **2** (70 mg, 0.18 mmol, 18.75% yield, 84.56% purity) as a black oil. LCMS[M+H] ⁺ = 398.0.

### Synthesis of compound 63

Dissolving intermediate **2** (80 mg, 200.91 µmol, 1 eq) in DMF (3 ml), and adding Pd(dppf)Cl₂ (29.16 mg, 40.18 µmol, 0.2 eq), stirring the mixture in a microwave at 160°C for 4 hours, after LCMS showing that the product is generated and the reaction is completed, adding water (30 mL), extracting with EA (30 mL × 3), and washing the organic phase with saturated brine (30 mL×2), drying with anhydrous sodium sulfate, concentrating, and purifying by Prep-HPLC to obtain **compound 63** (9.50 mg, 23.73 µmol, 11.81% yield, 99.77% purity) as a white solid. ¹ H-NMR (400 MHz, DMSO-d ₆ ) δ 9.05 (d, *J* = 8.4, Hz, 1H), 7.79-7.66 (m, 3H), 7.38-7.32 (m, 1H), 6.85-6.72 (m, 1H), 3.90-3.88 (m, 1H), 3.76-3.68 (m, 3H), 2.43-2.09 (m, 2H), 1.99-1.86 (m, 2H), 1.76-1.61 (m, 4H), 1.59-1.55 (m, 6H), 1.49-1.26 (m, 1H) . LCMS[M+H] ⁺ = 400.2.

### Embodiment 64: Synthesis and characterization of compound 64

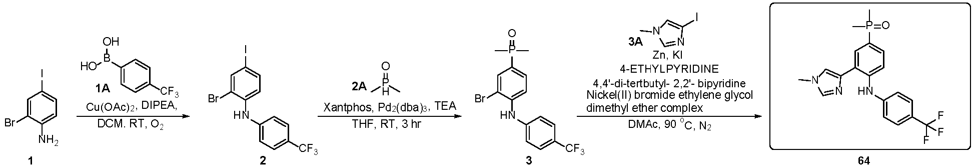

### Synthesis of Intermediate 2

Dissolving compound **1** (10 g, 33.57 mmol, 1 eq), compound **1A** (9.56 g, 50.35 mmol, 1.5 eq), anhydrous copper acetate (13.40 g, 67.13 mmol, 2 eq), DBU (21.69 g, 167.83 mmol, 29.23 mL, 5 eq) in DCM (250 mL) and stirring at room temperature for 48 hours under O₂ atmosphere. LCMS shows that the raw material remain and the reaction is not continued for a longer time, filtering, concentrating, and purifying by silica gel column (eluent: PE/EA = 10/1) to obtain Intermediate **2** (7 g, 15.84 mmol, purity of 82%, yield of 47.18%) as a yellow solid. LCMS: The product has no molecular weight.

### Synthesis of Intermediate 3

Under N₂ protection, dissolving XantPhos (1.31 g, 2.26 mmol, 0.05 eq), Pd₂ (dba)₃ (1.24 g, 1.36 mmol, 0.03 eq), TEA (9.16 g, 90.50 mmol, 12.58 mL, 2 eq) in anhydrous THF (300 ml), and stirring the mixture at room temperature for 30 minutes. Then, adding a THF (100 ml) solution of Intermediate 2 (20 g, 45.25 mmol, 1 eq) and Compound **2A** (3.88 g, 49.77 mmol, 1.1 eq), and stirring the mixture at room temperature for 3 hours, after monitoring the completion of the reaction by LCMS, filtering, and concentrating under reduced pressure. The obtained crude product is purified by silica gel column (eluent: dichloromethane/methanol = 20/1) to obtain intermediate **3** (13 g, 33.15 mmol, purity 88%, yield 73.26%) as a yellow solid. LCMS [M+H] ⁺ = 392.0.

### Synthesis of compound 64

Dissolving intermediate **3A** (9.38 g, 45.08 mmol, 1.7 eq), intermediate **3** (10.4 g, 26.52 mmol, 1 eq), 4-ETHYLPYRIDINE (2.84 g, 26.52 mmol, 3.02 mL, 1 eq), Zn (10.41 g, 159.12 mmol, 6 eq), KI (5.28 g, 31.82 mmol. 1.2 eq), 4,4'-di-tertbutyl-2,2'-bipyridine (7.12 g, 26.52 mmol, 1 eq), Nickel(II) bromide ethylene glycol dimethyl ether complex (8.18 g, 26.52 mmol, 1 eq) in DMAc (150 mL ). mL) and stirring at 90°C for 16 hours under N₂ protection. After LCMS showing that the reaction is completed, adding water (300 mL), extracting with EA (300 mL × 3), and washing the organic phase with saturated brine (300 mL × 2), drying with anhydrous sodium sulfate, concentrating, and purifying by reverse phase column (H₂O (0.1% NH ₄ OH) / ACN) to obtain **compound 64** (2.00 g, 5.08 mmol, 19.17% yield, 98.46% purity) as a white solid. ¹ H-NMR (400 MHz, DMSO- *d* ₆ ) δ 10.81 (s, 1H), 8.04 (d, *J* = 12.0 Hz, 1H), 7.81 (t, *J* = 24.8 Hz, 2H), 7.61-7.52 (m, 4H), 7.31 (d, *J* = 8.4 Hz, 2H), 3.75 (s, 3H), 1.67 (d, *J* = 13.2 Hz, 6H). LCMS[M+H] ⁺ = 394.0.

### Embodiment 65: Synthesis and characterization of compound 65

According to the synthetic route of compound **64,** the intermediate 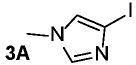 is replaced with 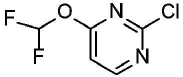 to synthesize compound **65.** LCMS [M+H] ⁺ =458.

### Embodiment 66: Synthesis and characterization of compound 66

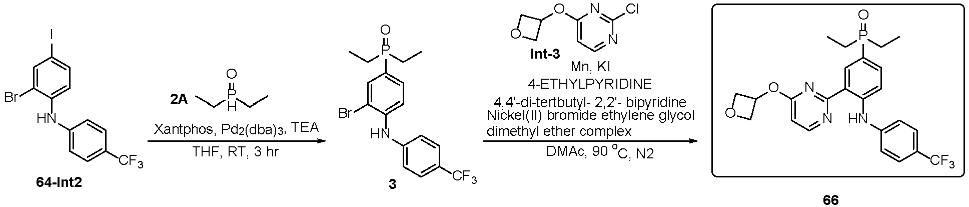

### Synthesis of intermediate 3

Under nitrogen protection, dissolving XantPhos (340.35 mg, 588.22 µmol, 0.1 eq), Pd ₂ (dba) ₃ (538.64 mg, 588.22 µmol, 0.1 eq), TEA (1.19 g, 11.76 mmol, 1.64 mL, 2 eq) in anhydrous THF (300 ml), and stirring the mixture at room temperature for 30 minutes. Then, adding a solution of intermediate 64-Int2 (2.6 g, 5.88 mmol, 1 eq) of **compound 64** and compound 2A (624.12 mg, 5.88 mmol, 1 eq) in THF (100 ml), and stirring the mixture at room temperature for 3 hours, after monitoring the completion of the reaction by LCMS, filtering, and concentrating under reduced pressure. The obtained crude product is purified by silica gel column (eluent: dichloromethane/methanol = 20/1) to obtain intermediate **3** (1.60 g, 3.43 mmol, 58.26% yield, 90.00% purity) as a yellow solid. LCMS [M+H] ⁺ = 419.8.

### Synthesis of compound 66

Dissolving intermediate Int- 3 (301.96 mg, 1.62 mmol, 1.7 eq), intermediate **3** (400 mg, 951.92 µmol, 1 eq), 4-ETHYLPYRIDINE (102.00 mg, 951.92 µmol, 108.28 µL, 1 eq), Mn (313.78 mg, 5.71 mmol, 6 eq), KI (189.62 mg, 1.14 mmol, 1.2 eq), 4,4'-di-tertbutyl-2,2'-bipyridine (766.49 mg, 2.86 mmol, 3 eq), Nickel(II) bromide ethylene glycol dimethyl ether complex (881.35 mg, 2.86 mmol, 3 eq) in DMAc (20 mL) and stirring at 100°C for 16 hours under N₂ protection. After LCMS showing that the reaction is completed, adding water (100 mL), extracting with EA (100 mL × 3), washing the organic phase with saturated brine (100 mL × 2), drying with anhydrous sodium sulfate, concentrating, and purifying by reverse phase column (H ₂ O (0.1% NH ₄ OH) / ACN) to obtain **compound 66** (50.00 mg, 99.71 µmol, 10.47% yield, 98.47% purity) as a yellow solid. ¹ H-NMR (400 MHz, DMSO-d ₆ ) δ 11.32 (s, 1H), 8.75 (d, *J* = 5.6 Hz, 1H), 8.65 (dd, *J* = 10.8, 2.4 Hz, 1H), 7.70-7.65 (m, 3H), 7.56 (dd, *J* = 8.4, 2.4 Hz, 1H), 7.47 (d, *J* = 8.4 Hz, 2H), 7.03 (d, *J* = 5.6 Hz, 1H), 5.79-5.74 (m, 1H), 4.95 (t, *J* = 7.2 Hz, 2H), 4.69 (dd, *J* = 7.6, 4.8 Hz, 2H), 2.04-1.82 (m, 4H), 1.033-0.953 (m, 6H). LCMS[M+H] = 492.1.

### Embodiment 67: Synthesis and characterization of compound 67

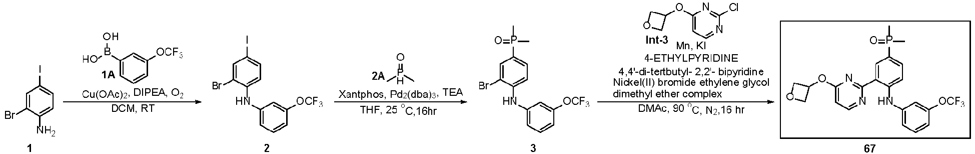

### Synthesis of Intermediate 2

Dissolving compound **1** (2 g, 6.71 mmol, 1 eq), compound **1A** (2.07 g, 10.07 mmol, 1.5 eq), anhydrous copper acetate (2.44 g, 13.43 mmol, 2 eq), and DIEA (4.34 g, 33.57 mmol, 5.85 mL, 5 eq) in DCM (50 mL) and stirring at room temperature for 48 hours under O₂ atmosphere. After LCMS showing that the raw material remain and the reaction is not continued for a longer time, filtering, concentrating, and purifying by silica gel column (eluent: PE/EA = 10/1) to obtain Intermediate **2** (1.3 g, 2.84 mmol, 42.32% yield, 83% purity) as a yellow solid. LCMS: The product has no molecular weight.

### Synthesis of intermediate 3

Under N₂ protection, dissolving XantPhos (12.63 mg, 21.83 µmol, 0.05 eq), Pd **₂** (dba) ₃ (12.00 mg, 13.10 µmol, 0.03 eq) and TEA (88.37 mg, 0.87 mmol, 2 eq) in anhydrous THF (15 ml), and stirring the mixture at room temperature for 30 min. Then , adding a THF (5 ml) solution of intermediate 2 (0.2 g, 436.67 µmol, 1 eq) and compound **2A** (36.52 mg, 480.34 µmol, 1.1 eq), and the mixture continues to react for 16 hours at 25°C, after monitoring the completion of the reaction was by LCMS, cooling to room temperature, adding water (70 mL) to dilute, extracting with ethyl acetate (50 mL×3 times), and washing the organic phase with saturated brine (70 mL×2 times), then drying with anhydrous sodium sulfate, and finally concentrating under reduced pressure. The obtained crude product is purified by silica gel column (eluent: dichloromethane/methanol = 20/1) to obtain intermediate **3** (130.00 mg, 318.51 µmol, 72.94% yield, 92.67% purity) as a yellow solid. LCMS[M+H] ⁺ = 407.7.

### Synthesis of compound 67

Dissolving intermediate Int- 3 (233.16 mg, 1.25 mmol, 1.7 eq), intermediate **3** (300 mg, 735.02 µmol, 1 eq), KI (146.42 mg, 882.03 µmol, 1.2 eq), Mn (161.53 mg, 2.94 mmol, 4 eq), 4-ETHYLPYRIDINE (78.76 mg, 735.02 µmol, 83.61 µL, 1 eq), 4,4'-di-tertbutyl-2,2'-bipyridine (59.18 mg, 220.51 µmol, 0.3 eq), Nickel(II) bromide ethylene glycol dimethyl ether complex (68.05 mg, 220.51 µmol, 0.3 eq) in DMAc (10 mL), and stirring the mixture at 90°C for 16 h. After LCMS showing that the reaction is completed, adding water (100 mL), extracting with EA (100 mL × 3), washing the organic phase with saturated brine (100 mL × 2), drying with anhydrous sodium sulfate, concentrating, and purifying by Prep-HPLC to obtain **compound 67** (63.00 mg, 131.42 µmol, 17.88% yield, 100% purity) as a white solid. ¹ H-NMR (400 MHz, DMSO- *d* ₆ ) δ 11.27 (s, 1H), 8.75-8.68 (m, 2H), 7.74-7.69 (m, 1H), 7.49 (t, *J* = 8.4 Hz, 1H), 7.42 (dd, *J =* 8.4, 2.0Hz, 1H), 7.35-7.33 (m, 1H), 7.27 (s, 1H), 7.06-7.01 (m, 2H), 5.81-5.75 (m, 1H), 4.97 (t, *J =* 7.2 Hz, 2H), 4.71-4.66 (m, 2H), 1.70 (s, 3H), 1.63 (s, 3H). LCMS [M+H] ⁺ = 480.1.

### Embodiment 68: Synthesis and characterization of compound 68

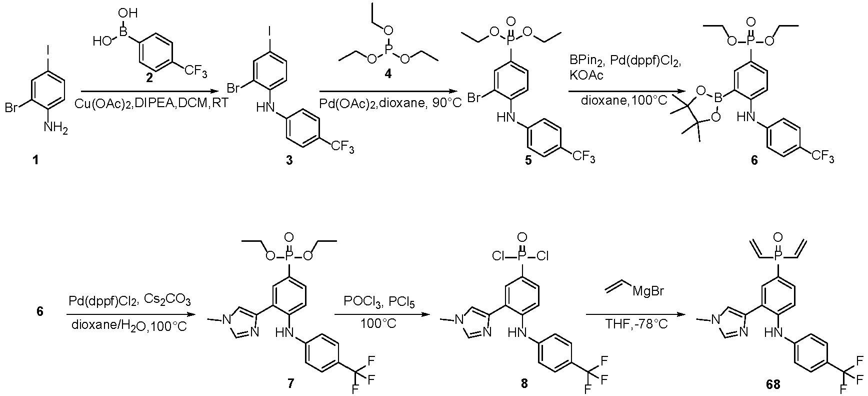

### Step 1: Synthesis of intermediate 3

Adding compound **1** (10 g, 33.57 mmol), compound **2** (6.38 g, 33.57 mmol), DIPEA (21.69 g, 167.83 mmol), anhydrous copper acetate (12.19 g, 67.13 mmol) to dichloromethane (50 mL), then adding 4A molecular sieves, replacing with O₂ three times and stirring at 25°C for 16 hours. Filtering, concentrating, and purifying by column (pure PE) to obtain the product brown liquid **intermediate 3** (4 g, 9.05 mmol, yield 26.95%).

### Step 2: Synthesis of intermediate 5

Adding **Intermediate 3** (44.7 g, 101.13 mmol), compound **4** (18.48 g, 111.24 mmol), palladium acetate (2.27 g, 10.11 mmol), anhydrous 1,4-dioxane (500 ml) into a single-mouth bottle, replacing with N₂ three times, and reacting at 90°C for 16 hours. Adding water to dilute, extracting with ethyl acetate, concentrating the organic phase, and purifying by column to obtain the product brown solid compound **intermediate 5** (22 g, 3.32 mmol, yield 48.10%).

### Step 3: Synthesis of intermediate 6

Adding **Intermediate 5** (20.9 g, 46.22 mmol), bis(pinacolato)diboron (23.47 g, 92.44 mmol), Pd(dppf)Cl₂ (3.39 g, 4.62 mmol), potassium acetate (13.61 g, 138.65 mmol) to 1,4-dioxane (100 mL), replacing with N₂ three times, and reacting at 100°C for 16 hours. Filtering and concentrating the organic phase to obtain the crude product brown liquid compound **intermediate 6** (22 g), which is used directly in the next step.

### Step 4: Synthesis of intermediate 7

Adding **Intermediate 6** (23 g), 4-iodo-1-methylimidazole (14.37 g, 69.1 mmol), Pd(dppf)Cl₂ (3.38 g, 4.61 mmol), cesium carbonate (45.03 g, 138.2 mmol) to 1,4-dioxane (100 mL) and water (20 mL), replacing with N₂ three times, and reacting at 100°C for 16 hours. Adding water to dilute, extracting with ethyl acetate, concentrating the organic phase, and purifying by column to obtain the product brown liquid compound **intermediate 7** (10 g, 22.06 mmol, yield 47.86%).

### Step 5: Synthesis of intermediate 8

Adding **Intermediate 7 (10 g, 22.06 mmol)** to phosphorus oxychloride (16.91 g, 110.28 mmol), replacing with N₂ three times, adding phosphorus pentachloride (11.48 g, 55.14 mmol) at 0°C, and reacting at 100°C for 16 hours. Concentrating the reaction solution to obtain a crude product brown solid compound **intermediate 8** (9 g), which is used directly in the next step.

### Step 6: Synthesis of compound 68

Adding **Intermediate 8** (9.5 g, 21.88 mmol) and anhydrous tetrahydrofuran (100 mL) into a three-necked flask, replacing with N₂ three times, and adding vinylmagnesium bromide (109.4 mL, 1M THF solution, 109.4 mmol) dropwise at -78°C, and reacting at -78°C for 2 hours.Adding ice water to quench the reaction, extracting with ethyl acetate, and concentrating the organic phase, and purifying by silica gel column to obtain a brown solid product, which is further purified by reverse phase column (0.5% ammonia/acetonitrile system) to obtain a light yellow powder solid **compound 68** (500 mg, 1.2 mmol, purity 95%, yield 5.48%). ¹ HNMR (400 MHz, DMSO-d ₆ ) δ 10.75 (s, 1H), 7.92 (dd, *J* = 12.4, 1.6 Hz, 1H), 7.84 (s, 1H), 7.75 (d, *J* = 0.8 Hz, 1H), 7.59 (d, *J* = 8.6 Hz, 2H), 7.52 (dd, *J =* 8.2, 2.8 Hz, 1H), 7.45-7.40 (m, 1H), 7.29 (d, *J =* 8.6 Hz, 2H), 6.77-6.62 (m, 2H), 6.25 (dd, *J* = 12.6, 2.2 Hz, 1H), 6.17 (s, 1H), 6.14-6.06 (m, 2H), 3.72 (s, 3H). LCMS [M-1] ⁻ = 416.1.

### Embodiment 69: Synthesis and characterization of compound 69

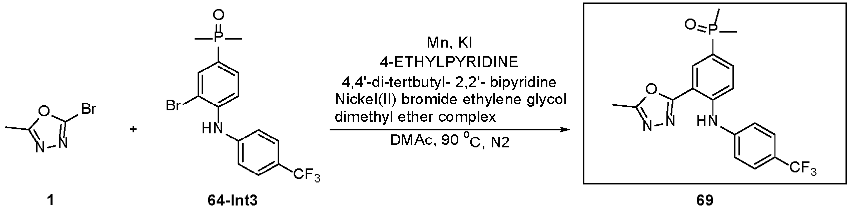

### Synthesis of compound 69

Dissolving compound 1 (282.60 mg, 1.73 mmol, 1.7 eq), intermediate 64 - Int3 of **compound 64** (400 mg, 1.02 mmol, 1 eq), Mn (336.23 mg, 6.12 mmol, 6 eq), KI (115.42 mg, 694.63 µmol, 1.2 eq), 4-ETHYLPYRIDINE (109.29 mg, 1.02 mmol, 116.02 µL, 1 eq), 4,4'-di-tertbutyl- 2,2'- bipyridine (821.32 mg, 3.06 mmol, 3 eq), Nickel(II) bromide ethylene glycol dimethyl ether complex (944.39 mg, 3.06 mmol, 3 eq) in DMAc (20 mL), and stirring the mixture at 90°C for 16 hours, after LCMS showing that the reaction is completed, adding water (100 mL), extracting with EA (100 mL × 3), washing the organic phase with saturated brine (100 mL × 2), drying with anhydrous sodium sulfate, concentrating, and purifying by Prep-HPLC to obtain **compound 69** (15.00 mg, 36.81 µmol, 3.61% yield, 97.70% purity) as a white solid. ¹ H-NMR (400 MHz, DMSO- *d* ₆ ) δ 9.59 (s, 1H), 8.25 (dd, *J =* 11.6, 2.0 Hz, 1H), 7.84-7.79 (m, 1H), 7.71 (d, *J =* 8.4 Hz, 2H), 7.61 (dd, *J =* 8.4, 2.0 Hz, 1H), 7.50 (d, *J =* 8.0 Hz, 2H), 2.63 (s, 3H), 1.69 (s, 3H), 1.66 (s, 3H). LCMS[M+H] ⁺ = 396.0.

### Embodiment 70: Synthesis and characterization of compound 70

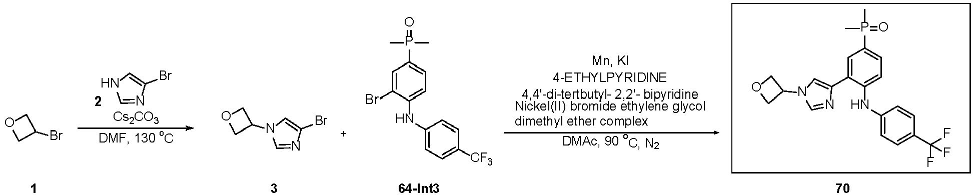

### Synthesis of Intermediate 3

Dissolving compound **1** (2.5 g, 18.25 mmol, 1.5 eq) and compound **2** (1.79 g, 12.17 mmol, 1 eq) in DMF (50 mL), adding cesium carbonate (7.93 g, 24.34 mmol, 2eq) , and stirring at 130°C for 16 hours under N₂ protection. After LCMS showing that the reaction is completed, adding water (100 mL), extracting with EA (100 mL × 3), washing the organic phase with saturated brine (100 mL × 2), drying with anhydrous sodium sulfate, concentrating, and purifying by silica gel column (eluent: EA) to obtain Intermediate **3** (2.00 g, 8.87 mmol, 72.86% yield, 90% purity) as a yellow solid. LCMS [M + H] ⁺ = 202.8.

### Synthesis of compound 70:

Dissolving Intermediate **3** (200 mg, 984.06 µmol, 1.7 eq), intermediate 64 of **compound 64** -Int3 (227 mg, 578.86 µmol, 1 eq), KI (115.42 mg, 694.63 µmol, 1.2 eq) , Mn (191.01 mg, 3.47 mmol, 6 eq), 4-ETHYLPYRIDINE (62.09 mg, 578.86 µmol, 65.91 µL, 1 eq), 4,4'-di-tertbutyl- 2,2'- bipyridine (466.56 mg, 1.74 mmol, 3 eq), Nickel(II) bromide ethylene glycol dimethyl ether complex (536.48 mg, 1.74 mmol, 3 eq) in DMAc (20 mL), and stirring the mixture at 90°C for 16 hours. After LCMS showed that the reaction is completed, adding water (100 mL), extracting with EA (100 mL × 3), washing the organic phase with saturated brine (100 mL × 2), drying with anhydrous sodium sulfate, concentrating, and purifying by Prep-HPLC to obtain **compound 70** (8.70 mg, 19.95 µmol, 3.45% yield, 99.84% purity) as a white solid. ¹ H-NMR (400 MHz, DMSO-d ₆ ) δ 10.71 (s, 1H), 8.26 (d, *J =* 1.2 Hz, 1H), 8.12-8.06 (m, 2H), 7.61-7.50 (m, 4H), 7.30 (d, *J =* 8.8 Hz, 2H), 5.59-5.52 (m, 1H), 4.98 (t, *J =* 7.4 Hz, 2H), 4.84 (t, *J* = 6.6 Hz, 2H), 1.69 (s, 3H), 1.66 (s, 3H). LCMS[M+H] ⁺ = 436.0.

### Embodiment 71: Synthesis and characterization of compound 71

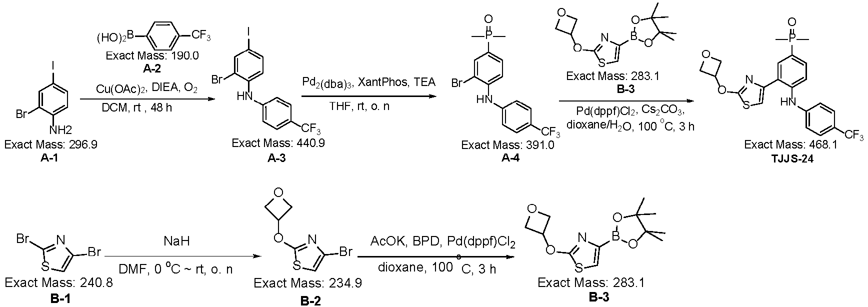

### Synthesis of intermediate A-3:

Adding raw materials A-1 (6.00 g, 20.14 mmol), A-2 (5.74 g, 30.21 mmol), copper acetate (7.32 g, 40.28 mmol), DIEA (7.81 g, 60.42 mmol) and appropriate amount of 4A molecular sieves to DCM (120 mL) in sequence, replacing with O₂, and stirring at room temperature for 48 hours under O₂ atmosphere. Filtering out insoluble matter to obtain the filtrate, and concentrating. Then purifying by flash column chromatography PE~EA (0 ~ 10 %) to obtain intermediate A-3 (3.10 g, yield 34.82 %) as a yellow solid. No liquid mass is produced. ¹ HNMR (400 MHz, Chloroform- *d* ) δ 7.87 (d, *J =* 2.0 Hz, 1H), 7.55 (d, *J* = 8.5 Hz, 2H), 7.50 (dd, *J* = 8.6, 2.0 Hz, 1H), 7.14 (d, *J =* 8.4 Hz, 2H), 7.10 (d, *J* = 8.6 Hz, 1H), 6.18 (s, 1H).

### Synthesis of intermediate A-4:

Adding raw material A-3 (3.00 g, 6.79 mmol), Pd ₂ (dba) ₃ (622 mg, 0.68 mmol), XantPhos (785 mg, 1.36 mmol), dimethylphosphine oxide (689 mg, 8.82 mmol) and TEA (1.03 g, 10.18 mmol) to 1, 4-dioxane (60 mL) in sequence, replacing with N₂, and stirring at room temperature overnight. Concentrating, adding 100 mL of water, extracting with EA, drying with anhydrous sodium sulfate, concentrating the organic phase, and purifying by flash column chromatography DCM- MeOH (0 ~ 5 %) to obtain intermediate A-4 (2.30 g, yield 86.41 %) as a yellow solid. LCMS: m/z = 392.0 (M+H ⁺ , ESI).

### Synthesis of intermediate B-2:

At 0 ° C, adding NaH (247 mg, 6.17 mmol, 60 % purity) to a DMF (5 mL) solution of oxetane-3-ol (396 mg, 5.35 mmol). After 1 hour, adding the solution dropwise to a DMF (5 mL) solution of B-1 (1.00 g, 4.12 mmol) at 0 ° C. Slowly heating to room temperature and stirring overnight. Quenching the reaction with ice water, extracting with EA, drying with anhydrous sodium sulfate, and concentrating the organic phase, then purifying by flash column chromatography PE-EA (0 ~ 20 %) to obtain intermediate **B-2** (730 mg, yield 75.11 %) as a colorless viscous oil. LCMS: m/z = 237.1 (M+H ⁺ , ESI).

### Synthesis of intermediate B-3:

Adding Pd(dppf)Cl₂ (93 mg, 0.13 mmol), potassium acetate (374 mg, 3.81 mmol) and B ₂ (Pin) ₂ (355 mg, 1.40 mmol) to a dioxane (5 mL) solution of compound B-2 (300 mg, 1.27 mmol) in sequence. After the addition, replacing with N₂, heating to 100 ° C and reacting for 3 hours. After concentration, obtaining Boric acid (256 mg, yield 100%, theoretical value) as a black solid, which directly used for the next step without purification. LCMS: m/z = 202.0 (M+H ⁺ , ESI). LCMS observed the molecular weight of boronic acid.

### Synthesis of compound 71 (TJJS-24):

Adding Pd(dppf)Cl₂(62 mg, 0.084 mmol) and cesium carbonate (548 mg, 1.68 mmol) to a dioxane (1 mL) and water (0.25 mL) solution of compound A-4 (330 mg, 0.84 mmol) and B-3 (220 mg, 1.09 mmol). After the addition, replacing with N₂, heating to 100° C and reacting for 3 hours. Concentrating, adding 50 mL of water, extract with EA, drying with anhydrous sodium sulfate, concentrating the organic phase, and then purifying by flash column chromatography C18 at water: acetonitrile (0 ~ 50 %) (0.1 % formic acid) to obtain **compound 71 (TJJS-24)** (80 mg, yield 20.30 %) as a white solid. LCMS: m/z = 469 (M+H ⁺ , ESI). ¹ H NMR (400 MHz, DMSO- *d* ₆ ) δ 8.81 (s, 1H), 7.97 (dd, *J* = 11.8, 1.9 Hz, 1H), 7.66 (ddd, *J* = 10.4, 8.3, 1.9 Hz, 1H), 7.57 (d, *J* = 8.5 Hz, 2H), 7.49 (dd, *J* = 8.4, 2.4 Hz, 1H), 7.45 (s, 1H), 7.20 (d, *J* = 8.5 Hz, 2H), 5.71 (ddd, *J =* 6.1, 4.7, 1.3 Hz, 1H), 4.85 (ddd, *J* = 7.3, 6.1, 1.1 Hz, 2H), 4.65 (ddd, *J* = 7.8, 4.8, 1.0 Hz, 2H), 1.68 (s, 3H), 1.65 (s, 3H).

### Embodiment 72: Synthesis and characterization of compound 72

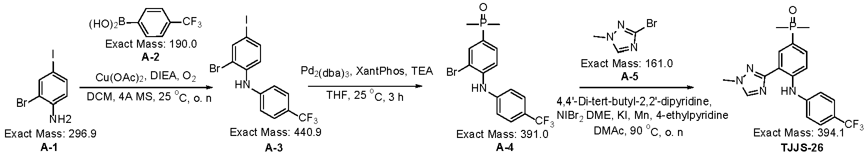

### Synthesis of intermediate A-3:

Adding raw materials A-1 (6.00 g, 20.14 mmol), A-2 (5.74 g, 30.21 mmol), copper acetate (7.32 g, 40.28 mmol), DIEA (7.81 g, 60.42 mmol) and appropriate amount of 4A molecular sieves to DCM (120 mL) in sequence, replacing with O₂, and stirring at room temperature for 48 hours under O₂ atmosphere. Filtering out insoluble matter by suction to obtain the filtrate, and concentrating. Then purifying by flash column chromatography PE~EA (0 ~ 10%) to obtain intermediate A-3 (3.10 g, yield 34.82%) as a yellow solid. No liquid mass is produced. ¹ HNMR (400 MHz, Chloroform- *d* ) δ 7.87 (d, *J =* 2.0 Hz, 1H), 7.55 (d, *J =* 8.5 Hz, 2H), 7.50 (dd, *J =* 8.6, 2.0 Hz, 1H), 7.14 (d, *J =* 8.4 Hz, 2H), 7.10 (d, *J* = 8.6 Hz, 1H), 6.18 (s, 1H).

### Synthesis of intermediate A-4:

Adding raw material A-3 (3.00 g, 6.79 mmol), Pd₂ (dba)₃ (622 mg, 0.68 mmol), XantPhos (785 mg, 1.36 mmol), dimethylphosphine oxide (689 mg, 8.82 mmol) and TEA (1.03 g, 10.18 mmol) to 1, 4-dioxane (60 mL) in sequence, replacing with N₂, and stirring at room temperature overnight. Concentrating, adding 100 mL of water, extracting with EA, drying with anhydrous sodium sulfate, concentrating the organic phase, and purifying by flash column chromatography DCM- MeOH (0 ~ 5 %) to obtain intermediate A-4 (2.30 g, yield 86.41 %) as a yellow solid. LCMS: m/z = 392.0 (M+H ⁺ , ESI).

### Synthesis of compound 72 (TJJS-26):

Adding DMAc (2 mL) to a mixture of compound A-4 (150 mg, 0.38 mmol), A-5 (93 mg, 0.57 mmol), 4,4'-di-tert-butyl-2,2'-bipyridine (31 mg, 0.11 mmol), 4-ethylpyridine (41 mg, 0.38 mmol), potassium iodide (63 mg, 0.38 mmol), manganese powder (63 mg, 1.15 mmol) and ethylene glycol dimethyl ether nickel bromide (45 mg, 0.11 mmol). After the addition, replacing with N₂, heating to 90°C and reacting for overnight. Concentrating, adding 30 mL of water, extracting with EA, drying with anhydrous sodium sulfate, concentrating the organic phase, and purifying by a 1 mm thin chromatography silica gel plate DCM-MeOH (10%) to obtain **compound 72 (TJJS-26)** (10 mg, yield 6.63%) as a white solid. LCMS: m/z = 393 (MH ⁺ , ESI). ¹ H NMR (400 MHz, DMSO- *d* ₆ ) δ 10.14 (s, 1H), 8.73 (d, *J* = 0.7 Hz, 1H), 8.48 (dd, *J* = 11.9, 1.9 Hz, 1H), 7.66 (dd, *J =* 9.4, 3.2 Hz, 3H), 7.57 (dd, *J =* 8.5, 2.5 Hz, 1H), 7.43 (d, *J =* 8.4 Hz, 2H), 4.00 (s, 3H), 1.67 (s, 4H), 1.63 (s, 4H).

### Embodiment 73: Synthesis and characterization of compound 73

According to the synthetic route of compound **64** , the starting material compound 1 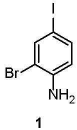 is replaced with 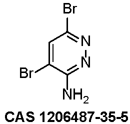 to synthesize compound **73.** LCMS [M+H] ⁺ =396.

### Embodiment 74: Synthesis and characterization of compound 74

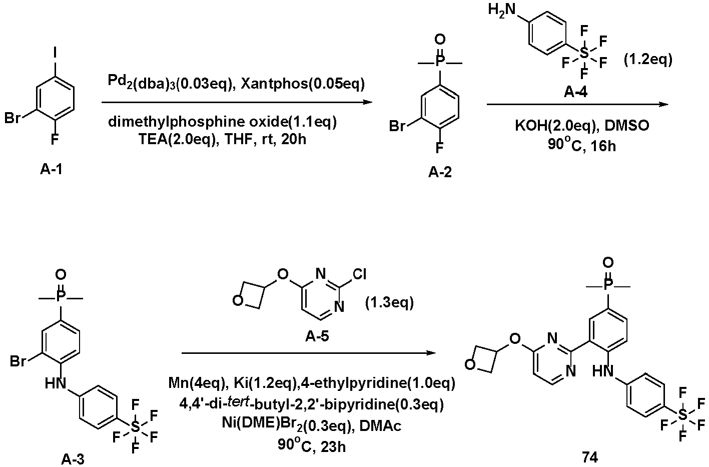

### Step 1: Synthesis of intermediate A-2

Dissolving compound **A-1** (1.204 g, 4 mmol), Pd ₂ (dba) ₃ (110 mg, 0.12 mmol), Xantphos (120 mg, 0.2 mmol) in 10 mL tetrahydrofuran, and adding dimethylphosphine oxide (343 mg, 4.4 mmol) and triethylamine (808 mg, 8 mmol) under N₂ protection, and stirring at room temperature for 16 hours. Filtering through diatomaceous earth to remove the solid, concentrating, and purifying by column (dichloromethane: methanol = 20: 1) to obtain the product brown liquid intermediate **A-2** (650 mg, yield 65.3%).

### Step 2: Synthesis of intermediate A-3

Dissolving intermediate **A-2** (310 mg, 1.0 mmol) and compound **A-4** (260 mg, 1.2 mmol) in 3 mL of dimethyl sulfoxide, adding potassium hydroxide (112 mg, 2 mmol), heating to 90°C and reacting for 16 hours. Adding 10 ml of water to quench the reaction, extracting with ethyl acetate (5 mL*2), combining the organic phases, washing with saturated brine (5 mL), drying with anhydrous sodium sulfate, concentrating, adding into a single-mouth bottle, replacing with N₂ three times, and reacting at 90°C for 16 hours. Diluting with water, extracting with ethyl acetate, concentrating the organic phase, and purifying by column (dichloromethane: methanol = 15:1) to obtain product **A-3** (200 mg, yield 44.4%).

### Step 3: Synthesis of compound 74

Dissolving the intermediate **A-3** (630 mg, 1.6 mmol), compound **A-5** (417 mg, 2.24 mmol), Mn (308 mg, 5.6 mmol), 4-ethylpyridine (150 mg, 1.4 mmol), potassium iodide (279 mg, 1.68 mmol), 4,4'-di-tert-butyl-2,2'-bipyridine (113 mg, 0.42 mmol), nickel (II) bromide ethylene glycol dimethyl ether complex (129 mg, 0.42 mmol) in N, N-dimethylacetamide (15 mL), heating to 90°C under N₂ protection and reacting for 16 hours. Filtering through diatomaceous earth to remove the solid, spin-drying the solvent, adding EA (10 mL) , adding silica gel to mix the sample, and purifying by column (dichloromethane: methanol = 12: 1) to obtain the product **74** (30 mg, yield 4.1%) . LCMS [M+1] ⁺ =522. ¹ HNMR (400 MHz, d6-DMSO) δ 11.31 (s, 1H), 8.74 (d, J = 5.9 Hz, 1H), 8.67 (dd, J = 12.3, 1.9 Hz, 1H), 7.86-7.80 (m, 2H), 7.76 (ddd, J = 10.3, 8.6, 1.9 Hz, 1H), 7.60 (dd, J = 8.5, 2.3 Hz, 1H), 7.42 (d, J = 8.9 Hz, 2H), 7.03 (d, J = 5.9 Hz, 1H), 5.75 (m, 1H), 4.95 (m, 2H), 4.68 (dd, J = 7.9, 5.3 Hz, 2H), 1.68 (d, J = 13.3 Hz, 6H).

### Embodiment 75: Synthesis and characterization of compound 75

Using **A-3** in the synthesis step of Embodiment 74 as a raw material, according to the third step reaction conditions of the synthesis step of Embodiment 64, undergoing a reductive coupling reaction with the compound 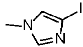 to obtain compound **75.** LCMS [M+1] ⁺ =452 .

### Embodiment 76: Synthesis and characterization of compound 76

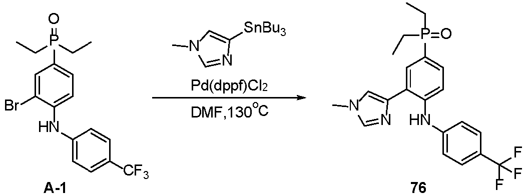

Dissolving the intermediate 3 of Embodiment 66, i.e. A-1 (250 mg, 594.95 µmol, 1 eq) in the above figure in DMF (5 ml), and adding 1-methyl-4-(tributylstannyl)imidazole (264.98 mg, 713.94 µmol, 1.2 eq) and Pd(dppf)Cl ₂ (43.17 mg, 59.50 µmol, 0.1 eq), stirring the system at 130°C for 16 hours under N₂ protection, when LC-MS showing that the product is generated, the reaction is completed, adding water (30 mL), extracting with EA (20 mL × 3), washing the organic phase with saturated brine (30 mL × 2), drying with anhydrous sodium sulfate, concentrating, and purifying by Prep-HPLC (acetonitrile/ammonia water) to obtain **76** (40.00 mg, 94.92 µmol, 15.95% yield, 100% purity) as a yellow solid. ¹ H-NMR (400 MHz, DMSO- *d* 6) δ 10.81 (s, 1H), 8.00-7.94 (m, 1H), 7.86-7.76 (m, 2H), 7.60 (d, *J =* 8.4 Hz, 2H), 7.55-7.44 (m, 2H), 7.31 (d, *J* = 8.4 Hz, 2H), 3.74 (s, 3H), 2.08-1.82 (m, 4H), 1.10-0.80 (m, 6H). LC-MS[M+H] ⁺ = 422.1.

### Test example 1. Test of the effect of compounds on the melting point curve of recombinant TEAD1/2/3/4 proteins

The ability of the compounds to increase the melting point of purified recombinant TEAD1/2/3/4 proteins is tested using a thermal shift assay. The strength of the compound's binding to the protein and the selectivity of the compound's binding to the four TEAD subtypes are evaluated by calculating the melting point shift Δ*T*m -°C. The larger the Δ*Tm* value, the stronger the compound's ability to stabilize the protein, reflecting its stronger binding ability. Based on the Δ *T* m value, the ability of the compound to bind to the TEAD protein is divided into four levels: ++++, +++, ++, +.

**Table 1**

| **Compound No.** | **Δ *T* m (TEAD1 *^{a}*)** | **Δ *T* m (TEAD2 *^{b}* )** | **Δ *T* m (TEAD3 *^{c}* )** | **Δ *T* m (TEAD4 *^{d}* )** |
|---|---|---|---|---|
| 1 | +++ | +++ | ++ | +++ |
| 2 | +++ | + | ++ | ++ |
| 3 | +++ | + | + | ++ |
| 4 | +++ | ++ | ++ | + |
| 5 | ++++ | +++ | ++ | ++++ |
| 6 | ++++ | ++++ | +++ | ++++ |
| 7 | ++++ | ++ | ++ | ++++ |
| 8 | ++++ | ++ | ++ | ++++ |
| 9 | +++ | ++ | + | +++ |
| 10 | ++++ | ++ | ++ | ++ |
| 11 | +++ | ++ | + | ++ |
| 12 | ++ | + | + | + |
| 13 | +++ | + | + | + |
| 14 | ++ | + | + | + |
| 15 | +++ | ++ | ++ | +++ |
| 16 | +++ | ++ | ++ | +++ |
| 17 | ++++ | ++ | + | ++ |
| 18 | ++++ | + | + | +++ |
| 19 | ++ | ++ | ++ | + |
| 20 | +++ | ++ | ++ | ++ |
| 21 | ++++ | ++++ | +++ | ++++ |
| 22 | +++ | ++++ | +++ | ++ |
| 23 | ++ | + | + | + |
| 24 | ++++ | ++ | + | ++++ |
| 25 | ++ | ++ | ++ | ++ |
| 26 | ++++ | ++ | + | ++++ |
| 27 | ++++ | ++ | + | ++++ |
| 28 | ++++ | ++ | + | ++++ |
| 29 | ++++ | ++ | + | ++++ |
| 30 | +++ | + | + | ++ |
| 31 | ++++ | + | + | +++ |
| 32 | ++ | + | ++ | + |
| 33 | ++ | + | + | ++ |
| 34 | ++ | + | + | ++ |
| 35 | ++ | + | ++ | + |
| 36 | ++ | + | + | +++ |
| 37 | ++ | ++ | ++ | ++ |
| 38 | +++ | + | + | +++ |
| 39 | ++++ | + | + | ++ |
| 40 | ++ | ++ | ++ | ++ |
| 41 | +++ | +++ | ++ | +++ |
| 42 | ++ | ++ | + | + |
| 43 | ++ | + | + | + |
| 44 | ++ | ++ | + | ++ |
| 45 | +++ | +++ | +++ | ++++ |
| 46 | +++ | ++ | ++ | ++ |
| 47 | +++ | + | ++ | ++ |
| 48 | ++ | + | + | + |
| 49 | +++ | + | ++ | ++ |
| 50 | ++ | ++ | ++ | ++ |
| 51 | ++ | ++ | ++ | ++ |
| 52 | ++ | ++ | + | ++ |
| 53 | ++++ | + | + | +++ |
| 54 | ++ | + | + | ++ |
| 55 | ++ | + | + | ++ |
| 56 | ++ | + | + | + |
| 57 | ++ | + | + | + |
| 58 | ++ | + | + | + |
| 59 | ++ | + | + | + |
| 60 | +++ | + | + | ++ |
| 61 | ++++ | +++ | ++ | ++++ |
| 62 | ++ | + | + | + |
| 63 | ++++ | +++ | + | +++ |
| 64 | ++++ | +++ | ++ | ++++ |
| 65 | +++ | ++ | + | ++ |
| 66 | ++++ | + | + | ++++ |
| 67 | +++ | ++ | + | +++ |
| 68 | ++++ | ++++ | ++ | ++++ |
| 69 | ++ | + | ++ | ++ |
| 70 | ++ | ++ | + | ++ |
| 71 | ++ | + | + | ++ |
| 72 | ++++ | +++ | ++ | +++ |
| 73 | ++++ | ++++ | +++ | ++++ |
| 74 | ++++ | + | + | ++++ |
| 75 | ++++ | ++++ | +++ | ++++ |
| 76 | ++++ | ++++ | +++ | ++++ |

| | | | | |
|---|---|---|---|---|
| *^{a}* **TEAD1 protein sequence: 194-411 (Uniprot ID: P28347)** *^{b}* **TEAD2 protein sequence: 218-447 (Uniprot ID: Q15562)** *^{c}* **TEAD3 protein sequence: 218-435 (Uniprot ID: Q99594)** *^{d}* **TEAD4 protein sequence: 217-434 (Uniprot ID: Q15561)** | | | | |

**Test method of protein melting point curve:** 10 µL of the protein melting point curve experimental system (10 µM TEAD protein, 50 µM test compound, SYPRO^{®} orange (S5692, sigma)) is prepared in a 384-well fluorescence quantitative PCR plate according to the following system, and reacted at 4°C in the dark for 20 minutes. On-machine reaction: ABI QuantStudio Dx fluorescence quantitative PCR instrument; program: from 25°C to 80°C, increasing the temperature at a rate of 0.05 °C/s, and reading the fluorescence channel signal of the corresponding wavelength of SYPRO^{®} orange every second.

From the above results, it can be seen that the compounds in the above Table 1 have different binding abilities to TEAD family proteins, some compounds such as 6, 7, 8, 21, 22, 45, 61, 64, 68, 72, 73, 75, and 76 have good binding abilities to each subtype of TEAD family proteins and thus have strong physiological activity.

### Test Example 2. Test of using high-resolution mass spectrometry to detect irreversible binding of covalent compounds to recombinant TEAD protein

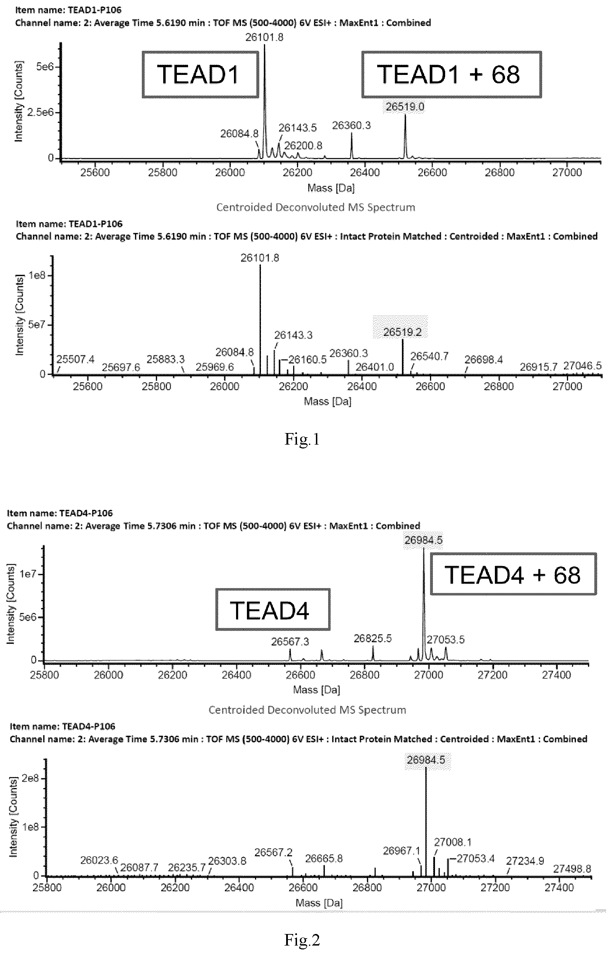

Covalent inhibitors can bind to target proteins irreversibly, thus exerting a lasting pharmacological effect. Compounds 61 and 68 have chemical groups that can bind irreversibly to conserved cysteine residues in the palmitoyl pocket of TEAD proteins, and are covalent inhibitors; however, most reported TEAD inhibitors such as VT103 are non-covalent compounds, so compounds 61 and 68 can bind to TEAD proteins irreversibly, thereby bringing about a more lasting pharmacological effect. In order to demonstrate the characteristics of covalent binding, 10 equivalents of compound 68 are incubated with 1 equivalent of TEAD1 or TEAD4 protein at 37°C for 1 hour, and the shift of protein molecular weight is detected by liquid chromatography-high-resolution mass spectrometry, as shown in Figs.1 and 2. The results show that the molecular weight of TEAD protein after incubation increases the molecular weight value of compound 68, proving that it has the ability to irreversibly bind and label TEAD protein.

The above results prove that compound 68 can covalently bind to TEAD protein, which has advantages over non-covalent inhibitors such as VT103.

### Test Example 3: Test of in vitro liver microsome stabilities of compounds

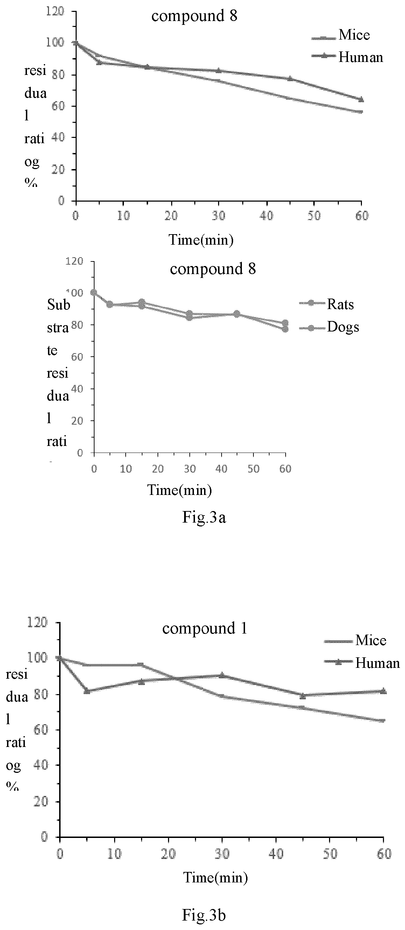

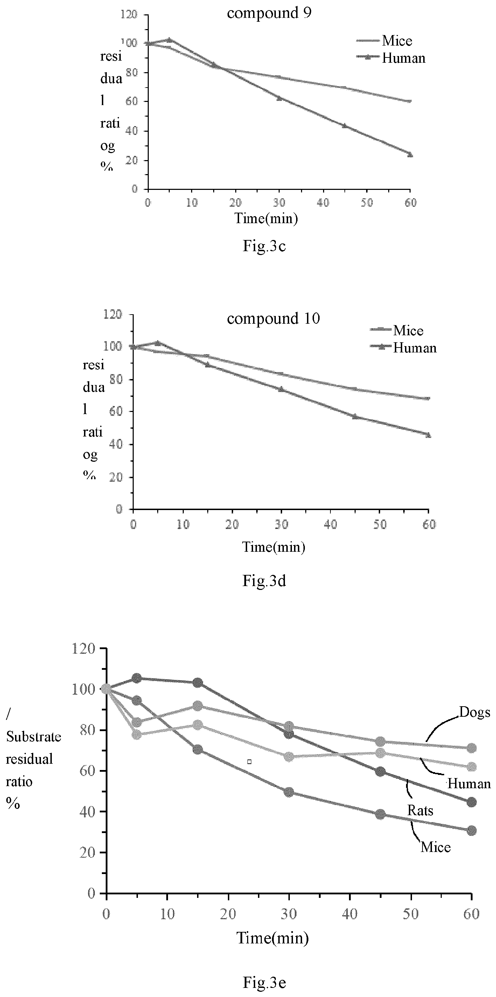

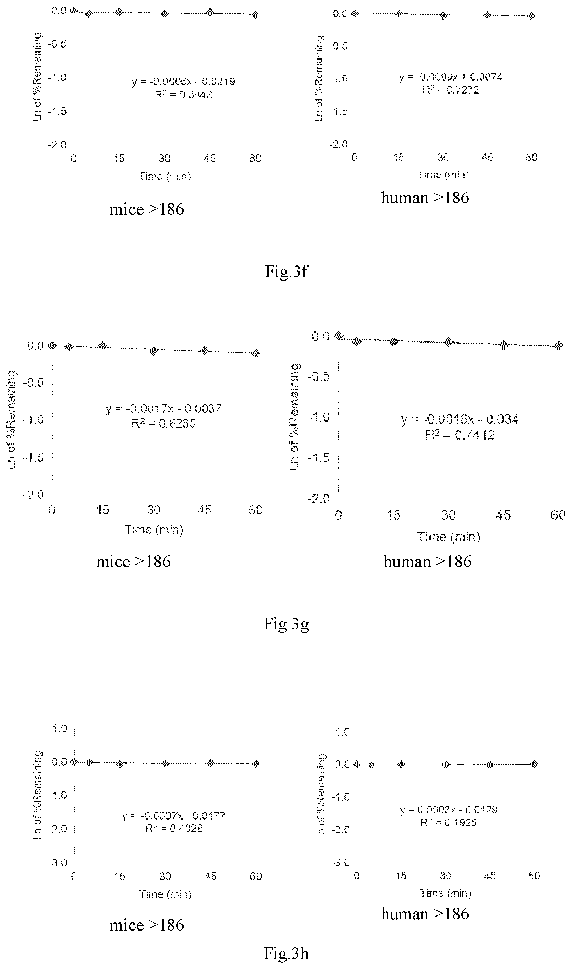

Testosterone is selected as the positive reference compound. The specific method is as follows:
Preparing 0.1M _{K 3} PO ₄ (pH 7.4) buffer and 3 ×NADPH stock solution (6mM, 5mg/mL), and preheating in a 37°C water bath; preparing spiking solution of tested compound and control compound: adding 5µL compound stock solution (10mM) to 95µL acetonitrile; preparing 1.5µM spiking solution in microsomes (0.75mg/mL): add 1.5µL spiking solution and 18.75µL liver microsome solution (20mg/mL) to 479.75µL K ₃ PO ₄ buffer; taking 30µL spiking solution in microsomes and adding it to the multiwell plate, and incubating at 37°C for 5min; adding 15µL of NADPH stock solution to each well, starting to react, and timing; adding 150 µL of acetonitrile solution containing IS at 0 min, 5 min, 15 min, 30 min, 45 min and 60 min respectively to terminate the reaction; after 10 min of oscillation, centrifuging the reaction at 6000 rpm for 15 min; taking 80 µL of supernatant from each well for LC/MS detection and calculating T_{1/2} . Some test results are shown in Figs. 3a-3h.

As shown in Figs. 3a-3h, compounds 1, 8, 28, 64 and 68 show good metabolic stabilities in the in vitro mouse and human liver microsome environment, and compound 8 shows good metabolic stabilities in the rat and dog liver microsome environment.

Liver microsomes of mice, rats, dogs, and human used in the experiment are purchased from Xenotech.

### Test Example 4: Test of mouse plasma pharmacokinetic of the compound

In this example, different compounds are used to perform a mouse pharmacokinetic experiment, and the specific steps are as follows:
Animal information: CD-1 mice, male, about 20 g, purchase from Beijing Weitonglihua Experimental Animal Technology Co., Ltd.
Animal dietary status: Animals are not fasted before administration and allowed to drink water freely.
Compound preparation method: for intragastric administration (IG)(G1 group), preparing with 0.5% CMC-Na, and for intravenous injection(IV) (G2 group), preparing 5% DMSO + 30% PEG400 + 65% water .
IG (Group G1): intragastricly administering the compound to the experimental animals at a dose of 10 mg/kg (10 mL/kg) (n = 9); IV (Group G2): intravenously administering the compound to the experimental animals at a dose of 1 mg/kg (10 mL/kg) (n = 9).
Sampling time points of G1 group: 8 points in total, namely 10 min, 30 min, 1 h, 2 h, 4 h, 6 h, 8 h and 24 h after administration, among them, animals G1M01-G1M03 are sampled at 3 time points, namely 10 min, 1 h and 6 h; animals G1M04-G1M06 are sampled at 3 time points, namely 30 min, 2 h and 8 h; animals G1M07-G1M09 are sampled at 2 time points, namely 4 h and 24 h.
Sampling time points of G2 group: 9 points in total, namely 5 min, 10 min, 30 min, 1 h, 2 h, 4 h, 6 h, 8 h, 24 h after administration. It is recommended that animals G2M01-G2M03 are sampled at 3 time points, namely 5 min, 1 h, and 6 h; animals G2M04-G2M06 are sampled at 3 time points, namely 10 min, 2 h, and 8 h; animals G2M07-G2M09 are sampled at 3 time points, namely 30 min, 4 h, and 24 h.
Sample collection: On the day of the experiment, collecting approximately 0.1 mL of whole blood samples at each preset time point and placing in EDTA-K2 anticoagulant tubes.
Sample processing: Within 30 minutes after whole blood sample collection, centrifuging at 4°C, 12,000 rpm for 5 minutes to separate plasma, collecting the upper plasma sample into a sample tube, and freezing the plasma sample in a - 10~-30°C refrigerator within 30 minutes and transferring to a -60~-90°C refrigerator within 24 hours.
Sample testing and data processing: LC/MS/MS testing, WinNonlin for fitting and pharmacokinetic parameter calculation. The results are shown in Fig.s 4a-4c and Table 2.

**Table 2 Pharmacokinetic parameters of CD-1 mice after single IG (10 mg/kg) and IV (1 mg/kg)**

| paramet er | unit | Compound 17 | | Compound 64 | |
|---|---|---|---|---|---|
| | | IG | IV | IG | IV |
| Cₘₐₓ | ng/mL | 1477 | 598 | 4063 | |
| Tmax | h | 0.5 | N/A | 2.67 | N/A |
| AUC ₀₋ₜ | h*ng/m L | 4662 | 899 | 57458 | 6596 |
| AUC _{0-∞} | h*ng/m L | 5688 | 924 | 69572 | 7208 |
| Vss | L/kg | N/A | 1.947 | N/A | 1.15 |
| CL | L/h/kg | N/A | 1.082 | N/A | 0.14 |
| MRT₀₋ₜ | h | 2.84 | 1.55 | 8.16 | 5.85 |
| MRT_{0-∞} | h | 4.66 | 1.80 | 13.3 | 8.26 |
| t_{1/2} | h | 3.46 | 1.94 | 9.43 | 6.98 |
| F% | | 51.9 | | 87.1 | |

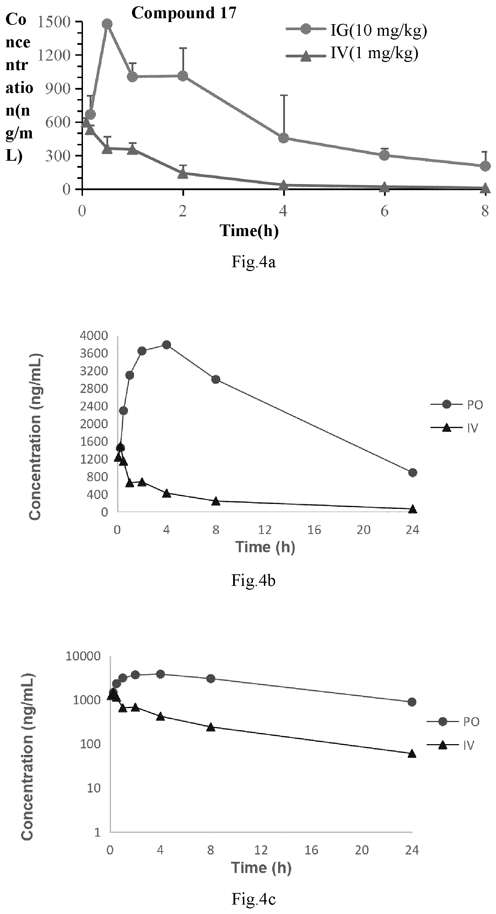

As shown in Figs. 4a-4c and Table 2, compounds 17 and 64 exhibit good pharmacokinetic properties in mice.

The existing TEAD palmitoyl pocket inhibitor VT103 is tested in the same manner, and the results are as follows: PK parameters of VT103 in mice: IV, T_{1/2} = 13.2h, Vss = 4.5L/kg, Cl = 4.7mL/min/kg; oral bioavailability = 75%, per oral (PO) 7mg/kg of Cₘₐₓ = 896ng/mL, plasma drug concentration after 24 hours C₂₄ₕ = 340ng/mL.

The inventors believe that VT103 (Tang et al , 2021, Mol Cancer Ther. 20(6):986-998.) is too lipophilic. The pharmacokinetic experiment in mice shows that the apparent volume of distribution is too large (V_{SS} = 4.5L/kg). After 24 hours of IG 7mg/kg, the blood concentration is still as high as 340ng/ml (828nM). This suggests that VT103 tends to enrich into tissues after being absorbed into the blood, and is eliminated slowly, resulting in accumulation and exposing high toxicity. The compound of the present invention has a reasonable plasma clearance rate and tissue distribution, which weakens the tendency of tissue accumulation and greatly improves oral bioavailability.

### Test Example 5: Permeability test of compounds on Caco2 cells

| | |
|---|---|
| Test methods | Caco-2 cell model |
| Drug concentration | 10 µM |
| Test conditions | Constant temperature shaking at 37°C for 60 min |
| Number of samples | n=2 |
| Analytical methods | LC-MS/MS, analysis by peak area ratio |

After resuscitation, the Caco-2 cells are transferred to a conventional culture dish and cultured in a high-glucose DMEM medium (containing 10% FBS) at 37°C and 5% CO2 in an incubator. When the cells cover 80% to 95% of the bottom of the dish, they are digested with trypsin containing 0.25% EDTA to disperse them into a single cell suspension, and the cell concentration is determined using a cell counter, after that, the concentration of the cell suspension is adjusted to 2 to 2.5 × 10⁵ /mL with fresh culture medium and inoculated into a Transwell polycarbonate membrane 12-well plate, the fresh culture medium is replaced every two days in the first two weeks and once a day in the last week, the culture is continued for 19 to 21 days to obtain a fully differentiated single cell layer.

| | | | | | | |
|---|---|---|---|---|---|---|
| **Dosing solution preparation** | Name of dosing solution | Preparation process | | | | Penetration Type |
| | | concentration | Solution used | | Blank HBSS solution addition amount | |
| | | | concentration | Addition amount | | |
| | Fluorescein | 100 µg/mL | 10 mg/mL | 20 µL | 2 mL | Zero penetration |
| | Ranitidine | 20 µM | 20 mM | 2 µL | 2 mL | Medium penetration |
| | Metoprolol | 20 µM | 20 mM | 2 µL | 2 mL | High penetration |
| | Compound 8 | 10 µM | 10 mM | 7 µL | 7 mL | |
| | Compound 17 | 10 µM | 10 mM | 7 µL | 7 mL | |

Sucking away the culture medium on the AP and BL ends of the Transwell chamber, adding blank HBSS solution preheated at 37°C, and equilibrating at 37°C for 20 min; sucking away the HBSS solution, adding preheated drug solution to the AP or BL side, and adding blank HBSS solution to the opposite side (BL or AP side); culturing at 37°C with constant temperature shaking for 120 min; collecting all samples on the AP and BL sides and storing at -60~-90°C for testing.

**Table 3 Results of permeability test of compounds on Caco2 cell model**

| Group | Penetrati on Type | Directions for administra tion | Receiving chamber concentration | *P ₐₚₚ* (10 ⁻⁶ cm/s) | | | | Ejecti on rate |
|---|---|---|---|---|---|---|---|---|
| | | | Area ratio/OD | *P ₐₚₚ* | averag e value | SD | CV(%) | |
| Compound 8 | High penetrati on | AP→BL | 0.249 | 6.90 | **7.80** | 1.27 | 16.3 | **6.48** |
| | | AP→BL | 0.314 | 8.70 | | | | |
| | | BL→AP | 5.70 | 52.7 | 50.6 | 2.94 | 5.81 | |
| | | BL→AP | 5.25 | 48.5 | | | | |
| Compound 17 | | AP→BL | 0.114 | 8.81 | **8.96** | 0.219 | 2.44 | 2.02 |
| | | AP→BL | 0.118 | 9.12 | | | | |
| | High penetrati on | BL→AP | 0.699 | 18.0 | **18.1** | 0.16 | 0.9 | |
| | | BL→AP | 0.708 | 18.2 | | | | |
| Control group-fluorescein | Zero penetrati on | AP→BL | 4.40E+07 | 0.364 | **0.349** | 0.0206 | 5.91 | N/A |
| | | AP→BL | 4.05E+07 | 0.334 | | | | |
| Control group - ranitidine | Medium penetrati on | AP→BL | 3.32 | 1.99 | **1.84** | 0.212 | 11.5 | |
| | | AP→BL | 2.82 | 1.69 | | | | |
| Control group - Metoprolol | High penetrati on | AP→BL | 13.6 | 17.8 | **18.1** | 0.371 | 2.05 | |
| | | AP→BL | 14.0 | 18.4 | | | | |

From the above test results, it can be seen that compounds 8 and 17 have high intestinal hypertonicity , suggesting that they can be fully absorbed through the intestine.

### Test Example 6: Test of the Binding Ability of Compounds to the Central Pocket of Recombinant TEAD Protein (Competitive Thiol Binding Assay, CPM Assay)

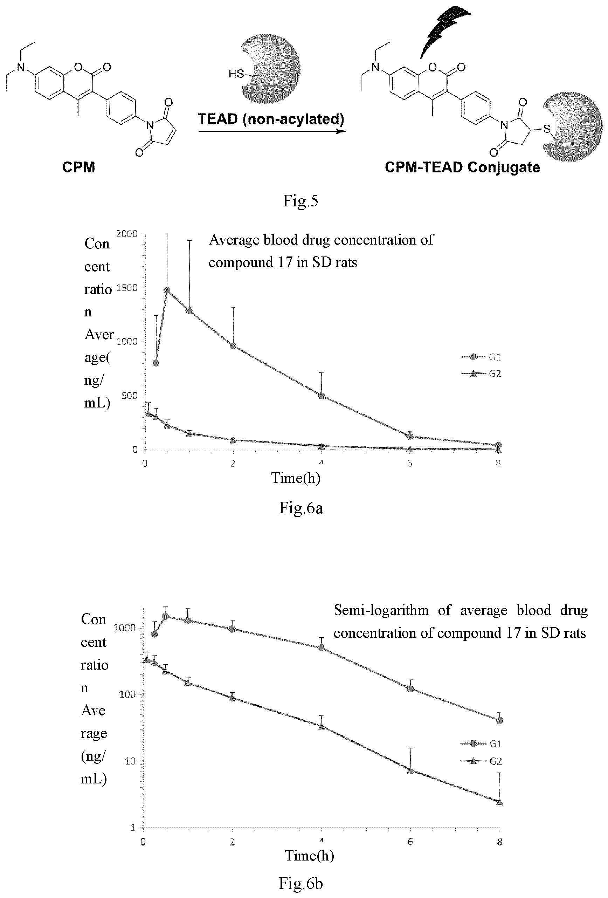

Diluting the tested compound in gradient to 5uL with buffer (25mM HEPES, 150mM NaCl, pH6.5), then adding 5uL of the newly prepared 0.45uM recombinant TEAD protein solution, and incubating at room temperature for 10 minutes. Then adding 5uL of 0.45uM CPM [7-diethylamino-3-(4- - phenylmaleimide)-4-methylcoumarin] solution, and the obtained 15uL final volume contains 150nM recombinant TEAD protein and 150nM CPM. After shaking the plate at room temperature for 1 hour, measuring the fluorescence intensity (Ex/Em: 380/470nm). When the tested compound does not bind to the central pocket of the TEAD protein, the cysteine thiol group in the central pocket will react chemically with CPM to generate a conjugated system group with fluorescence absorption (see Fig. 5); when the tested compound can bind to the central pocket of the TEAD protein, the pocket is occupied, and the cysteine thiol group in the pocket cannot react chemically with CPM, and no group with fluorescence absorption can be generated. The test results of some compounds are shown in the following table:

**Table 4 Results of using the CPM assay to test the ability of compounds to bind to the central pocket of recombinant TEAD protein**

| Compound No. | IC ₅₀ and TEAD protein types |
|---|---|
| 17 | 0.29 µM, TEAD1 |
| VT103 | 1.2 µM, TEAD1 |
| VT103 | 0.88 µM, TEAD2 |
| VT103 | 2.7 µM, TEAD3 |
| VT103 | 2.5 µM, TEAD4 |
| 8 | 0.11 µM, TEAD1 |
| 8 | 0.19 µM, TEAD4 |
| 6 | 0.23 µM, TEAD1 |
| 6 | 0.26 µM, TEAD2 |
| 6 | 0.19 µM, TEAD4 |
| 7 | 0.17 µM, TEAD1 |
| 7 | 0.25 µM, TEAD4 |
| 18 | 0.16 µM, TEAD1 |
| 18 | 0.21 µM, TEAD4 |
| 21 | 0.20 µM, TEAD1 |
| 21 | 0.31 µM, TEAD2 |
| 21 | 0.11 µM, TEAD4 |
| 22 | 0.33 µM, TEAD1 |
| 22 | 0.29 µM, TEAD2 |
| 22 | 0.63 µM, TEAD3 |
| 22 | 0.24 µM, TEAD4 |
| 24 | 0.18 µM, TEAD1 |
| 24 | 0.29 µM, TEAD4 |
| 27 | 0.35 µM, TEAD1 |
| 27 | 0.29 µM, TEAD4 |
| 28 | 0.10 µM, TEAD1 |
| 28 | 0.17 µM, TEAD4 |
| 29 | 0.18 µM, TEAD1 |
| 29 | 0.10 µM, TEAD4 |
| 30 | 0.69 µM, TEAD1 |
| 30 | 0.81 µM, TEAD4 |
| 31 | 0.41 µM, TEAD1 |
| 31 | 0.34 µM, TEAD4 |
| 45 | 0.22 µM, TEAD1 |
| 45 | 0.38 µM, TEAD2 |
| 45 | 0.49 µM, TEAD3 |
| 45 | 0.18 µM, TEAD4 |
| 61 | 0.07 µM, TEAD1 |
| 61 | 0.12 µM, TEAD4 |
| 64 | 0.54 µM, TEAD1 |
| 64 | 0.60 µM, TEAD2 |
| 64 | 0.75 µM, TEAD3 |
| 64 | 0.72 µM, TEAD4 |
| 66 | 0.14 µM, TEAD1 |
| 68 | 0.09 µM, TEAD1 |
| 68 | 0.15 µM, TEAD2 |
| 68 | 1.1 µM, TEAD3 |
| 68 | 0.22 µM, TEAD4 |
| 72 | 0.19 µM, TEAD1 |
| 72 | 0.41 µM, TEAD2 |
| 72 | 0.77 µM, TEAD3 |
| 72 | 0.52 µM, TEAD4 |
| 73 | 0.39 µM, TEAD1 |
| 73 | 0.44 µM, TEAD2 |
| 73 | 0.89 µM, TEAD3 |
| 73 | 0.62 µM, TEAD4 |
| 74 | 0.19 µM, TEAD1 |
| 74 | 0.21 µM, TEAD4 |
| 75 | 0.17 µM, TEAD1 |
| 75 | 0.29 µM, TEAD2 |
| 75 | 0.81 µM, TEAD3 |
| 75 | 0.20 µM, TEAD4 |
| 76 | 0.34 µM, TEAD1 |
| 76 | 0.53 µM, TEAD2 |
| 76 | 0.71 µM, TEAD3 |
| 76 | 0.22 µM, TEAD4 |

It can be seen from the above test results that the compounds of the present invention have a higher binding ability to the central pocket of the TEAD protein, and the binding force of the compounds of the present invention is stronger than that of the reported positive control compound VT103.

### Test Example 7: SD rat plasma pharmacokinetic test of the compound

Animal information: SD rats, male, n = 6, about 180-200 g, purchased from Sibeifu (Beijing) Biotechnology Co., Ltd.

Animal dietary status: Animals are fasted for at least 12 h before administration and resumed eating 4h after administration; during the experiment, animals are allowed to drink water freely.

Administration: administering the compound to experimental animals (n = 3) by intragastric administration at a dose of 10 mg/kg, and the solvent is 0.5% CMC-Na; administering the compound to experimental animals (n = 3) by intravenous injection at a dose of 1 mg/kg, and the solvent is DMSO:PEG400:normal saline = 5:30:65, preparing immediately before use.

Sample collection: collecting about 0.25 mL of whole blood at each time point, anticoagulating with EDTA-K2, and placing on ice immediately after collection, within 0.5 h after collection, centrifuging at 4°C, 2000 g for 5 min, and sucking up all plasma and placing in labeled EP tubes, placing the EP tubes on ice, and freezing the plasma samples in a -60~ -90°C refrigerator within 0.5 h.

Sample processing: centrifuging whole blood samples at 4°C, 2000 g for 5 min within 30 min after collection to separate plasma, and collecting the upper plasma sample into a sample tube.

Sample testing and data processing: Using LC-MS/MS method to detect the concentration of the original drug in plasma samples at each time point. WinNonlin is used for fitting and pharmacokinetic parameter calculation. The results are shown in Figures 6a and 6b and Table 5.

**Table 5 Pharmacokinetic parameters of SD rats after single IG of 10 mg/kg and IV of 1 mg/kg**

| parameter | unit | Average pharmacokinetic parameters of the original drug in plasma of rats in group G1 after IG of 10 mg/kg compound 17 | | |
|---|---|---|---|---|
| Cₘₐₓ | ng/mL | 1480 | ± | 576 |
| Tₘₐₓ | h | 0.50 (0.5,0.5) | | |
| AUC₀₋ₜ | h*ng/mL | 4440 | ± | 1860 |
| MRT₀₋ₜ | h | 2.25 | ± | 0.0707 |
| AUC_{0-∞} | h*ng/mL | 4510 | ± | 1880 |
| MRT_{0-∞} | h | 2.37 | ± | 0.0718 |
| t_{1/2} | h | 1.26 | ± | 0.0466 |
| Absolute bioavailability (%) | 84.9 | | | |

| parameter | unit | Average pharmacokinetic parameters of the original drug in plasma of rats in group G2 after IV of 1 mg/kg compound 17 | | |
|---|---|---|---|---|
| Cₘₐₓ | ng/mL | 333 | ± | 105 |
| AUC₀₋ₜ | h*ng/mL | 506 | ± | 113 |
| AUC_{0-∞} | h*ng/mL | 523 | ± | 103 |
| MRT₀₋ₜ | h | 1.52 | ± | 0.491 |
| MRT_{0-∞} | h | 1.73 | ± | 0.518 |
| t_{1/2} | h | 1.18 | ± | 0.395 |
| CL | mL/h/kg | 1970 | ± | 417 |
| V_{SS} | mL/kg | 3360 | ± | 957 |

As shown in Figures 6a and 6b and Table 5, Compound 17 exhibited good pharmacokinetic properties in SD rats.

Those skilled in the art will appreciate that the above-mentioned embodiments are specific examples for implementing the present invention, and in actual applications, various changes may be made thereto in form and detail without departing from the spirit and scope of the present invention.

## Claims

1. A compound having a structure represented by general formula (I), 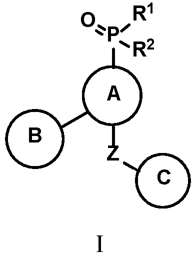
or pharmaceutically acceptable salts, stereoisomers, solvates or prodrugs thereof,
wherein Z is -CH₂-, -NH(CH₂)n-, -O(CH₂ )n-, -S-, -SO-, -SO₂-, each n is independently an integer of 0 to 3;
R¹ is C₁₋₆ alkyl, C₂₋₆ alkenyl, C₁₋₆ alkyl with at least one hydrogen replaced by R¹⁻¹, C₂₋₆ alkenyl with at least one hydrogen replaced by R¹⁻¹;
R² is C₁₋₆ alkyl, C₂₋₆ alkenyl, C₁₋₆ alkyl with at least one hydrogen replaced by R¹⁻¹, C₂₋₆ alkenyl with at least one hydrogen replaced by R¹⁻¹;
or, R¹ and R² are bonded to form a 4 to 6-membered ring or a 4 to 6-membered ring with at least one hydrogen replaced by R¹⁻¹, wherein each R¹⁻¹ is independently amino, hydroxyl, hydroxyl-substituted C₁₋₆ alkyl, and 5- or 6-membered monocyclic heteroaryl;
Ring A is 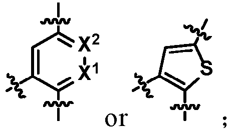 wherein X¹ is CH or N, X² is CH or N; Ring B is a 5 to 10-membered heteroaryl, 5 to 10-membered heteroaryl with at least one hydrogen replaced by 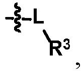 5 to 10-membered heteroaryl ketone group, 5 to 10-membered heteroaryl ketone group with at least one hydrogen replaced by 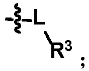
wherein L is none, -CH₂-, -NH-, -O-, -CH₂O-, -S-, -SO-, or -SO₂-;
each R³ is independently none, hydroxyl, cyano 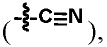 C₁₋₆ alkyl, C₁₋₆ alkyl with at least one hydrogen replaced by R³⁻¹, amino, aminowith at least one hydrogen replaced by R³⁻¹, 3 to 6-membered heterocycloalkyl, 3 to 6-membered heterocycloalkyl with at least one hydrogen replaced by R³⁻¹, 5- or 6-membered monocyclic heteroaryl, 5- or 6-membered monocyclic heteroaryl with at least one hydrogen replaced by R³⁻¹;
wherein each R³⁻¹ is independently hydroxyl, C₁₋₄ alkyl with at least one hydrogen replaced by hydroxyl , C₁₋₄ alkoxy, halogen;
Ring C is phenyl, phenyl with at least one hydrogen replaced by R⁴, 3 to 7-membered monocyclic alkyl, 3 to 7-membered monocyclic alkyl with at least one hydrogen replaced by R⁴ , C₅₋₁₂ bridged bicycloalkyl, C₅₋₁₂ bridged bicycloalkyl with at least one hydrogen replaced by R⁴, C₁₀₋₂₀ bridged tricycloalkyl, C₁₀₋₂₀ bridged tricycloalkyl with at least one hydrogen replaced by R⁴, 8 to 10-membered benzocycloalkyl, 8 to 10-membered benzocycloalkyl with at least one hydrogen replaced by R⁴;
wherein each R⁴ is independently C₁₋₄ alkyl, C₁₋₄ alkyl with at least one hydrogen replaced by halogen, halogen, 3 to 6-membered cycloalkyl, C₁₋₄ alkoxy, C₁₋₄ alkoxy with at least one hydrogen replaced by halogen, C₁₋₄ alkyl thiol, C₁₋₄ alkyl thiol with at least one hydrogen replaced by halogen, halogen-substituted thiol.

2. The compound according to claim 1, wherein
each R¹ is independently methyl, ethyl, methyl with at least one hydrogen atom replaced by R¹⁻¹ , ethyl with at least one hydrogen atom replaced by R¹⁻¹ , or ethenyl,
each R² is independently methyl, ethyl, methyl with at least one hydrogen atom replaced by R¹⁻¹, ethyl with at least one hydrogen atom replaced by R¹⁻¹, or ethenyl, wherein R¹⁻¹ is amino, hydroxyl, or a 5- or 6-membered nitrogen-containing monocyclic heteroaryl;
or, R¹ and R² are bonded to each other to form a 4 to 6-membered ring, or R¹ and R² are bonded to each other to form a 4 to 6-membered ring with at least one hydrogen atom replaced by R¹⁻¹, wherein each R¹⁻¹ is independently amino, hydroxy, hydroxy-substituted methyl, hydroxy-substituted ethyl, or a 5- or 6-membered nitrogen-containing monocyclic heteroaryl;
and/or, Ring A is 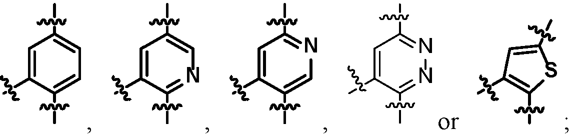
and/or, Ring B is a 5-membered nitrogen-containing monocyclic heteroaryl, a 6-membered nitrogen-containing monocyclic heteroary, a 5-membered nitrogen-containing monocyclic heteroaryl with at least one hydrogen replaced by 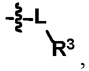 a 6-membered nitrogen-containing monocyclic heteroaryl with at least one hydrogen replaced by 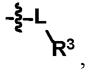 an 8 to 10-membered nitrogen-containing fused ring heteroaryl, an 8 to 10-membered nitrogen-containing fused ring heteroaryl with at least one hydrogen replaced by 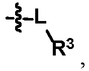 a 5-membered nitrogen-containing monocyclic heteroaryl ketone group, a 6-membered nitrogen-containing monocyclic heteroaryl ketone group, a 5-membered nitrogen-containing monocyclic heteroaryl ketone group with at least one hydrogen replaced by 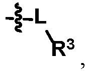 or a 6-membered nitrogen-containing monocyclic heteroaryl ketone group with at least one hydrogen replaced by 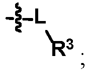
and/or, Ring C is phenyl, phenyl with at least one hydrogen replaced by R⁴ , 4 to 6-membered monocycloalkyl, 4 to 6-membered monocycloalkyl with at least one hydrogen replaced by R⁴, C₅₋₈ bridged bicycloalkyl, C₅₋₁₂ bridged bicycloalkyl with at least one hydrogenreplaced by R₄ , C10-20 bridged tricycloalkyl, C₁₀₋₂₀ bridged tricycloalkyl with at least one hydrogen replaced by R⁴, 8 to 10-membered benzocycloalkyl, 8 to 10-membered benzocycloalkyl with at least one hydrogen replaced by R⁴ .

3. The compound according to claim 2, wherein
in the Ring B, the 5- or 6-membered nitrogen-containing monocyclic heteroaryl is 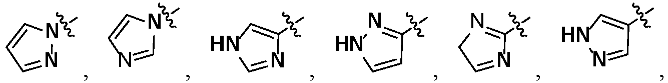 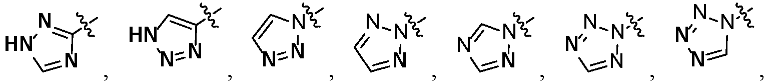 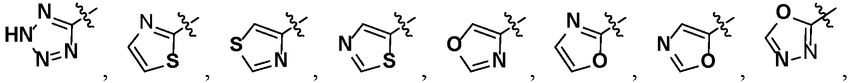 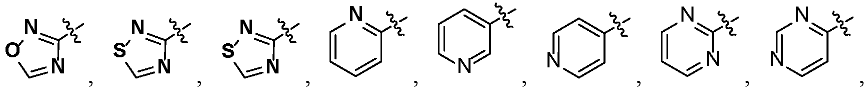 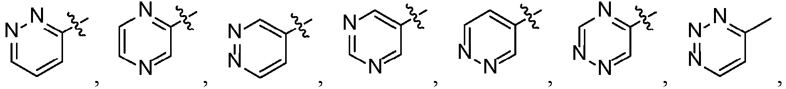 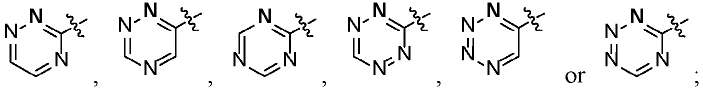
and/or, in the Ring B, the 5-membered nitrogen-containing monocyclic heteroaryl gwith at least one hydrogen replaced by 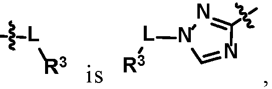 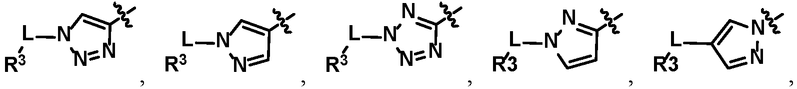 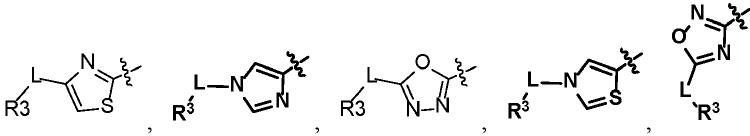 or 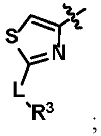
and/or, in the Ring B, the 6-membered nitrogen-containing monocyclic heteroaryl with at least one hydrogen replaced by 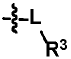 is 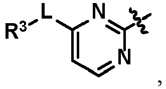 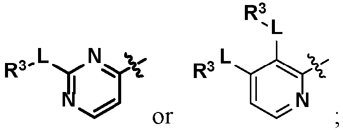
and/or, in the Ring B, the 8 to 10-membered nitrogen-containing monocyclic heteroaryl is 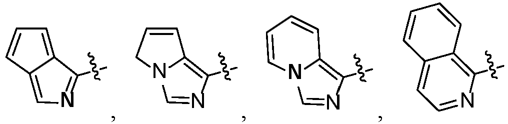 or 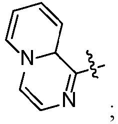
and/or, in the Ring B, the 8 to 10-membered nitrogen-containing monocyclic heteroaryl with at least one hydrogen replaced by 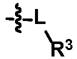 is 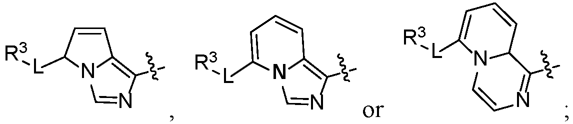
and/or, in the Ring B, the 6-membered nitrogen-containing monocyclic heteroaromatic ketone group is 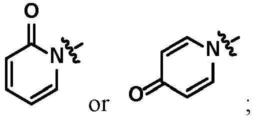
and/or, the 6-membered nitrogen-containing monocyclic heteroaryl ketone group with at least one hydrogen replaced by 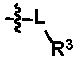 is 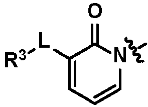 or

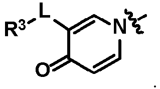

4. The compound according to claim 2, wherein
in the Ring C, the phenyl with at least one hydrogen replaced by R⁴ is 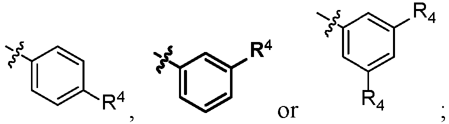
and/or, in the Ring C, the 4 to 6-membered monocyclic alkyl is cyclohexyl, cyclopentyl or cyclobutyl;
and/or, in the Ring C, the 4 to 6-membered monocycloalkyl with one hydrogen replaced by R⁴ is 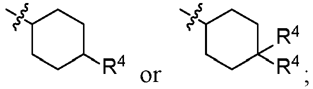
and/or, in the Ring C, the C₅₋₈ bridged bicycloalkyl is bicyclo[1.1.1]pentyl or bicyclo[2.2.2]octyl;
and/or, in the Ring C, the C₅₋₁₂ bridged bicycloalkyl with at least one hydrogen replaced by R⁴ is bicyclo[1.1.1]pentyl with one hydrogen replaced by R⁴ or bicyclo[2.2.2]octyl with one hydrogen replaced by R⁴ ;
and/or, in the Ring C, the C₁₀₋₂₀ bridged tricycloalkyl is adamantyl;
and/or, in the ring C, the C₁₀₋₂₀ bridged tricycloalkyl with at least one hydrogen replaced by R⁴ is adamantyl with one hydrogen replaced by R⁴ ;
and/or, in the Ring C, the 8 to 10-membered benzocycloalkyl is benzocyclopentyl, benzocyclobutyl or benzocyclohexyl;
and/or, in the ring C, the 8 to 10-membered benzocycloalkyl with at least one hydrogen is replaced by R⁴ is benzocyclopentyl group with at least one hydrogen is replaced by R⁴, benzocyclobutyl with at least one hydrogen is replaced by R⁴ , or benzocyclohexyl with at least one hydrogen is replaced by R⁴.

5. The compound according to claim 1, wherein
each R³ is independently hydroxyl, cyano, C₁₋₄ alkyl, C₁₋₄ alkyl with at least one hydrogen replaced by R³⁻¹, amino, aminowith at least one hydrogen is replaced by R³⁻¹, 3 to 6-membered nitrogen heterocycloalkyl, 3 to 6-membered oxygen heterocycloalkyl, 3 to 6- membered nitrogen heterocycloalky with at least one hydrogen replaced by R³⁻¹, 3 to 6-membered oxygen heterocycloalkyl with at least one hydrogen replaced by R³⁻¹, 6-membered nitrogen-containing monocyclic heteroaryl, 6-membered nitrogen-containing monocyclic heteroaryl with at least one hydrogen replaced by R³⁻¹;
and /or, each R³⁻¹ is independently hydroxy, methyl with at least one hydrogen replaced by hydroxy, ethyl with at least one hydrogen replaced by hydroxy, n-propyl with at least one hydrogen replaced by hydroxy, isopropyl with at least one hydrogen replaced by hydroxy, methoxy, ethoxy, n-propoxy, isopropoxy, fluorine or chlorine;
and/or, each R⁴ is independently methyl, ethyl, n-propyl, isopropyl, n-butyl, isobutyl, tert-butyl, methyl with at least one hydrogen replaced by fluorine, ethyl with at least one hydrogen replaced by fluorine, n-propyl with at least one hydrogen replaced by fluorine, isopropyl with at least one hydrogen replaced by fluorine, fluorine, chlorine, bromine, iodine, cyclopropane, cyclobutane, cyclopentane, cyclohexane, methoxy, ethoxy, n-propoxy, isopropoxy, trifluoromethoxy, hexafluoroethoxy, methylthiol, ethylthiol, n-propylthiol, isopropylthiol, methylthiol with at least one hydrogen replaced by fluorine, ethylthiol with at least one hydrogen replaced by fluorine, or halogen-substituted thiol.

6. The compound according to claim 1, wherein
each R³ is independently hydroxyl, cyano, methyl, ethyl, n-propyl, isopropyl, n-butyl, isobutyl, tert-butyl, methyl with at least one hydrogen replaced by R³⁻¹, ethyl with at least one hydrogen replaced by R³⁻¹, n-propyl with at least one hydrogen replaced by R³⁻¹, isopropyl with at least one hydrogen replaced by R³⁻¹, amino, amino with at least one hydrogen replaced by R³⁻¹,

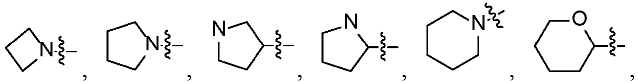

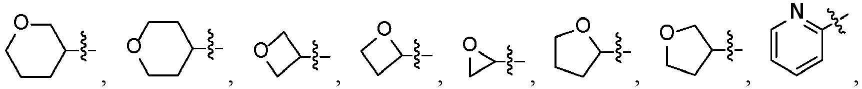

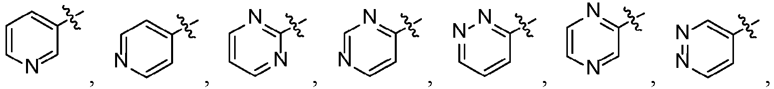

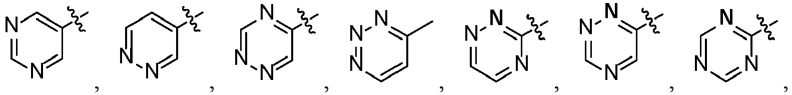

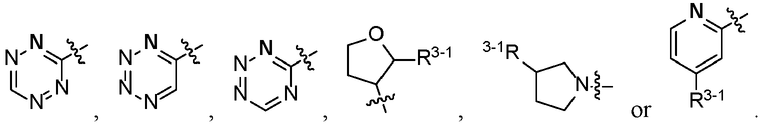

7. The compound according to any one of claims 1 to 6, wherein the compound is selected from any one of the following:
| | |
|---|---|
| 1 | 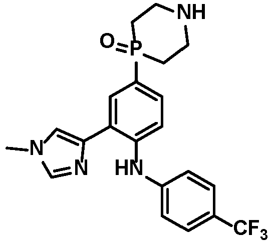 |
| 2 | 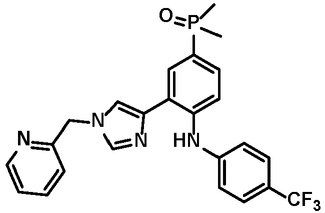 |
| 3 | 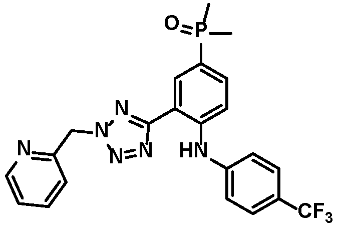 |
| 4 | 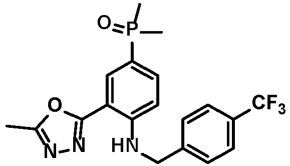 |
| 5 | 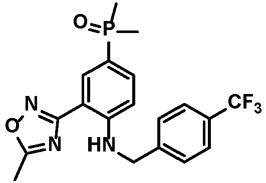 |
| 6 | 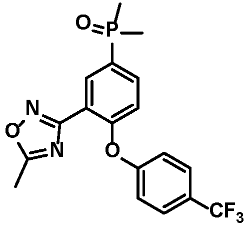 |
| 7 | 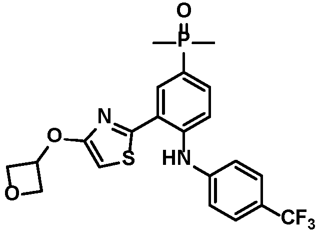 |
| 8 | 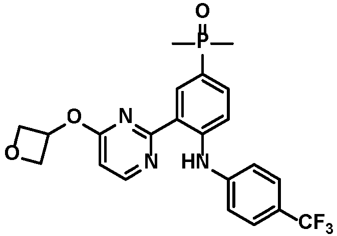 |
| 9 | 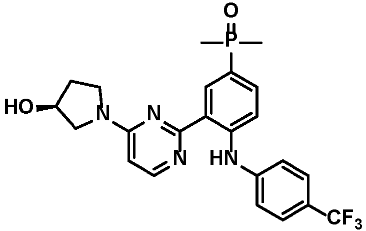 |
| 10 | 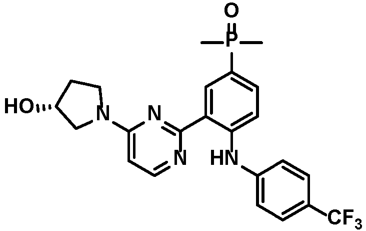 |
| 11 | 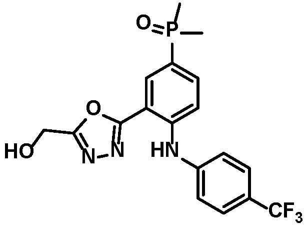 |
| 12 | 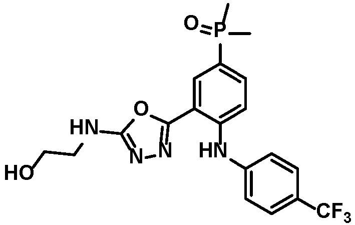 |
| 13 | 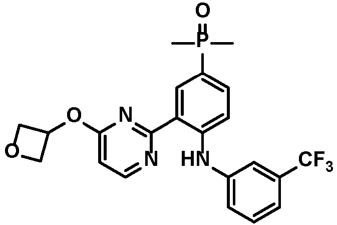 |
| 14 | 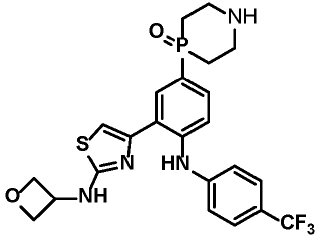 |
| 15 | 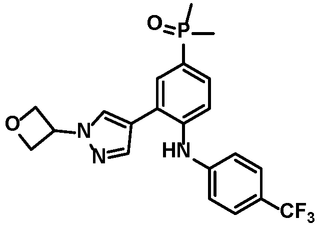 |
| 16 | 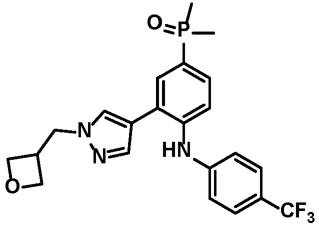 |
| 17 | 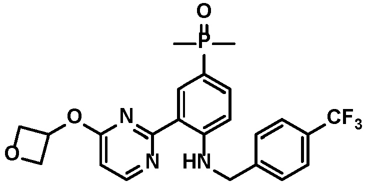 |
| 18 | 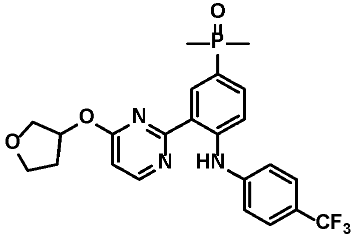 |
| 19 | 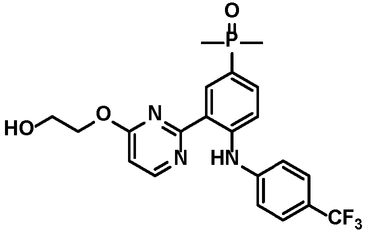 |
| 20 | 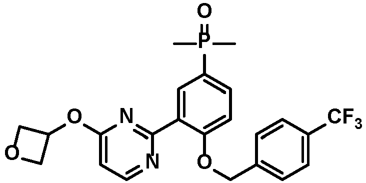 |
| twenty one | 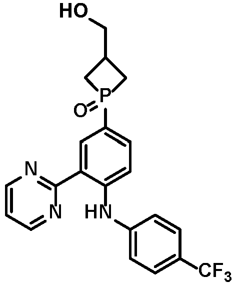 |
| twenty two | 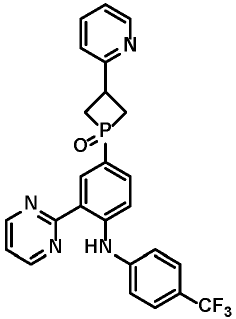 |
| twenty three | 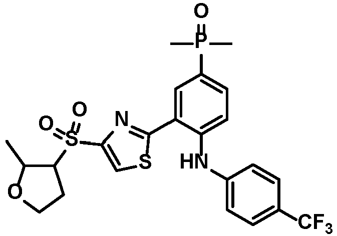 |
| twenty four | 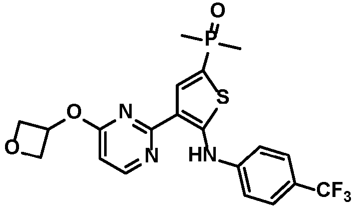 |
| 25 | 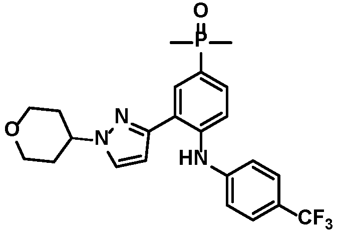 |
| 26 | 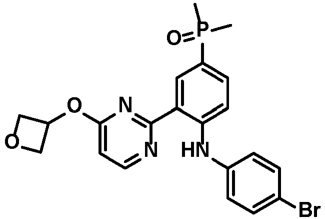 |
| 27 | 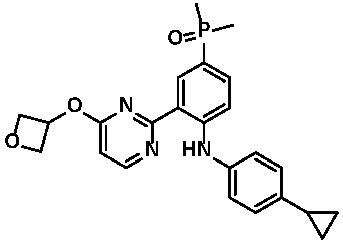 |
| 28 | 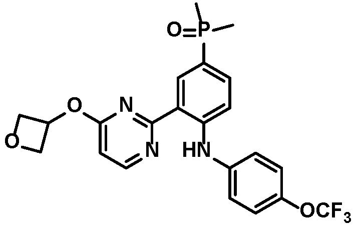 |
| 29 | 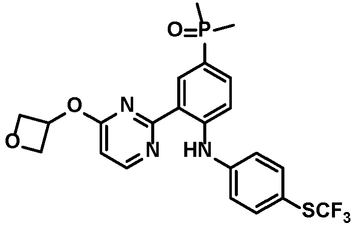 |
| 30 | 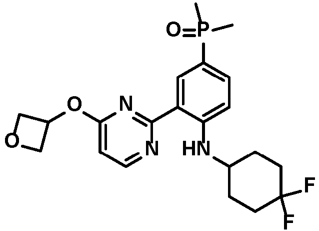 |
| 31 | 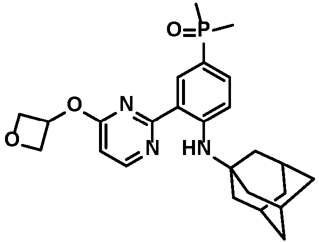 |
| 32 | 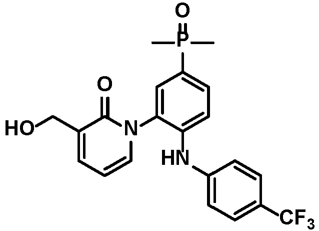 |
| 33 | 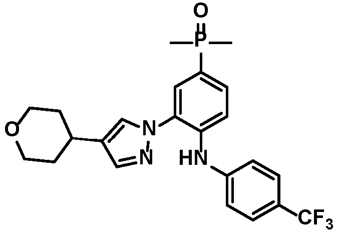 |
| 34 | 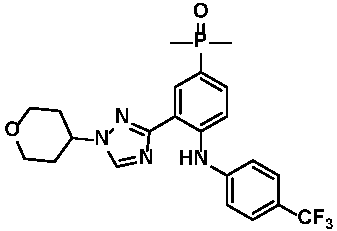 |
| 35 | 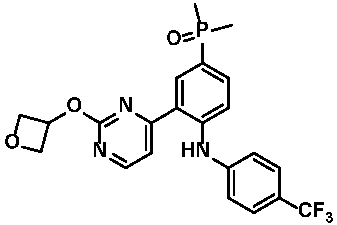 |
| 36 | 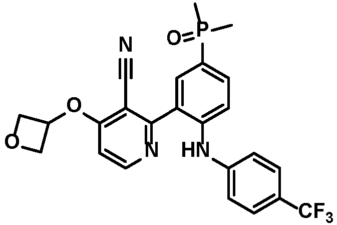 |
| 37 | 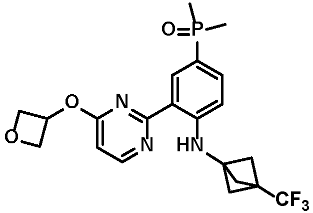 |
| 38 | 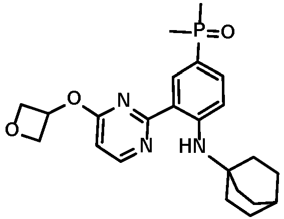 |
| 39 | 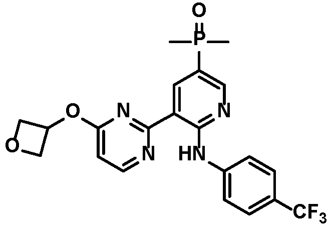 |
| 40 | 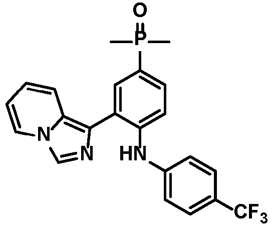 |
| 41 | 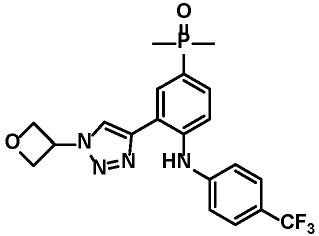 |
| 42 | 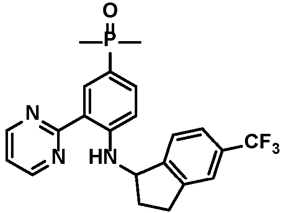 |
| 43 | 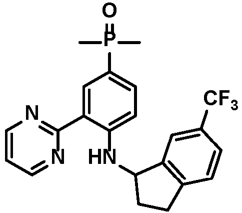 |
| 44 | 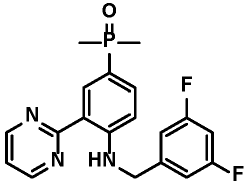 |
| 45 | 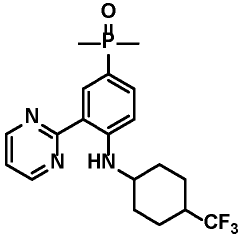 |
| 46 | 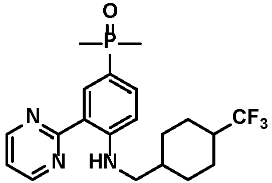 |
| 47 | 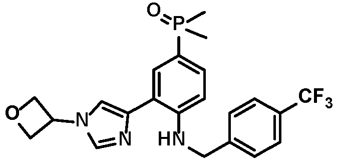 |
| 48 | 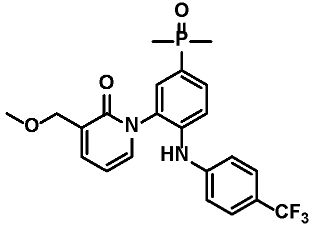 |
| 49 | 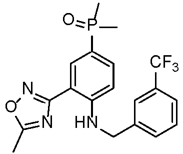 |
| 50 | 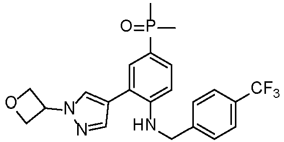 |
| 51 | 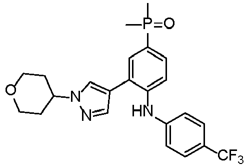 |
| 52 | 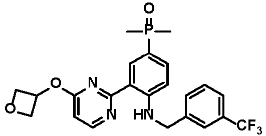 |
| 53 | 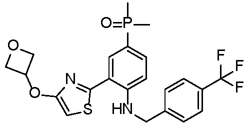 |
| 54 | 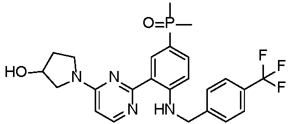 |
| 55 | 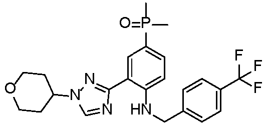 |
| 56 | 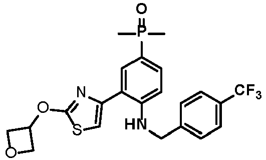 |
| 57 | 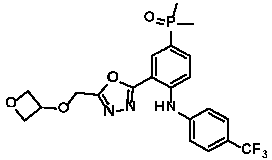 |
| 58 | 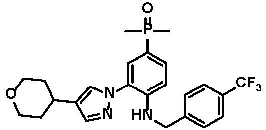 |
| 59 | 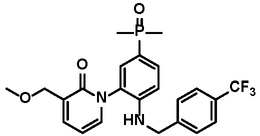 |
| 60 | 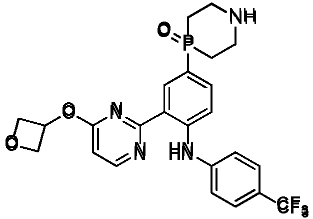 |
| 61 | 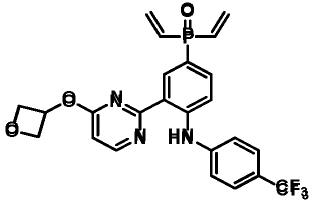 |
| 62 | 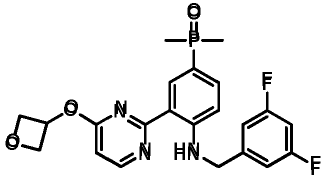 |
| 63 | 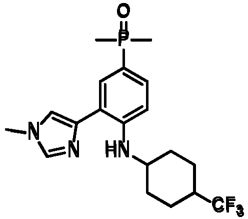 |
| 64 | 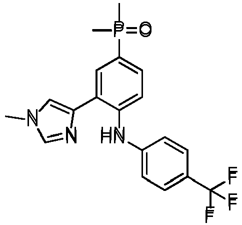 |
| 65 | 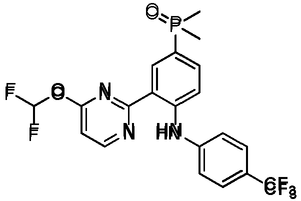 |
| 66 | 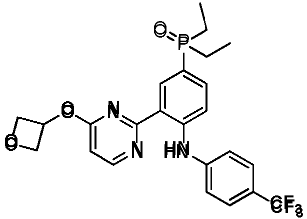 |
| 67 | 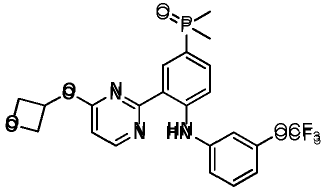 |
| 68 | 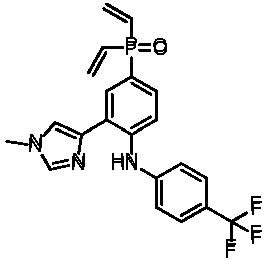 |
| 69 | 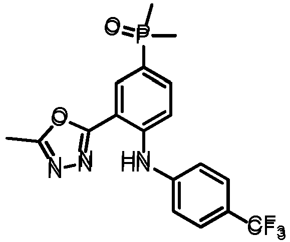 |
| 70 | 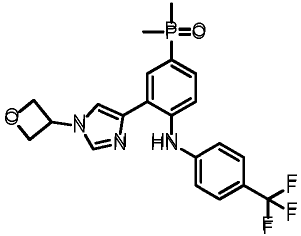 |
| 71 | 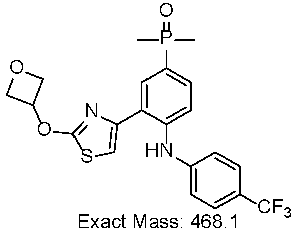 |
| 72 | 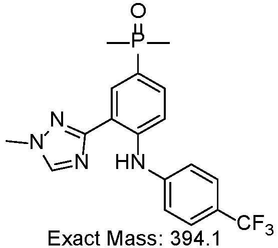 |
| 73 | 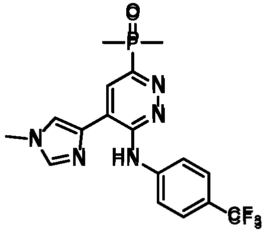 |
| 74 | 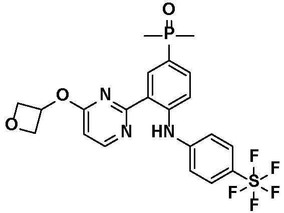 |
| 75 | 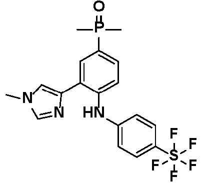 |
| 76 | 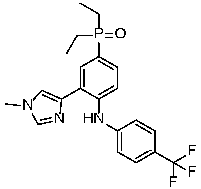 |

8. A pharmaceutical composition, **characterized in that** the pharmaceutical composition comprises the compound or pharmaceutically acceptable salts, stereoisomers, solvates or prodrugs thereof according to any one of claims 1 to 7, and a pharmaceutically acceptable carrier.

9. Uses of the compound or pharmaceutically acceptable salts, stereoisomers, solvates or prodrug sthereof according to any one of claims 1 to 7 or the pharmaceutical composition according to claim 8, wherein they are used for preparing drugs or pharmaceutical compositions, wherein the drugs or pharmaceutical compositions are used for one or more uses selected from the following:
(i) binding to TEAD, preferably binding to the palmitoyl pocket of TEAD;
(ii) inhibiting TEAD transcription levels;
(iii) inhibiting/blocking YAP-TEAD binding;
(iv) regulating the Hippo signaling pathway;
(v) treating diseases associated with increased TEAD transcription levels and/or YAP phosphorylation disorders and/or Hippo signaling pathway disorders; and/or
(vi) preparing drugs for treating diseases associated with increased TEAD transcription levels and/or YAP phosphorylation disorders and/or Hippo signaling pathway disorders.

10. A method for treating diseases associated with increased TEAD transcription levels and/or YAP phosphorylation disorders and/or Hippo signaling pathway disorders, wherein the method comprises the steps of:
administering the compound according to any one of claims 1 to 7 or pharmaceutically acceptable salts, stereoisomers, solvates or prodrugs thereof to a subject, or
administering the pharmaceutical composition according to claim 8 to a subject.
